# EUROPEAN PATENT SPECIFICATION

(11) **EP 3 095 440 B1**
(45) Date of publication and mention of the grant of the patent: **15.01.2020**
(21) Application number: 15001524.6
(22) Date of filing: 19.05.2015
(51) Int. Cl.: A61K 9/00, A61K 9/127, A61K 39/00, A61K 39/35, A61K 9/06, A61K 47/34, A61K 31/573, A61K 31/593, A61K 47/69

(54) **ANTIGEN-SPECIFIC IMMUNOTHERAPY USING TOLERIZING LIPOSOMES**
ANTIGEN-SPEZIFISCHE IMMUNTHERAPIE MIT TOLERANZINDUZIERENDEN LIPOSOMEN
IMMUNOTHÉRAPIE SPÉCIFIQUE D'UN ANTIGÈNE À L'AIDE DE LIPOSOMES DE TOLÉRANCE

(43) Date of publication of application: 23.11.2016
(73) Proprietor: PLS-Design GmbH, 20255 Hamburg (DE)
(72) Inventor: Bredehorst, Reinhard, D-20255 Hamburg (DE); Grunwald, Thomas, D-22459 Hamburg (DE)
(74) Representative: Jungblut, Bernhard Jakob

(56) References cited:
- EP-A1- 2 746 396
- EP-A1- 2 918 262
- WO-A2-2015/023796
- US-A1- 2015 050 332

## Description

### INTRODUCTION

Allergen- or autoantigen-specific immunotherapy (SIT) offers the promise of restoring lasting immunological tolerance to allergens or autoantigens. For the treatment of allergy, specific immunotherapy is efficient when patients are mono-sensibilized against seasonal allergens, but can be less or not efficient if the patient is atopic or if the patient reacts to perennial allergens. Therefore, there is a need to design more effective allergen-tolerogenic therapies. For the treatment of asthma and autoimmune diseases including type I diabetes, rheumatoid arthritis, and multiple sclerosis, specific immunotherapy has shown some efficacy, but currently used treatment protocols need to be combined with immune modulatory techniques to enhance restoration of lasting clinical tolerance.

Regulatory T cells (Tregs) represent most promising targets for such supporting immune modulatory techniques. Numerous studies have demonstrated that control of the development, survival, and function of Tregs is instrumental for effective control of immune responses.The activation, proliferation and effector functions of a large spectrum of immune-competent cells such as CD4+ cells, CD8+ cells, NK cells, NKT cells, dendritic cells, macrophages, and B cells are susceptible to suppression mediated by regulatory T cells (Tregs).

The term Treg encompasses at least 5 subsets of cellpopulations including a)natural thymus-derived CD4(+)CD25(+)Foxp3(+) nTregs producing IL-10 and TGFβ, b) CD8(+)CD25(+)Foxp3(+) Tregs producing IL-10, IL-17 and IFNγ, c) peripherally induced CD4(+)Foxp3(+) iTregs producing IL-10 and TGFβ (IL-10- and TGFβ-producing Th3 cells belong to this subset), d) inducibleCD4(+)CD25(+)Foxp3(-) Tr1 cells producing IL-10, and e) CD4(+)Foxp3(+) Tregs producing IL-17 (for review, see Zhang et al., 2014). In addition to Tregs, a newly described population of regulatory B cells (Breg) producing IL-10 and/or TGFβ also contributes to immunosuppression both directly and via enhancement of Treg function (for a review, see Grant et al., 2015).

Both nTregs and inducible Tr1 cells inhibit the development of allergy via several mechanisms, including suppression of effector Th1, Th2,and Th17 cells, suppression of eosinophils, mast cells and basophils, induction of antibody isotype change from IgE to IgG4, suppression of inflammatory dendritic cells (DC), and suppression of inflammatory cellmigration to tissues (for a review, see Palomares et al., 2010). Furthermore, it is well established that autoimmune responses result from the breakdown of immune tolerance maintained by Treg cells. Treg impairment responsible for allergic and autoimmune diseases includes Treg numerical and functional defects and conversion into effector cells in response to inflammation, although resistance of effector T cells to Treg control has also been described (for a review, see Grant et al., 2015).

The induction of Treg-suppressive activity is specific and requires antigenic stimulation through the T cell receptor (TCR). The exact mechanisms of Treg-mediated suppression remain to be elucidated, but cell-to-cell contacts and several molecules such as IL-10, TGFβ (TGF-beta), CTLA-4 (cytotoxic T lymphocyte antigen 4; CD152) and granzyme/perforin are reported to contribute to the suppressive activity of Tregs.

However, the suppressive activity of Tregs is not antigen-specific. Regulatory or suppressive T-cell responses targeted to specific antigens (antigen-specific Tregs) can simultaneously suppress bystander responses in the same location. As a result, a wide range of immune responses can be inhibited by Tregs via bystander suppression. In principle, bystander suppression enables allergen/autoantigen-specific Tregs of a single specificity to suppress allergic or autoimmune responses despite the presence of immunoreactivity to multiple allergens or auto-antigens. For example, joint inflammatory responses in patients with rheumatoid arthritis could be suppressed by arthritogenic antigen-specific Tregs of a single specificity even if auto-reactivity to multiple auto-antigens exists.

### Adoptive transfer of antigen- or allergen-specific Tregs.

The efficacy of bystander suppression of has been demonstrated in several animal models in which transfer of antigen- or allergen-specific regulatory T cells suppressed or markedly reduced clinical signs of various allergic and autoimmune diseaseincluding experimental autoimmune encephalomyelitis (EAE) (Kohm et al., 2002),experimental collagen-induced arthritis (CIA) (Morgan et al., 2005; Kelchtermans et al., 2009; Kong et al., 2012), experimental autoimmune diabetes (Tang et al., 2004; Jaeckel et al., 2005), renal disease in lupus-prone NZB/NZW mice (Scalapino et al., 2006),an experimental murine model of inflammatory bowel disease (Mottet et al., 2003), and murine models of allergic airway inflammation (Kearley et al., 2005; Xu et al., 2012).

However, efficient control of established disease by adoptive transfer of Tregs apparently requires transfer of relatively high numbers of Tregs. In one study, augmentation of Treg numbers by ∼50-75% (2 × 10⁶ Treg cells transferred to naive murine recipients normally containing an estimated 2.5-3 × 10⁶ Treg cells) conferred significant protection against EAE induction/progression as measured by both disease score and the promotion of protective Th2 cytokines(Kohm et al., 2002). Therefore, in other adoptive transfer studies abundant naive CD4⁺ T cells were used to induce ex vivo CD4(+)CD25(+)Foxp3(+) Tregs by treatment with TGFβ, rather than isolating the small numbers of naturally occurring Treg cells (e.g. Xu et al., 2012).

In humans, purified Tregs from patients with recent-onset type 1 diabetes (T1D) and from healthy individuals have been successfully expanded ex vivousing IL-2 and microbeads coated with anti-CD3 and anti-CD28 (Putnam et al., 2009). The resulting population of Tregs displayed functional properties and showed stable expression of regulatory-cell markers and cytokines. In a recent clinical trial with 10 T1D children, a significant improvement of β-cell function was achieved after administration of 10-20 x 10⁶ ex vivo expanded autologous CD3(+)CD4(+)CD25(high)CD127(-) Tregs/kg body wt, although further follow-up analyses are needed to confirm whether this is a sustained remission or only delay in the destruction of islets (Marek-Trzonkowska et al., 2012. In any case, these results represent a first step in developing a personalized therapeutic Treg product for T1D, and potentially other autoimmune diseases (for reviews, see Esensten et al., 2009; Thompson et al., 2012; Herold et al., 2013).

The therapeutic application of adoptive transfer of antigen-or allergen-specific Tregs, however, poses also several serious problems. First of all, the function of these cell populations and the persistence of their function needs to be predictable once administered. Feared risks are the possible trans-differentiation of Tregs into other T cell subsets such as Th17 cells, the so far unknown influence of danger signals on Treg generation and stability and the possibility of panimmunosuppression by activated or ex vivo expanded Tregs with the emergence of infections and cancers.

Furthermore, autologous cell therapy is extremely challenging to develop for widespread clinical use. A major challenge pertains to the regulatory requirements for standardisation, sterility and quality control of cell therapies. If used to obtain PB cells, leukapheresis is associated with a degree of morbidity, and is logistically difficult in many centers. There are also difficulties designing protocols with adequate control groups, and as trials continue in this area there will be difficulties comparing results from individual small trials in which varying cell culture protocols or antigen preparations have been used. Last not least, the cost of carrying out autologous cell therapy trials is also a major impediment to the scale-up required for later-phase trials. This cost may be justifiable in cancer settings where therapeutic options are few, but this is more difficult in allergic or autoimmune diseases.

### Adoptive transfer of tolerogenic dendritic cells (DCs).

DCs are widely recognized as the most professional antigen-presenting cells (APCs). Moreover, they are indispensable in the regulation of the delicate balance between immunity and tolerance. By interacting with other cells of the immune system through cell-cell contact or the production of cytokines, DCs induce an appropriate answer to a specific antigen. DCs can also prevent autoimmunity by inducing apoptosis of autoreactive T cells in the thymus (central tolerance) and by induction of anergy, deletion, or tolerance through cooperation with regulatory T cells (Treg) in the periphery (peripheral tolerance). The ability of DCs to induce T cell immunosuppression by Treg expansion or T cell anergy made them particularly attractive as therapeutic targets for T cell dependent autoimmune diseases.

Recent investigations have shown that antigen-specific tolerance can be induced in vivo via vaccination with antigen-pulsed ex vivo generated DCs, so calledtolerogenic DCs (tolDCs). Such tolDCs are phenotypically immature DCs characterized by a low expression of MHC-II as well as costimulatorymolecules such as CD40, CD80, CD86, and a reduced production ofpro-inflammatory IL-12 and increased secretion of anti-inflammatory IL-10 (for reviews, see Thomas, 2013; Gross and Wiendl, 2013; Van Brussel et al., 2014; Mackern-Oberti et al., 2015).

Currently, it is possible to generate tolDCs by several methods, such as in vitro modulation by pharmacologicalagents and gene therapy. Among the pharmacological agents used to manipulate DC function and induce tolDCs, maturation inhibitors have been successfully employed. Inhibition of DC maturationresults in DCs that do not respond to pathogenassociated molecular pattern molecules (PAMPs) or danger-associated molecular pattern molecules (DAMPs) and proinflammatory stimulation.

Suitable pharmacological maturation inhibitors include dexamethasone (Dex), 1α,25-dihydroxyvitamin D3 (VD3); acetylsalicylic acid (aspirin), rapamycin (RAPA), estriol, vasoactive intestinal pepide (VIP), BAY-117082, andrographolide (a labdane diterpenoid), simvastatin (HMG-CoA reductase inhibitor), curcumin (diferuloylmethane), quercetin (flavonoid), and cytokines such as IL-10 and TGFβ. Biological agents that can also modulate immune responses are components derived from pathogens.Furthermore, recent advances in the interference RNA (iRNA) technology have provided researchers new strategies for autoimmune therapy design. It has been demonstrated that gene silencing of different pro-inflammatory molecules, such as CD40, CD80, CD86 and IL-12 promotes a tolerogenic phenotype to DCs (for reviews, see Van Brussel et al., 2014; Mackern-Oberti et al., 2015).

The efficacy of vaccination with antigen-pulsed ex vivo generated tolDCs has been demonstrated in several animal models of autoimmune diseases including experimental collagen-induced arthritis (CIA) (Chorny et al., 2005; van Duivenvoorde et al., 2007; Stoop et al., 2010), experimental antigen-induced arthritis (AIA) (Martin et al., 2007), experimental autoimmune encephalomyelitis (EAE) (Xiao et a., 2004; Toscano et al., 2010; Papenfuss et al. 2011; Matsuda et al., 2012), and experimental autoimmune diabetes (Clare-Salzler et al., 1992; Feili-Hariri et al., 1999; Machen et al., 2004).

Encouraging results with tolDCs were also obtained in clinical Phase I studies. In a Phase I randomized placebo-controlled trial with T1D patients (ClinicalTrials.gov identifier NCT00445913), administration (once every two weeks for two months) of 1 × 10⁷ autologous tolDCs (generated ex vivo in GM-CSF/IL-4 conditions with anti-sense oligonucleotides for the co-stimulatory molecules CD40, CD80, and CD86), induced up-regulation of the frequency of potentially beneficial peripheral B220(+)CD11c(-) B cells (Giannoukakis et al., 2011). In another Phase I study, patients with rheumatoid arthritis received only one intradermic dose of 1 × 10⁷ autologous tolDCs (generated by the tolerogenic agent BAY11-7082, an NFκB inhibitor, and loaded with citrullinated peptides (cit-vimentin, cit-fibrinogen, cit-fibrinogen, cit-collagen type II). Only mild adverse effects such as headache and minimal changes in hematology parameters were reported while the expected therapeutic effect could already be observed in some patients (Thomas et al., 2011).

In principle, tolerizing DC immunotherapy poses the same serious limitations as therapeutic applications of adoptive transfer of antigen- or allergen-specific Tregs. Most important is the identification of a maturation-resistant subtype of DCs. Using current methods for the generation of tolDCs, phenotypically immature DCs are produced. However, that does not guarantee stability of the tolerogenic properties, especially after vaccination. Given the risk of in vivo reactivation, this is particularly of importance in any pathological state with an underlying inflammatory microenvironment. For example, TNF-α-treated DCs, characterized by marked surface expression of MHC class II and costimulatory moleculesbut failure to secrete IL-1β, IL-6, TNF-α and IL-12, are able to reverseautoimmunity in an EAE mouse model (Menges et al., 2002). However, upon asecond stimulation with LPS and CD40 ligation in vitro and after subcutaneousinjection in vivo these tolDCs differentiate towards a more activatedstate, as demonstrated by upregulation of the production ofpro-inflammatory cytokines (Voigtlander et al., 2006). Future studies investigating the in vivo stability of therapeutic modifications of Tregs or DCs as well as the long-term outcome of animals undergoing Treg- or DC-based therapies are required until additional clinical trials in humans are justifiable (Fessler et al., 2013).

In an attempt to avoid many of the problems associated with therapeutic modifications of Tregs or DCs, liposomes were formulated to generate tolDCs under in vivo conditions. Egg phosphatidyl choline liposomes (400 nm) were loaded with antigen (OVA or methylated BSA) and a lipophilic NF-κB inhibitor (curcumin, quercetin, or Bay11-7082) and injected (intravenously or subcutaneously) into mice with antigen-induced inflammatory arthritis (Capini et al., 2009). As demonstrated in this study, the liposomes targeted antigen-presenting cells including several subtypes of DCs and macrophages in vivo, suppressing the responsiveness of these cells to NF-κB and inducing antigen-specific Tregs. Thereby, effector T-cell responses and the clinical signs of full-blown antigen-induced arthritis could be suppressed. Neither Bay11-7082 alone, curcumin liposomes containing irrelevant antigen, nor curcumin liposomes and soluble arthritogenic antigen administered concurrently, were sufficient to induce Foxp3(+) Tregcells or to suppress arthritis (Capini et al., 2009). Liposomes are also disclosed in EP 2 918 262, US 2015/050332, EP 2 746 396 and WO 2015/023796.

While these results clearly demonstrate that DC function can be effectively altered by NF-κB inhibitors using liposome-based in vivotargeting, there are also serious risks associated with this therapeutic approach. Intravenously injected tolerizing liposomes carry the risk of producing global tolerance via Treg-mediated bystander suppression in lymphoid organs of the reticuloendothelial system, especially spleen and mesenteric lymph nodes. This could be associated with the risk of increased infections and the emergence of cancer.

Subcutaneous injection of small amounts of tolerizing liposomes can avoid the risk of global tolerance via bystander suppression in various organs of the reticuloendothelial system. However, the uptake of tolerizing liposomes by phagocytes in subcutaneous tissues is not very effective. Therefore, this approach requires repeated injections, a procedure that is not acceptable for physicians and patients. In consequence, there is a need in the field for a cell-free therapeutic technology which provides the therapeutic benefits of Treg- and DC-based approaches, but avoids the risks associated with currently available liposomal targeting techniques.

### SUMMARY OF THE INVENTION

For the treatment of allergy, asthma and autoimmune diseases including but not limited to type I diabetes (T1D), rheumatoid arthritis (RA), and multiple sclerosis (MS), various forms of antigen-specific Tregs or antigen-loaded/antigen-exposed tolerizing DCs have the potential of restoring lasting immunological tolerance, but novel strategies are needed to translate this concept to an approach that is more broadly applicable than adoptive transfer of autologous cell products.

The present invention solves these problems and provides broadly applicable methods for restoring lasting immunological tolerance in patients suffering from allergy, allergic asthma, T1D, RA and MS.

The present invention relates to a pharmaceutical composition made of at least one preparation, wherein the preparation comprises:
tolerogenic liposomes tailored for effective phagocytosis and loaded with at least one maturation inhibitor of dendritic cells (DCs) selected from a) calcipotriol, b) glucocorticoids, and c) antisense oligonucleotides capable of gene silencing of different pro-inflammatory molecules including CD40, CD80, and CD86 and
at least one antigen or allergen or peptide derived thereof selected from ovalbumin (OVA), methylated BSA (mBSA), or the myelin oligodendrocyte glycoprotein (MOG)-derived peptide 35-55 (MOG(35-55),
   at least one immune modulator of phagocytosis selected from the nucleotides ATP and UTP,
   wherein at least said liposomes are embedded in a matrix suitable for locally restricted sustained release of therapeutically effective doses of said liposomes selected from PLGA-PEG-PLGA triblock copolymers.

In one embodiment, the present invention discloses matrices for a locally restricted but sustained delivery of matrix-embedded phosphatidylserine-presenting liposomes (PS-liposomes), containing one or more encapsulated inhibitor of DC maturation and one or more encapsulated allergens or autoantigens or peptides derived thereof (named tolerizing PS-liposomes), one or more matrix-embedded find-me signals for efficient peripheral phagocytosis by DCs and macrophages, andoptionally one or more matrix-embedded immune modulators capable of enhancing the suppressive activity of Treg cells and inhibiting effector T cell responses at the site of autoantigen or allergen presentation.

Using these components, the method of the present invention provides several important therapeutic advantages over currently available techniques. First, the matrix-based approach allows sustained local delivery of tolerizing PS-liposomes and supporting immune modulators for the period of time necessary for the development of immunologic memory upon antigen or allergen exposure whichrequires the engagement of the T cell receptor (TCR) over 12-48 hours.Second, the matrix-based technology of the present invention allows to support this approach by one or more additional immune modulators capable of addressing at the site of autoantigen or allergen presentation immune cells other than DCs or macrophages directly, resulting in enhanced suppressive activity of Treg cells and inhibition of effector T cell responses. Third, the matrix-mediated sustained delivery technology allows the application of the rapeutic agents with a short plasma half-life for the method of the present invention, thereby minimizing adverse side effects despite high local concentrations of such agents at the site of allergen or autoantigen presentation. Fourth, the matrix-mediated sustained delivery technology allows also to establish a gradient of low molecular weight eat-me signals with a short plasma half-life such as ATP and UTP for efficient peripheral phagocytosis of tolerizing PS-liposomes by dendritic cells and macrophages. Fifth, due to efficient peripheral phagocytosis mediated by find-me and the tolerance-promoting eat-me signal phosphatidylserine on the surface of tolerizing liposomes, the method of the present invention allows the application of small amounts of tolerizing PS-liposomes, which eliminates the risk of global tolerance via global bystander suppression, while the therapeutic efficacy of this approach is not affected. Finally, the matrix-based locally restricred approach allows to combine different tolerizing agents which is much more complicated if these agents are administered systemically.

In another embodiment, the present invention discloses suitable matrices for sustained local delivery of tolerizing PS-liposomes, one or more matrix-embedded find-me signals for efficient peripheral phagocytosis by DCs and macrophages, andoptionally one or more matrix-embedded immune modulators. Preferred matrices include but are not limited to biodegradable polymers which are suitable as depot for substantial quantities of tolerizing PS-liposomes, find-me signals and immune modulators, which allow the release of sufficient quantities of such agents for efficient local induction of tolerance over a prolonged period of time, and which are chemically and physically compatible with the different types of tolerizing agents. Most preferably are injectable in situ-forming gel systems which are biodegradable. Preferred in situ-forming gel systems (hydrogels) undergo a sol-gel-sol transition, which is free flowing sol at room temperature and a non-flowing gel at body temperature. Compared to other biodegradable polymers, the injectable thermo-gelling polymers possess several advantages including easy preparation, high encapsulation efficiency of bioactive preparations such as tolerizing PS-liposomes, and free of harmful organic solvents in the formulation process.

In another embodiment, the present invention discloses tolerizing phosphatidyl-L-serine (PS)-liposomes containing one or more DC maturation inhibitors and one or more allergens or autoantigens of fragments derived thereof, wherein said tolerizing PS-liposomes are capable a) to mimick the anti-inflammatory effect of apoptotic cells by PS presentation to macrophages and DCs, b) to induce allergen- or autoantigen-specific Treg cells by the generation of maturation-inhibited DCs, and c) to reduce the number of disease-relevant macrophages and DCs by induction of apoptosis via NF-κB inhibition.

In another embodiment, the present invention discloses suitable pharmacological DC maturation inhibitors for encapsulation in tolerizing PS-liposomes including but are not limited to vitamin D3 (1α,25-dihydroxyvitamin D3) and derivatives thereof; glucocorticoids such as dexamethasone (Dex), salicylates such as acetylsalicylic acid (aspirin), rapamycin (RAPA), estriol, vasoactive intestinal pepide (VIP), BAY11-7082, andrographolide, curcumin (diferuloylmethane), quercetin, and cytokines such as IL-10 and TGFβ, biological agents derived from pathogens that can also modulate immune responses, and agents for gene silencing of different pro-inflammatory molecules, such as CD40, CD80, CD86 and IL-12, wherein preferred DC maturation inhibitors include but are not limited to vitamin D3 and anlogs thereof with short plasma half-lives such as calcipotriol, glucocorticoids, and antisense oligonucleotides capable of gene silencing of different pro-inflammatory molecules including CD40, CD80, and CD86.

In another embodiment, the present invention discloses allergens and autoantigens or peptides derived thereof for encapsulation in tolerizing PS-liposomes, wherein allergens include but are not limited to natural allergens, recombinant allergens, and peptides derived thereof, and wherein preferred autoantigens of peptides derived thereof include but are not limited to those which have been identified and evaluated in previous studies.

In another embodiment, the present invention discloses find-me signals capable of triggering effective local phagocytosis, thereby enhancing the tolerance-promoting effect of tolerizing PS-liposomes, wherein suitable find-me signals are the nucleotides ATP and UTP.

In another embodiment, the present disclosure discloses additional low molecular weight immune modulators capable of enhancing the suppressive activity of Tregs, inhibiting the production of pro-inflammatory cytokines, and inhibiting the biological activity of secreted pro-inflammatory cytokines. Suitable low molecular weight immune modulators include but not limited to vitamin D3 and selected vitamin D3 analogs such as calcipotriol, glucocorticoids, aptamer-based therapeutics for the inhibition of interleukins including but not limited to IL-4, IL-5, IL-13, IL-17, IL-23, IL-25, and IL-33, low molecular weight complement inhibitors, glutathione-, salicylate- and oligonucleotide-based therapeutics for the inhibition of TNFR1-mediated pathways, and medium molecular weight proteins such as IL-4 muteinsas described in detail in patent application EP 13075040.9. Preferred are low molecular weight immune modulators which provide a relatively short serum half-life that is sufficient to be locally active upon their release from the matrix at the site of allergen or autoantigen presentation, and which allows fast removal from circulation upon diffusion and transport away from the site of allergen/autoantigen presentation.

In another embodiment, the present invention discloses pharmaceutical compositions for the treatment of allergy, asthma, RA, T1D and MS disease-related preferred DC maturation inhibitors, disease-related preferred autoantigens, and disease-related preferred additional low molecular weight immune modulators; wherein said preferred DC maturation inhibitors include but calcipotriol, glucocorticoids, and antisense oligonucleotides capable of gene silencing of different pro-inflammatory molecules including CD40, CD80, and CD86; wherein said preferred autoantigens for the treatment of MS include MOG₃₅₋₅₅.

In another embodiment, the present invention discloses preferred patient populations for the treatment of allergy, asthma, RA, T1D, and MS, wherein genetic and non-genetic risk factors as well as the emergence of diagnostic indicators and disease progression are considered, wherein in a preventive approach individuals or families at genetic risk are targeted, wherein in a preferred approach patients with early established disease are targeted, and wherein in a more preferred approach patients at genetic risk are targeted after the emergence of one or more diagnostic indicator of the disease, but prior to onset of clinical disease.

In another embodiment, the present invention discloses the application of compositions in the treatment of allergy, asthma, RA, T1D, and MS, wherein preferred compositions comprise a biodegradable PLGA-PEG-PLGA (PLGA: poly(lactic-co-glycolic acid); PEG: poly(ethylene glycol)) thermo-gelling triblock hydrogel solution containing a) tolerizing PS-liposomes with at least two, more preferably at least three different encapsulated allergens or autoantigens or fragments thereof, and one or two different encapsulated DC maturation inhibitors, b) at least one hydrogel-embedded find-me signals for efficient peripheral phagocytosis of tolerizing PS-liposomes, and c) one or two different additional immune modulators with short plasma half-lives, wherein.

In another embodiment, the present invention discloses methods for incorporating compositions into pharmaceutical formulations suitable for administration.

Specific preferred embodiments of the present invention will become evident from the following more detailed description and the claims.

### DETAILED DESCRIPTION OF THE INVENTION

In order to translate the concept of various forms of antigen-specific Tregs or antigen-loaded/antigen-exposed tolerizing DCs to a therapeutic that is more broadly applicable than adoptive transfer of autologous cell products, a liposomal approach has been developed and its efficacy has been demonstrated in a murine model of antigen-induced arthritis (Capini et al., 2009). As outlined in the foregoing, however, this approach is either associated with the risk of global tolerance via bystander suppression in various organs of the reticulo-endothelial system, or there is a need for repeated injections of small amounts of tolerizing liposomes which is not acceptable for physicians and patients.

The present invention solves these problems by disclosing matrices for a locally restricted but sustained delivery of matrix-embedded phosphatidylserine-presenting liposomes (PS-liposomes), containing one or more encapsulated inhibitor of DC maturation and one or more encapsulated allergens or autoantigens or peptides derived thereof (tolerizing PS-liposomes), one or more matrix-embedded find-me signals for efficient peripheral phagocytosis by DCs and macrophages, and optionally one or more matrix-embedded immune modulators capable of enhancing the suppressive activity of Treg cells and inhibiting effector T cell responses at the site of autoantigen or allergen presentation.

The present invention allows subcutaneous injection of small amounts of tolerizing PS-liposomes due to efficient peripheral phagocytosis mediated by find-me and the tolerance-promoting eat-me signal phosphatidylserine. Thereby, the risk of global tolerance via bystander suppression is eliminated, while the therapeutic efficacy of this approach is not affected. Furthermore, the matrix-based technology of the present invention allows to support this approach by one or more additional immune modulators capable of addressing at the site of autoantigen or allergen presentation immune cells other than DCs or macrophages directly, resulting in enhanced suppressive activity of Treg cells and inhibition of effector T cell responses.

### I. TOLERIZING PS-LIPOSOMES

In one embodiment, the present invention discloses tolerizing phosphatidyl-L-serine (PS)-liposomes containing one or more DC maturation inhibitors and one or more allergens or autoantigens of fragments derived thereof, wherein said tolerizing PS-liposomes are capable a) to mimick the anti-inflammatory effect of apoptotic cells by PS presentation to macrophages and DCs, b) to induce allergen- or autoantigen-specific Treg cells by the generation of maturation-inhibited DCs, and c) to reduce the number of disease-relevant macrophages and DCs by induction of apoptosis via NF-κB inhibition.

### I.1. Immunological effects mediated by the presentation of PS

Phosphatidylserine (PS), which is exposed on the surface of apoptotic cells, has been implicated in immune regulation. In viable cells, PS is kept exclusively on the inner leaflet of the lipid bilayer via ATP-dependent translocases. In apoptotic cells, the concentration of PS on the outer leaflet of the lipid bilayer is estimated to increase by more than 280-fold within only a few hours after induction of apoptosis. PS exposed on the surface of apoptotic cells represents the key signal for triggering phagocytosis by macrophages (for a review, see Hochreiter-Hufford and Ravichandran, 2013). This is indicated by the observation that macrophage phagocytosis of apoptotic lymphocytes was inhibited in a dose-dependent manner by PS and PS-containing liposomes, but not by liposomes containing other anionic phospholipids including phosphatidyl-D-serine (Fadok et al., 1992). Several membrane receptors including BAI1 (brain-specific angionesis inhibitor 1), Tim4 (T cell immunoglobulin and mucin domain-containing protein 4), Tim1 and Stablisin-2 have been shown to mediate uptake of apoptotic cells by directly binding PS (for a review, see Chekeni and Ravichandran, 2011).

As demonstrated by several studies, apoptotic cells presenting antigens on their surface have the capability to induce antigen-specific tolerance. For example, infusion of peptides cross-linked to the surface of apoptotic splenic leukocytes using ethylene carbodiimide has been demonstrated to be a highly efficient method for inducing antigen-specific T cell tolerance for treatment of autoimmune diseases (Jenkins and Schwartz, 1987; Getts et al., 2011). A single intravenous injection of syngeneic splenocytes coupled with encephalitogenic myelin peptides/proteins has been shown to induce antigen-specific tolerance in experimental autoimmune encephalomyelitis (e.g., Tan et al., 1991). Both the production of IL-10 and the expression of PD-L1 on macrophages upon phagocytosis of apoptotic cells have been identified as important factors for the induction of tolerance. PD-L1 mediates T cell-negative costimulation. Neither IL-10-deficient mice nor mice treated with anti-IL-10 can be tolerized with ethylene carbodiimide-fixed splenocytes (Getts et al., 2011). Furthermore, PD-L1 blockade at the time of injection of syngeneic splenocytes coupled with encephalitogenic myelin peptides/proteins abrogated tolerance induction (Getts et al., 2011).

Several studies suggest that liposomes presenting PS on the surface have the potential to mimick the anti-inflammatory effect of apoptotic cells and to inhibit immune responses of antigen-specific CD4+ T cells and B cells in vivo. For example, PS-containing liposomes specifically inhibited responses in mice to antigens as determined by decreased draining lymph node tissue mass, reduced numbers of total leukocytes and antigen-specific CD4+ T cells and decreased levels of antigen-specific IgG in blood. TGF-β appears to play a critical role in this inhibition, as the inhibitory effects of PS-containing liposomes were reversed by in vivo administration of anti-TGF-β antibodies (Hoffmann et al., 2005).

PS-containing liposomes exert also inhibitory effects on certain macrophage functions (Kornbluth, 1994). For example, PS-containing liposomes inhibited the IFN-γ-mediated induction of anti-leishmanial activity in murine macrophages (Gilbreath et al., 1985). A recent study demonstrated that after uptake of PS-containing liposomes in vitro and in vivo macrophages secrete high levels of the anti-inflammatory cytokines TGF-β and IL-10 and upregulate the expression of CD206 (mannose receptor C type 1; MRC1), concomitant with down-regulation of pro-inflammatory markers such as TNFα and the surface marker CD86 (Harel-Adar et al., 2011).CD86 (also known as B7-2) is a protein expressed on antigen-presenting cells that provides costimulatory signals necessary for T cell activation and survival.

Furthermore, inhibition of DC maturation upon exposure to large unilamellar PS-liposomes was reported (Chen et al., 2004). PS liposomes inhibited the up-regulation of HLA-ABC, HLA-DR, CD80, CD86, CD40, and CD83, as well as the production of IL-12p70 by human DCs in response to LPS. PS did not affect DC viability directly but predisposed DCs to apoptosis in response to LPS. DCs exposed to PS had diminished capacity to stimulate allogeneic T cell proliferation and to activate IFN-γ-producing CD4⁺ T cells. Furthermore, activated CTLs proliferated poorly to cognate antigen presented by DCs exposed to PS. Apparently, PS exposure provides a sufficient signal to inhibit DC maturation and to modulate adaptive immune responses (Chen et al., 2004).

### I.2. Induction of antigen-specific Treg cells by inhibitors of DC maturation

A recent study has demonstrated that egg phosphatidylcholine liposomes loaded with antigen (OVA or methylated BSA) and a lipophilic NF-κB inhibitor (curcumin, quercetin, or Bay11-7082) suppressed preexisting immune responses in an antigen-specificmanner. By targeting antigen presenting cells (APCs) via injection of loaded liposomes into mice primed with antigen or into mice suffering from antigen-induced inflammatory arthritis, antigen-specific FoxP3 (+) regulatory T cells were induced, which suppressed effector T cell responses and the clinical signs of full-blown antigen-induced arthritis (Capini et al., 2009).

NF-κB inhibitors are known to inhibit the maturation process of DCs, thereby generating tolerizing DCs which are capable of inducing regulatory T cells. If allergens or antigens are presented by tolerizing DCs, allergen- or antigen-specific Tregs are induced which have the potential to suppress allergic and autoimmune diseases.

For the present invention, various pharmacological agents capable of inhibiting maturation of DCs are suitable for incorporation into tolerizing PS-liposomes. Tolerogenic DCs (tolDCs) are phenotypically immature DCs characterized by a low expression of MHC-II as well as co-stimulatory molecules such as CD40, CD80, CD86, and a reduced production of pro-inflammatory IL-12 and increased secretion of anti-inflammatory IL-10. Inhibition of DC maturation results in DCs that do not respond to PAMPs/DAMPS and pro-inflammatory stimulation (for reviews, see Thomas, 2013; Gross and Wiendl, 2013; Van Brussel et al., 2014; Mackern-Oberti et al., 2015).

For the present invention, suitable pharmacological DC maturation inhibitors include but are not limited to vitamin D3 derivative calcipotriol; glucocorticoids such as dexamethasone (Dex), antisense oligonucleotides capable of gene silencing of different pro-inflammatory molecules, such as CD40, CD80, CD86 and IL-12 promotes a tolerogenic phenotype to DCs(for reviews, see Van Brussel et al., 2014; Mackern-Oberti et al., 2015).

It should be noted, however, that the various pharmacological agents induce different tolerogenic properties in DCs (Naranjo-Gomez et al., 2011; Boks et al., 2012). Tolerogenic DCs have differing functional capacities including their capacity to migrate toward secondary lymphoid organs and to induce Tregs, and sometimes these properties are not reflected by an immature phenotype. For example, RAPA-DCs show a rather mature phenotype, but they have a high migratory capacity (Boks et al., 2012) and promote CD4(+)CD25(high) CD127(low/negative)Foxp3(+) T cells (Naranjo-Gomez et al., 2011). Apparently, the definition of DC maturation using phenotype markers is not a distinguishing feature of tolerogenicity.

### I.2.1.Blockade of the NF-κB pathway.

Blockade of the NF-κB pathway has been extensively used to enhance the tolerogenic potential of DCs. Inhibition of NFκB promotes a tolerogenic phenotype in DCs that prevents LPS-maturation and reduces the ability to prime effector T cells.

The NF-κB family consists of five proteins: NF-κB1 (p105/p50), NF-κB2 (p100/p52), RelA (p65), RelB and c-Rel. These proteins form homo- or heterodimers that interact with specific cis-DNA sequence (κB element) to regulate a wide range of genes including those involved in immunity and inflammatory responses.

A crucial negative regulator that controls NF-κB activation is the inhibitor of κB (IκB), which binds to p65 in the cytosol to block the nuclear translocation of p65/p50 heterodimer. Phosphorylation of IκB by activated IκB kinase (IKK) initiates the ubiquitylation and eventual proteasomal degradation of IκB, and a direct consequence of IκB degradation is nuclear entry of p65/p50 to trans-activate gene expression. Thus IKK plays an essential role in NF-κB activation. The kinase activity of IKK depends on the formation of IKK complex by IKKα, β and γ subunits, which is activated upon phosphorylation by growth factors, pro-inflammatory cytokines (such as TNFα) and hormones through the TNF receptor or Toll-like receptor superfamily. IKK also phosphorylates p65 to promote its activity.

For the present invention, suitable inhibitors of the NF-κB pathway include but are not limited to vitamin D3 derivative calcipotriol and glucocorticoids.

### 1.2.2. NF-κB inhibition by vitamin D3 and derivatives thereof.

In one embodiment, the present invention uses for the generation of tolerogenic PS-liposomes low molecular weight molecules capable of inhibiting DC maturation via vitamin D-mediated pathways including calcipotriol is used for the method of the present invention. As compared to 1,25-(OH)₂D3, calcipotriol has 100-200 times less effect on calcium metabolism including activation of calcium absorption (Kissmeyer and Binderup, 1991), while in vitro effects on proliferation and differentiation on human keratinocytes are comparable (Reichrath and Holick, 2010; Binderup et al., 1991).An important advantage for the method of the present invention is the short half-life of calcipotriol in circulation which is measured in minutes (Kragballe, 1995). After i.v.injection of 10 µg/kg calcipotriol in rats, calcipotriol was detectable in serum by HPLC analysis only up to 5 min, and after i.v. injection of 50 µg/kg up to 10 min (Kissmeyer and Binderup, 1991). The rate of clearance (half-life of 4 min) was approximately 140 times higher for calcipotriol than for 1,25-(OH)₂D3. Furthermore, calcipotriol is rapidly metabolized and effects of the metabolites have been demonstrated to be 100 times weaker than those of the parent compound (Kissmeyer and Binderup, 1991). Due to these characteristics, calcipotriol has been used clinically for more than 10 years for topical treatment of psoriasis without systemic toxicity (for a review, see Plum and DeLuca, 2010).

The activity of vitamin D3 (VD3) is mediated by the vitamin D receptor (VDR), a member of the nuclear receptor superfamily. The classic VDR action model is that upon VD3 activation VDR moves into the nucleus and hetero-dimerizes with retinoid X receptor (RXR), which together binds to vitamin D-response element (VDRE) in the target gene promoter to up-regulate gene transcription. However, it has also been reported that VDR can down-regulate gene transcription by directly interacting with other regulatory proteins such as β-catenin and CREB through VDRE-independent mechanisms.

VDR signaling intrinsically suppresses NF-κB activation resulting in down-regulation of a variety of genes including IL-12, IL-8, MCP-1, PAI-1, angiotensinogen and microRNA-155. The molecular mechanism underlying 1,25(OH)2D3 regulation of NF-κB is complex. It has been reported that 1,25(OH)2D3 arrests p65 nuclear translocation, blocks NF-κB DNA binding, increases IκBα levels or stabilizes IκBα protein. It has also been shown that 1,25(OH)2D3 suppresses RelB transcription and reduces pl05/p50 and c-rel protein levels.

### 1.2.3. NF-κB inhibition by glucocorticoids.

In another embodiment, the present invention utilizes glucocorticoids for the generation of tolerogenic PS-liposomes. Glucocorticoids, such as dexamethasone and prednisone, are widely used for their anti-inflammatory and immunosuppressive properties. These agents interact with the steroid receptor to down-regulate the expression of specific genes that regulate the inflammatory process. There are several proposed mechanisms to explain the inhibitory effects of glucocorticoids on the NF-κB pathway.

The first mechanism is consistent with a role for glucocorticoids in inducing expression of IκBα to enhance the cytosolic retention of NF-κB. Dexamethasone induces the synthesis of IκBα mRNA in glucocorticoid receptor-expressing Jurkat cells and in monocytic cells, increasing the level of IκBα and resulting in the cytoplasmic retention of p65. The majority of newly synthesized IκBα induced by dexamethasone is associated with p65 in pre-existing NF-κB complexes. NF-κB is thus maintained in an inactive cytoplasmic complex so that the expression of genes involved in the pathogenesis of the immune response is reduced.

However, other mechanisms are also likely involved in glucocorticoid-mediated repression of the NF-κB pathway. For example, dexamethasone can repress IL-6 expression and p65-dependent transactivation in murine endothelial fibroblasts without changing IκB protein levels or NF-κB DNA-binding activity. Similarly, in primary endothelial cells, dexamethasone reduces NF-κB-mediated transcriptional activity without altering IκB protein levels or the nuclear translocation of NF-κB. These results indicate that in certain cell types the down-modulation of NF-κB-directed gene expression by glucocorticoids is due to other mechanisms. For example, direct protein-protein interactions between the activated glucocorticoid receptor and NF-κB can also prevent activation of this pathway.

### 1.2.4. Treatment of DCs with vitamin D3 and glucocorticoids.

DCs treated with dexamethasone (Dex) or 1α, 25-dihydroxyvitamin D3 (calcitriol; VD3) show a stable, semi-mature phenotype with intermediate expression of molecules involved in T cell activation such as MHC-II and CD86.IL-12p70 secretion was lost byVD3-DC and Dex-DC, whereas IL-10 secretion was unaffected.VD3-DC distinctly produced large amounts of TNF-α. Dex or VD3 treated DCs are resistant to maturation by pro-inflammatory stimulation without affecting IL-10 production (Unger et al., 2009; Xing et al., 2002).

Both VD3-DC and Dex-DC possessed the capacity to convert CD4(+) T cells into IL-10-secreting Treg potently suppressing the proliferation of responder T cells(Unger et al., 2009; Xing et al., 2002). However, only Treg induced by VD3-DC exhibited antigen specificity (Unger et al., 2009). VD3-DC, but notDex-DC expressed significant high levels of PD-L1 (programmed death-1 ligand), upon activation (Unger et al., 2009).

Glucocorticoids and vitamin D3 modulate DCs via distinct and additive signaling pathways (Xing et al., 2002). CombinedDEX and VD3 analogtreatment of DCs resulted in significant additive inhibition of pro-inflammatory cytokines, T-cell stimulation, chemokines, chemokine receptors,and NF-κB components (Xing et al., 2002).

### 1.2.13. Genetic manipulation of DCs.

In still another embodiment, the present invention utilizes siRNA-based approaches for the generation of tolerogenic PS-liposomes. It has been demonstrated that gene silencing of different pro-inflammatory molecules, such as CD40, CD80, CD86 and IL-12 promotes a tolerogenic phenotype to DCs with the capacity to ameliorate the clinical score of arthritis in the CIA mice model after tolDC transfer mainly by the suppression of T and B cell immune responses and expanding Treg subset (Li et al., 2012; Zheng et al., 2010).

In the EAE model, transfer of tolDCs induced by lentiviral transduction of CD40 and IL-23 specific shRNA (small hairpin RNA) decreased disease symptoms (Kalantari et al., 2014).

In non-obese diabetic (NOD) mice, transfer of tolDCs induced by antisense oligonucleotide-mediated down-regulation of costimulatory transcripts has been shown to confer diabetes-preventive properties to non-obese diabetic mouse DCs (Machen et al., 2004). A single injection of bone marrow-derived NODDCs treated ex vivo with a mixture of antisense oligonucleotides targeting the CD40, CD80, and CD86 transcripts, into syngeneic pre-diabetic female NOD mice significantly delayed the incidence of TIDM. In NOD-scidrecipients, oligonucleotide-treated NOD DC administration in cotransfer with T cells promoted an increased prevalence of CD4(+)CD25(+)CD62L(+) T cells(Machen et al., 2004).

### I.3. Induction of apoptosis by NF-κB inhibitors

In addition to the regulation of APC function, NF-κB also plays an important regulatory role in cellular survival and apoptosis, specifically in cases of infection and inflammation. NF-κB suppresses programmed cell death mediated by TNFα-induced JNK and caspase-8 activation.

A recent study has demonstrated that murine bone marrow-derived macrophages and dendritic cells (DC), as well as macrophage and DC lines, underwent rapid programmed cell death (PCD) after treatment with several IKK/NF-κB inhibitors through a TNFα-dependent mechanism. PCD was induced proximally by reactive oxygen species (ROS) formation, which causes a loss of mitochondrial membrane potential and activation of a caspase signaling cascade (Tilstra et al., 2014).

Based on these data it was speculated that APC death, in both macrophages and monocyte-derived DC, may contribute to the anti-inflammatory effects of NF-κB inhibitors observed in mammalian models of disease (Tilstra et al., 2014).However, the PCD-inducing capacity of NF-κB inhibitors can differ significantly. As demonstrated in other studies, vitamin D3 exhibited only a slight tendency to promote DC apoptosis (Naranjo-Gomez et al., 2011), while dexamethasone did not induce cell death in monocyte-derived DCs at any of the tested concentrations (Fazekasova et al., 2009).

### I.4. Encapsulation of allergens/autoantigens in PS-liposomes

For the present invention, one or more allergens or autoantigens or peptides derived thereof are encapsulated in PS-liposomes together with one or more inhibitors of DC maturation. Thereby, allergens or autoantigens are presented to macrophages and dendritic cells in the presence of agents mediating the induction of tolerance by PS-signalling and inhibitors of DC maturation.

Suitable allergens include but are not limited to natural allergens, recombinant allergens, and peptides derived thereof. For the generation of tolerizing PS-liposomes, encapsulation of a limited number of different allergens or allergen-derived peptides may be sufficient. While the induction of Treg-suppressive activity is specific and requires allergenic stimulation through the T cell receptor (TCR), the suppressive activity of Tregs is not allergen-specific. Allergen-specific Tregs can simultaneously suppress bystander responses in the same location. As a result, a wide range of immune responses can be inhibited by Tregs via bystander suppression. In principle, bystander suppression enables allergen-specific Tregs of a single specificity to suppress allergic responses despite the presence of immunoreactivity to multiple allergens.

Comparable considerations apply to treatment of autoimmune diseases with autoantigen-loaded tolerizing PS-liposomes. Most likely, however, the efficacy of autoantigen-specific therapies will depend not only on knowledge of the specific target autoantigen(s) but also on the ability to block epitope spreading at an early stage and thereby stop diversification of T cell autoreactivity. Consequently, autoantigen-specific therapies should simultaneously target previously activated autoreactive T cells and also naive autoreactive T cells specific for multiple autoantigen epitopes.

### 1.4.1. Target autoantigens in multiple sclerosis (MS).

In MS, the primary target antigens are not known for certain, but it is well accepted that proteins within the myelin sheath, such as myelin basic protein (MBP), myelin oligodendrocyte protein (MOG), and proteolipid protein (PLP), are important targets of the autoreactive immune response.

However, the target epitopes of myelin proteins differ between MS patients, and it is likely that the myelin-specific T cell reactivity may change over time. In relapsing-remitting (RR) animal models of MS, chronic demyelination leads to the generation of new T cell responses against multiple endogenous antigens, a process called epitope spreading, and these newly generated T cells are able to induce relapses, which can be inhibited by tolerance to the spread epitope.

### 1.4.2. Auto-antigens used in clinical MS trials.

In a recent successful Phase 1 trial, 7 myelin peptides (MBP₁₃₋₃₂, MBP₈₃₋₉₉, MBP₁₁₁₋₁₂₉, MBP₁₄₆₋₁₇₀, MOG₁₋₂₀, MOG₃₅₋₅₅, and PLP₁₃₉₋₁₅₄), which were previously identified as important targets of autoreactive T cells in MS, were coupled to the surface of apoptotic PBMCs (Lutterotti et al., 2013).

In another recent successful double-blind, placebo-controlled trial, 3 myelin peptides (MBP₈₅₋₉₉, PLP₁₃₉₋₁₅₁, and MOG₃₅₋₅₅) were applied transdermally as a skin patch in 30 patients with relapsing-remitting MS over the course of 1 year (Walczak et al., 2013).

Still another study has demonstrated thathuman DC pulsed with 7 myelin peptides (MBP₁₃₋₃₂, MBP₈₃₋₉₉, MBP₁₁₋₁₂₉, MBP₁₄₆₋₁₇₀, MOG₁₋₂₀, MOG₃₅₋₅₅, and PLP₁₃₉₋₁₅₄), can induce anergy in myelin-specific T cells obtained from relapsing-remitting MS patients (Raich-Regué et al., 2012). In this study, monocyte-derived DCs (MMDCs) from RR-MS patients were treated with a proinflammatory cytokine cocktail in th epresence of 1α, 25-dihydroxyvitamin-D3.

Based on these studies, application of tolerizing PS-liposomes loaded with the 7 myelin peptides MBP₁₃₋₃₂, MBP₈₃₋₉₉, MBP₁₁₁₋₁₂₉, MBP₁₄₆₋₁₇₀, MOG₁₋₂₀, MOG₃₅₋₅₅, and PLP₁₃₉₋₁₅₄ represents the most promising approach for the treatment of MS.

### 1.4.3. Target autoantigens in rheumatoid arthritis (RA).

Some of the autoantigens described are joint-derived proteins, such as type II collagen and human cartilage-derived glycoprotein HCgp39 (Tsark et al., 2002).

Other antigens are stress-associated proteins, including grp78/BiP, which is an intracellular chaperone involved in endoplasmic reticulum stress and angiogenesis in proliferative RA synovial tissue (Blass et al., 2001; Yoo et al., 2012).

Citrullinated autoantigens have emerged as a major group of post-translationally modified autoantigens in RA. Citrullination or deimination is the conversion of the amino acid arginine in a protein into the amino acid citrulline. Enzymes called peptidyl arginine deiminases (PADs) replace the primary ketimine group (=HN) by a ketone group (=O). PADs are are Ca²⁺-dependent enzymes and are activated upon intracellular Ca²⁺ flux into inflammatory settings. Arginine is positively charged at neutral pH, whereas citrulline is uncharged. As a result, citrullination can substantially affect the protein structure and function.

Approximately 70% of RA patient sera contain autoantibodies reactive to a variety of citrullinated peptide antigens (ACPA) (Vossenaar & Venrooij, 2004). These autoantigens include vimentin, fibrinogen, collagen type II, α-enolase, clusterin, histones and peptidyl arginine deiminase-4 itself (Anzilotti et al., 2010; Masson-Bessiere et al., 2001; Sokolove et al., 2012). Citrullinated autoproteins are found in inflamed RA joints, but are not specific to RA.Anti-citrullinated protein antibodies (ACPA) may predate the onset of clinical RA by up to15 years.

The major histocompatibility complex (MHC) contributes about one-third of the genetic susceptibility to RA. Specific RA-associated human leukocyte antigen (HLA)DR alleles encode a conserved amino acid sequence in the HLA-DR antigen-binding groove, known as the shared epitope (SE). Antibodies to citrullinated peptide antigens (ACPA), reflecting autoreactivity to citrullinated autoantigens, are much more likely to occur in patients with the HLA-DR SE (van der Helmvan Mil et al., 2007).

Several studies have demonstrated citrullinated autoantigen-specific T-cell autoimmunity in RA patients carrying HLA susceptibility alleles (Law et al., 2012; von Delwing et al., 2010; Snir et al., 2011). It was found that proinflammatory cytokines were secreted by peripheral blood (PB) CD4⁺ T cells of RA patients and healthy controls, in response to citrullinated but not unmodified peptides in the context of the HLA-SE sequence. RA patient T cells secreted a broader range of cytokines than healthy control T cells. Of the peptides tested, citrullinated aggrecan was most immunogenic (Law et al., 2012).

### 1.4.4. Autoantigens used in clinical RA trials.

Antigens used in tolerance trials include type II bovine or chicken collagen, HCgp39, lyophilised *Escherichia coli* extract, dnaJp1, and citrullinated peptides (for a review, see Thomas, 2013).

Oral administration of chicken or bovine type II collagen was safe when administered to patients with RA. While some clinical improvement was noted in open-labelled studies, placebo-controlled trials found no statistically significant improvement in collagen-fed patients, including among patients with early RA.

Oral administration ofhuman gp39 was also trialled by two companies, but with little evidence of efficacy.

Oral administration of lyophilised *E. coli* extract containing several bacterial heat shock proteins with immunomodulatory properties, showedin a placebo-controlled trial clinical efficacy equivalent to D-penicillamine. This extract is more likely to be a nonspecific immunomodulator than to induce antigen-specific tolerance.

Oral administration of the 15-mer synthetic peptide dnaJp1 (QKRAAYDQYGAAFE), derived from HSP dnaJ (Albani et al., 1995), was trialled in phase I and phase II clinical trials in RA. This bacterial heat shock protein sequence has been proposed to be cross-reactive with corresponding self-peptides in RA because it is homologous with the HLA-DR SE sequence. Oral dnaJp1 had an excellent safety profile and demonstrated immune modulatory effects. Whereas patients generally made Th1-type T-cell cytokine and proliferative responses to dnaJp1 at baseline, dnaJ-specific proliferation and IFNγ decreased and IL-4 and IL-10 increased after the treatment. In a placebo-controlled phase II clinical trial, the dnaJp1 treated group showed statistically significant improvement in American College of Rheumatology scores after 6 months of daily oral dnaJp1 peptide, associated with increased expression of regulatory molecules and decreased secretion of TNF in PB (Koffeman et al., 2009).

Recently, a Phase I clinical trial in RA patients has evaluated the feasibility and safety of autologous tolDC therapy. TolDCs were generated by the tolerogenic agent BAY11-7082, an NFκB inhibitor, and loaded with citrullinated peptides (cit-vimentin, cit-fibrinogen, cit-fibrinogen, cit-collagen type II). RA patients received only one intradermic dose of 1 × 10⁷ tolDCs. Only mild adverse effects such as headache and minimal changes in hematology parameters were reported while the expected therapeutic effect could already be observed in some patients (Thomas et al., 2011).

Based on these studies, application of tolerizing PS-liposomes loaded with the 15-mer synthetic peptide dnaJp1 and citrullinated peptides derived from cit-vimentin, cit-fibrinogen, cit-fibrinogen, and cit-collagen type II, represents the most promising approach for the treatment of RA.

### 1.4.5. Target auto-antigens in type 1 diabetes (T1D).

Triggers of islet autoimmunity have not been identified. The only markers of islet autoimmunity are islet autoantibodies to insulin, GAD65 (glutamic acid decarboxylase), IA-2 (islet antigen 2; tyrosine phosphatase), and the ZnT8 transporter (zink transporter 8, localized on the membrane of insulin secretory granules) (for a review, see Lenmark and Larsson, 2013).

### 1.4.6. Autoantigens used in clinical T1D trials.

The first trial ever attempted to preserve β-cell function was based on intensive insulin therapy with continuous insulin infusion delivered by an external artificial pancreas, termed the 'Biostator' (Shah et al., 1989). Compared to the conventionally-treated group, the experimental group showed preservation of β-cell function with an increase in C-peptide levels at 1 year post-treatment; however, all T1D individuals remained insulin-dependent (Shah et al., 1989).

Two mainly large trials were performed, the DPT-1 trial, in which parental insulin was administered to individuals with positive autoantibodies, and the ENDIT trial in which individuals with positive autoantibodies were enrolled and received nicotinamide. Both trials failed to show any significant effect on halting the progression of the disease (Gale et al., 2004; Schatz and Bingley, 2001; Skyler et al., 2005).

The administration of oral insulin to first- and second-degree relatives of individuals with T1D at high genetic risk for developing T1D, showed some beneficial (Skyler et al., 2005).

Therapy of T1D individuals with immunomodulatory peptide DiaPep277 (derived from hsp60 protein) resulted in preservation of stimulated C-peptide for 10 months post-treatment. Furthermore, the DiaPep277 group required less exogenous insulin than the placebo group (Raz et al, 2001). Two other randomized trials using DiaPep277 reported stability of C-peptide levels compared to placebo (Schloot et al., 2007). DiaPep277 phase III or known as DIA-AID 1 trial, showed some preservation of β-cell function and an improved glycemic control in T1D individuals (Hegele et al., 2013).

Therapy with GAD-alum (glutamic acid decarboxylase formulated in alum) in subjects with T1D within 6 months from diagnosis also showed preservation of stimulated C-peptide levels (Ludvigsson et al., 2008). However, phase 2 and 3 trials of GAD-alum therapy failed to confirm the preliminary observation (Wherrett et al., 2011) and (Ludvigsson et al., 2012).

Therapy with DNA plasmid encoding proinsulin (BHT-3021) showed promising results. Proinsulin is a major target of the adaptive immune response in T1D. 80 subjects over 18 years of age who were diagnosed with T1D within the past 5 years were randomized 2:1 to receive intramuscular injections of BHT-3021 or BHT-placebo, weekly for 12 weeks, and then monitored for safety and immune responses in a blinded fashion. No serious adverse events related to BHT-3021 were observed. C-peptide levels improved relative to placebo at all doses. Proinsulin-reactive CD8⁺ T cells, but not T cells against unrelated islet or foreign molecules, declined (Roep et al., 2013).

Tolerogenic DCs induced ex vivo were administered in a recent Phase I randomized placebo-controlled trial in patients with T1D (Giannoukakis et al., 2011). TolDCs were generated in GM-CSF/IL-4 conditions with anti-sense oligonucleotides for the co-stimulatory molecules CD40, CD80, and CD86 (Machen et al., 2004). The administration consisted in an intradermal injection of 1 × 10⁷ autologous tolDCs once every two weeks for two months. The tolDC transfer was well-tolerated, with no adverse effects after one year. Immune cell counts in blood as well as the immune response in allogeneic mixed lymphocyte reaction were unaffected, suggesting the absence of systemic immunosuppression.

The rationale for the latter approach is based on the many studies demonstrating the effectiveness of CD80/CD86-CD28 blockade in generating immune hypo-responsiveness to alloantigens and in preventing autoimmunity (e.g., Sayegh et al., 1995; Woodward et al., 1998).For full activation of naive CD4(+) T lymphocytes to occur, twosignals are required. The first is the presentation of the antigen to the T cell receptor (TCR) in the context of class II MHC on DC. This will cause the responding T cell to up-regulate the CD154 molecule (CD40 ligand) to its cell surface, thereby activating the initiation of the second signal. In this process of co-activation, CD154 will interact with the CD40 molecule at the surface of the APC resulting in the up-regulation of CD80 and CD86 at the cell surface of the APC. Immediately thereafter, CD80 and CD86, acting as the second signal, in the process of costimulation, will engage the CD28 molecule on the T cell resulting in its full activation. In the absence of the interactions between CD80, CD86, and CD28, the T cell willeither enter a state of functional silence, termed anergy, or will beprimed for apoptosis, perhaps in a CD95-CD95L (Fas-FasL)-dependent manner (Lu et al., 1997).

Based on these studies, application of tolerizing PS-liposomes loaded with proinsulin, GAD and HSP60-derived peptides represents the most promising approach for the treatment of T1D. Alternatively, tolerizing PS-liposomes loaded with anti-sense oligonucleotides for the co-stimulatory molecules CD40, CD80, and CD86represent also promising therapeutics for the treatment of T1D.

### I.5. Methods for the preparation of tolerizing PS-Liposomes

For the present invention, a variety of liposomal carrier systems are applicable for the generation of tolerizing PS-liposomes including conventional liposomes, ethosomes, niosomes, and elastic liposomes (the initial formulation approach being termed transferosomes). Preferred for the method of the present invention are conventional PS-liposomes.

Conventional PS-liposomes are composed of PS and other phospholipids such as phosphatidylcholine (PC) from soybean or egg yolk, with or without cholesterol (CH). The most common applied PS is derived from bovine brain, but other PS sources and synthetic PS preparations such as 1-palmitoyl-2-oleyl-*sn-*3-glycerophosoho-L-serine or 1,2-distearoyl-sn-3-glycero-phosoho-L-serine are also suitable. Cholesterol may be used to stabilize the system. For the preparation of conventional PS-liposomes various lipid mixtures containing PS, PC and, optionally, CH are applicable including but not limited to lipid mixtures comprising molar ratios of PS:PC of 30:70 (Gilbreath et al., 1985) or 50:50 (Fadok et al., 2001) for PS-liposomes without cholesterol and molar ratios of PS:PC:CH of 1:1:1.33 (Harel-Adar et al., 2011) or 30:30:40 (Hoffmann et al., 2005) for PS-liposomes with cholesterol. As demonstrated recently, however, efficient uptake by phagocytes can also be achieved with liposomes containing PS as low as 6 mol% (Geelen et al., 2012).

Conventional liposomes can be prepared in several ways. Most frequently, a film hydration method is employed, where a thin layer of lipid is deposited on the walls of a container by evaporation of a volatile solvent. An aqueous solution containing the molecule to be entrapped is added at a temperature above the transition temperature of the lipids, resulting in the formation of multilamellar vesicles. These systems contain several lipid bilayers surrounding the aqueous core. Further processing by sonication or filter extrusion generates large unilamellar vesicles (LUV, 1-5 µm diameter), or small unilamellar vesicles (LUV, 0.1-0.5 µm diameter). PS-liposomes with 1 µm diameter have been shown to trigger efficient uptake by macrophages (Harel-Adar et al., 2011).

For the present invention, one or more inhibitors of DC maturation and one or more allergens or autoantigens or peptides derived thereof are encapsulated in the liposomal carrier. Liposomes have two compartments, an aqueous central core, and a lipophilic area within the lipid bilayer. Hydrophilic molecules such as hydrophilic antigens or allergens or peptides thereof can be incorporated into the inner aqueous volume, while hydrophobic molecules such as lipophilic inhibitors of DC maturation can be entrapped in the lipid bilayers. Non-entrapped material is removed by centrifugation or size exclusion chromatography.

PS-liposomes which contain one or more entrapped inhibitors of DC maturation and one or more antigen or allergenattached to the liposomal surface, or one or more fragments thereofattached to the liposomal surface, are also suitable for the present invention. For the attachment of allergens and antigens of fragments thereof to the liposomal surface suitable surface conjugation methodologies include but are not limited to reactions between activated carboxyl groups and amino groups yielding amide bonds, reactions between pyridyldithiols and thiols yielding disulphide bonds, and reactions between maleimide derivatives and thiols yielding thioether bonds. Other approaches also exist, such as those that yield carbamate bonds via the reaction of p-nitrophenylcarbonyl groups and amino groups (for a review, see Torchilin, 2005). All of these conjugation reactions can be used to directly attach ligands to the liposomal surface (e.g., via phosphatidylethanolamine) or to attach ligands to liposomes via spacer molecules such as polyethylene glycol (PEG) spacer molecules (e.g., via PEGylated phosphatidylethanolamine).

### II.MATRICES FOR SUSTAINED LOCAL DELIVERY OF TOLERIZING AGENTS

Effective generation of tolerizing DCs and supportive inhibition of effector T cell responses at the site of allergen or autoantigen presentation according to the method of the present invention requires a locally restricted, but sustained delivery of tolerizing PS-containing liposomes in combination with one or more find-me signals for efficient peripheral phagocytosis by DCs and macrophages.

In one embodiment, the present invention discloses suitable matrices for sustained local delivery of a) tolerizing PS-containing liposomes capable of generating tolerizing DCs in vivo, b) one or more find-me signals for efficient peripheral phagocytosis by DCs and macrophages, andoptionally c) one or more immune modulators capable of enhancing the suppressive activity of Treg cells and inhibiting effector T cell responses at the site of autoantigen or allergen presentation. Preferred matrices allow a) to embed sufficient quantities of tolerizing PS-containing liposomes, one or more find-me signals, and individual combinations of additional immune modulators, allow b) a sustained release of sufficient quantities of the embedded components for at least two days, and c) are chemically and physically compatible with all embedded components.

### II.1. Biodegradable polymers

In one specific embodiment of the invention, biodegradable polymers are used for a sustained delivery of tolerizing PS-liposomes, one or more find-me signals and, optionally, individual combinations of additional immune modulators. Possible biodegradable polymers approved by FDA and used in clinical trials, include but are not limited to poly(D,L-lactic acid), poly(lactic-co-glycolic acid) (PLGA), and copolymers of L-lactide and D,L-lactide. An important characteristic of such polymers is their ability to be applied locally. All FDA approved polymers have been studied extensively for their biocompatibility, toxicology, and degradation kinetics. Furthermore, these polymers have been shown to release embedded therapeutics for several hours up to 40 weeks in vitro and several weeks in vivo.

### II.2. Biodegradable thermogelling hydrogels

In a preferred specific embodiment, injectable in situ-forming gel systems which are biodegradable, are used for sustained delivery of PS-liposomes, one or more find-me signals and, optionally, individual combinations of additional immune modulators (for a review, see Ruel-Gariepy and Leroux, 2004). Preferred in situ-forming gel systems (hydrogels) undergo a sol-gel-sol transition, which is a free flowing sol at room temperature and a non-flowing gel at body temperature. Compared to other biodegradable polymers, the injectable thermo-gelling polymers possess several advantages including easy preparation, high encapsulation efficiency of bioactive molecules including therapeutic proteins, and free of harmful organic solvents in the formulation process (Qiao et al. 2005).

Useful for the method of the present invention are biodegradable thermogelling block polymers which are based on monomethoxy poly(ethylene glycol) (MPEG) including but not limited to a) diblock copolymers consisting of MPEG and poly(ε-caprolactone) (PCL) (Hyun et al., 2007), b) MPEG-*b-*(PCL-ran-PLLA) diblock copolymers (Kang et *al.,* 2010), and c) diblock copolymers consisting of MPEG and PLGA (Peng et al., 2010). MPEG copolymers containing PCL provide the advantage that they do not create an acidic environment upon biodegradation in contrast to MPEG copolymers containing PLLA and PLGA (Hyun et al., 2007).

According to the present invention biodegradable thermogelling triblock polymers are used, namely) PLGA-PEG-PLGA (Qiao et al., 2005). Various biodegradable thermogelling triblock polymers made up of PLGA and PEG are disclosed in patent application WO 99/18142. At lower temperatures, hydrogen bonding between hydrophilic PEG segments of the copolymer chains and water molecules dominate in aqueous solutions, resulting in the dissolution of these copolymers in water. As the temperature increases, the hydrogen bonding becomes weaker, while hydrophobic forces of the hydrophobic segments such as PLGA segments are getting stronger, leading to sol-gel transition. PEG, PLGA and PCL are well-known FDA-approved biodegradable and biocompatible materials which have been widely used in the biomedical field.

### II.5. Liposome-thermogelling hydrogel formulations

For a sustained local delivery of tolerizing PS-liposomes according to the method of the present invention, different liposome-hydrogel formulations are suitable (e.g., Xing et al., 2014; Nie et al., 2011).

In a preferred specific embodiment, thermo-sensitive copolymeric hydrogels composed of PLGA-PEG-PLGA are used for sustained delivery of PS-liposomes, one or more find-me signals and, optionally, individual combinations of additional immune modulators. As demonstrated in a recent study, liposome-loaded PLGA-PEG-PLGA hydrogels exhibit still reversible thermo-sensitive properties (Xing et al., 2014). However, its sol-gel and gel-precipitate transition temperatures decreased with increasing liposome concentration. Most important, the particle size of free liposomes and and those released from the PLGA-PEG-PLGA hydrogels were found to be close regardless of particle size of liposomes, indicating that the liposomes were stable in the hydrogel and intact liposomes could be released (Xing et al., 2014).

### II.6. Stability of thermo-gelling polymers

The various biodegradable thermo-gelling polymers provide different stability characteristics. Therefore, most preferred biodegradable thermo-gelling polymers for the method of the present invention are those which maintain their structural integrity for a few days but do not remain in the body for more than a month.

In a specific embodiment of the present invention, the rate of PLGA-PEG-PLGA hydrogel erosion can be modified by altering the molar ratio of DL-lactide/glycolide in the PLGA segment. The DL-lactide moiety is more hydrophobic than the glycolide moiety. Therefore, by increasing the molar ratio of DL-lactide/glycolide in the PLGA segment of PLGA-PEG-PLGA triblock copolymers, more stable hydrogels are formed due to stronger hydrophobic interactions among the copolymer molecules (Qiao et al. 2005).

### II.7. Sustained release of therapeutics from thermogelling polymers

Several of the biodegradable thermogelling polymers have been analyzed for their ability to mediate sustained release of proteins. Although different proteins are likely to affect the release behavior of each copolymer in individual ways, characterization of the release of model proteins such as bovine serum albumin (BSA) provides important information.

Using composite hydrogels containing different percentages of PECE and Pluronic F127, the in vitro release behavior of BSA proved to be dependent on the hydrogel composition, initial BSA loading amount, and hydrogel concentration (Gong et al., 2009b). Sustained release of BSA above 15 days was achieved with a composite hydrogel containing 60% PECE and 40% Pluronic F127, loaded with 4 mg BSA in the presence of 30wt% hydrogel. Using MPEG-PCL copolymers, sustained in vitro release of BSA proved to be above 20 days, and under in vivo conditions (after subcutaneous injection into rats) sustained release lasted for more than 30 days (Hyun et al., 2007).

While for most clinical applications a sustained release of therapeutic drugs from biodegradable thermogelling polymers over a period of several weeks is desirable, the method of the present invention does not require such an extended sustained release of active substance since the development of immunologic memory requires the engagement of the T cell receptor (TCR) for a period of only 1 to 2 days. Therefore, preferred are biodegradable thermo-gelling polymers which deliver TNFR1 inhibitors for a limited period only which does not exceed a week.

In a preferred specific embodiment, triblock copolymers based on PLGA-PEG-PLGA triblock copolymers represent one example of hydrogels providing advantageous degradation and release kinetics for the method of the present invention. As demonstrated in a recent study, the release of insulin from PLGA-PEG-PLGA triblock copolymers lasted for approximately 4 days with an initial burst effect during the first day (Choi et al, 2003). The initial burst effect may be advantageous in the initial phase by the release of find-me signals for effective phagocytosis tolerogenic PS-liposomes as well as for effective inhibition of effector T cell responses at the site of autoantigen or allergen presentation. However, the release kinetics from PLGA-PEG-PLGA triblock copolymers can be modified according to the requirements of individual therapeutic approaches. For example, modifying the copolymer composition and/or increasing the polymer concentration (e.g., from 25% (w/v) to 30% (w/v)) has been shown to decrease the burst release and to extend the release of proteins (Singh et al., 2007).

Although Poloxamer gels are not biodegradable, Poloxamer 407 (trade name Pluronic F127) gels represent also an example of thermogelling polymers providing advantageous degradation and release kinetics for the method of the present invention. Although under in vitro conditions the release of BSA from Poloxamer 407 gels proved to be almost complete within 1 day, the sustained release of BSA under in vivo conditions (after subcutaneous injection into rats) lasted for 3 days (Hyun et al., 2007).

### II.8. Safety aspects of PEG/PLA/PLGA-based hydrogels

Preferred biodegradable polymers include but are not limited to poly(ethylene glycol) (PEG), poly(D,L-lactic acid) (PLA), copolymers of L-lactic acid and D,L-lactic acid, poly(glycolic acid), and poly(lactic-co-glycolic acid) (PLGA). The use of these polymers as sustained-release protein delivery systems has been studied extensively (for a review, see Pai et al., 2009) and they represent the most compelling biodegradable polymers for depot systems due to their inclusion in the FDA's General Recognized as Safe (GRAS) list for use in medical devices and drug formulations (FDA, http://www.fda.gov/Food/IngredientsPackagingLabeling/GRAS/SCOG S/default.htm).

### III. FIND-ME SIGNALS FOR ENHANCED PERIPHERAL PHAGOCYTOSIS

Effective local uptake of tolerizing PS-liposomes by dendritic cells and macrophages in subcutaneous tissues requires the presence of released find-me signals. For example, apoptotic cells are quickly recognized and removed by phagocytes, which can be either neighboring healthy cells or professional phagocytes recruited to the site of apoptotic cell death. Phagocytes are extremely efficient in sensing and detecting the dying cells at the earliest stages of apoptosis. This is a result of find-me signals released from apoptotic and the exposure of eat-me signals on apoptotic cells. In contrast to apoptotic cells, however, tolerizing PS-liposomes do not release find-me signals.

In the recent past, several find-me signals released from apoptotic cells have been identified (for a review, see Ravichandran, 2011). In one embodiment, the present invention utilizes these find-me signals capable of triggering effective local phagocytosis including but not limited to fractalkine (chemokine CXC3CL1), lysophosphatidylcholine (LPC), sphingosine-1-phosphate (S1P) and the nucleotides ATP and UTP. Both nucleotides have been described as non-redundant find-me signals released by apoptotic cells (Elliott et al., 2009).UTP acts only on P2Y-family receptors and UDP produced via degradation of released UTP by extracellular enzymes has been shown to promote phagocytic activity via the P2Y6 nucleotide receptor. In contrast, ATP acts on P2X- and P2Y-family receptors, whereas ADP produced via degradation of released ATP by extracellular enzymes acts only on P2Y-family receptors (for a review, see Gombault et al., 2013).

At concentrations of more than 1 µM, ATP acts as a danger signal via activation of the nucleotide receptor P2X7 (EC₅₀>100 µM), which in turn leads to activation of the inflammasone and release of pro-inflammatory cytokines. However, at lower concentrations, ATP activates receptors mediating chemotaxis such as P2Y2 (EC₅₀<1 µM). Furthermore, it has been demonstrated that at lower concentrations ATP exerts anti-inflammatory effects by suppressing the secretion of pro-inflammatory cytokines and promoting the release of of anti-inflammatory cytokines (for a review, see Chekeni and Ravichandran, 2011).

Preferred are find-me signals which can be embedded in substantial quantities in matrices selected for controlled delivery of PS-liposomes, which are chemically and physically compatible with such matrices, and which can be released in sufficient quantities from such matrices over a period of one to two days.

In a preferred embodiment, equimolar quantities of ATP and UTP are employed as find-me signals. Using a transwell migration assay, both nucleotides have been demonstrated to effect maximal migration (approximately a threefold increase) of phagocytesat a concentration of about 100 nM (Elliott et al., 2009). In anotherembodiment, one find-me signal selected from UTP, ATPor UDP is employed. For the method of the present invention it is important to restrict the concentration of ATP and UTP to the lower nanomolar range since extracellular nucleotides at higher concentrations (more than 1 µM, for example, by necrotic cells) are considered pro-inflammatory (Kono and Rock, 2008).

### IV. ADDITIONAL IMMUNE MODULATORS FOR LOCAL SUPPRESSION OF EFFECTOR T CELL RERSPONSES

DCs have a key role in the induction and activation of both effector T cells and Tregsand via phagocytosis of tolerizing PS-liposomes according to the method of the present invention DCs are used to initiate suppression and redirection of immune responses in an antigen/allergen-specific manner. However, in an inflammatory environment tolerizing DCs may require additional support to be most effective. Effector T cells and other inflammation-associated cells producing pro-inflammatory cytokines at the site of allergen/autoantigen presentation are likely to counteract the induction of tolerance. Therefore, application of additional immune modulators capable of directly inhibiting inflammatory responses by effector T cells and other cells can be assumed to enhance the tolerizing effect of allergen/autoantigen-loaded PS-liposomes.

In one embodiment, the present invention discloses low molecular weight immune modulators capable of a) enhancing the suppressive activity of Tregs, b) inhibiting the production of pro-inflammatory cytokines, and c) inhibiting the biological activity of secreted pro-inflammatory cytokines.

In another embodiment, the present invention discloses methods for restricting high local concentrations of said low molecular weight immune modulators mainly to the site of allergen/autoantigen presentation to reduce adverse effects due to interaction of the immune modulators with targets distal from the site of allergen or antigen presentation. Preferred are low molecular weight immune modulators which provide a relatively short serum half-life that is sufficient to be locally active upon their release from a depot at the site of allergen or autoantigen presentation, and which allows fast removal from circulation upon diffusion and transport away from the site of allergen/autoantigen presentation.

Preferred therapeutics providing such desired characteristics include a) calcipotriol, b) glucocorticoids, c) antisense oligonucleotides capable of gene silencing of different pro-inflammatory molecules including CD40, CD80, and CD86.

### IV.1.Vitamin D3 and selected vitamin D3 analogs.

Vitamin D, in particular the biologically active metabolite 1α, 25-dihydroxyvitamin D3 (calcitriol; 1,25-(OH)₂D3), is a pleiotropic hormone exerting a variety of biological effects including the regulation of calcium, bone and mineral metabolism, as well as the modulation of immune responses by promoting both directly and indirectly regulatory T cell populations (for reviews, see Hart et al., 2011; Chambers and Hawrylowicz, 2011; Fletcher et al., 2012).

In a preferred specific embodiment, calcipotriol is used for the method of the present invention. As compared to 1,25-(OH)₂D3, calcipotriol has 100-200 times less effect on calcium metabolism including activation of calcium absorption and bone calcium mobilization (Kissmeyer and Binderup, 1991). An important advantage for the method of the present invention is the short half-life of calcipotriol in circulation which is measured in minutes (Kragballe, 1995). The rate of clearance (half-life of 4 min) was approximately 140 times higher for calcipotriol than for 1,25-(OH)₂D3. Furthermore, calcipotriol is rapidly metabolized and effects of the metabolites have been demonstrated to be 100 times weaker than those of the parent compound (Kissmeyer and Binderup, 1991).

### IV.2.Glucocorticoids

The glucocorticoid receptor (GR) is a member of the steroid-hormone-receptor family of proteins. It binds with high affinity to cortisol. The bound cortisol promotes the dissociation of molecular chaperones, including heat-shock proteins, from the receptor. Within the cell, cortisol acts in three ways. First, the cortisol-glucocorticoid receptor complex moves to the nucleus, where it binds as a homodimer to DNA sequences called glucocorticoid-responsive elements. The resulting complex recruits either coactivator or corepressor proteins that modify the structure of chromatin, thereby facilitating or inhibiting assembly of the basal transcription machinery and the initiation of transcription by RNA polymerase II. Second, regulation of other glucocorticoid-responsive genes involves interactions between the cortisol-glucocorticoid receptor complex and other transcription factors, such as NF-κB. The third mechanism is glucocorticoid signalling through membrane-associated receptors and second messengers (so-called nongenomic pathways) Evidence indicates that the glucocorticoid receptor inhibits inflammation through all three mechanisms: direct and indirect genomic effects and nongenomic mechanisms.

Human glucocorticoid receptor (GR) messenger RNA (mRNA) has alternative splice variants.The glucocorticoid receptor α isoform binds cortisol, DNA, and other transcription factors, thereby modifying transcriptional activity of target genes. Glucocorticoid receptor β protein formshomodimers that bind DNA, but it does not bind any ligands examined so far and fails to activate transcription. Glucocorticoid receptor β can also form heterodimers with glucocorticoid receptor α and interfere with the function of this protein. The relative levels of glucocorticoid receptor α and β in a cell influence the cell's sensitivity to glucocorticoid, with higher levels of glucocorticoid receptor β leading to glucocorticoid resistance.The inflammatory cytokines TNFα and IL-1 can selectively up-regulate the levels of glucocorticoid receptor β, suggesting its role in inflammation.

### IV.2.1. Anti-inflammatory mechanisms of glucocorticoids.

Glucocorticoids and the glucocorticoid receptor regulate a complex network that inhibits a variety of inflammatory pathways via several mechanismssuch as expression of anti-inflammatory proteins, induction and inhibition of cytokines, inhibition of inflammatory receptors, reduced expression of adhesion molecules, and the induction of Tregs(for reviews, see Barnes, 2001; Rhen and Cidlowski, 2005; Longui, 2007; Robinson, 2010).

For the application of glucocorticoids as additional immune modulator it is important that the immunosuppressive and anti-inflammatory effects of glucocorticoids affect in addition to dendritic cells also various other immune cells including T cells, macrophages, eosinophils, mast cells, and neutrophils. However, there are marked differences in the response ofdifferent cells and of different cytokines to the inhibitory action of glucocorticoids, and these differences may depend on the relative abundance of transcription factors within different cell types. Thus, in alveolar macrophages and peripheral blood monocytes, GM-CSF secretion is more potently inhibited by corticosteroids than IL-1β or IL-6 secretion.

### Inhibition of NF-κB and AP-1.

The repression of NF-*κ*B- and activator protein-1 (AP-1)-dependent transcription is a major component of the glucocorticoid-mediated inhibition of gene expression. Both transcription factors play an important role in the induction of pro-inflammatory factors.

NF-κB stimulates the transcription of cytokines, chemokines, cell-adhesion molecules, complement factors, and receptors for these molecules. NF-κB also induces the transcription of cyclooxygenase 2, an enzyme essential for prostaglandin production. Prostaglandins are derived from arachidonic acid. They both sustain homeostatic functions and mediate pathogenic mechanisms, including the inflammatory response.

AP-1 (activator protein-1) is a transcription factor which is a heterodimeric protein composed of proteins belonging to the c-Fos, c-Jun, ATF (activating transcription factor) and JDP(jun dimerization partner) families. AP-1, functioningas a proinflammatory transcription factor, trans-activates a number of genes that are expressed in inflammation.

The glucocorticoid receptor (GR) inhibits AP-1 target gene transcription.The cross-talk between the glucocorticoid receptor and AP-1 is mutual in that c-Fos, c-Jun, and JunD are blocked by ligand activated glucocorticoid receptor and, vice versa, glucocorticoid receptor function is impaired by overexpressed c-Fos and c-Jun.

### Expression of anti-inflammatory proteins.

Glucocorticoids suppress inflammation by increasing the synthesis of anti-inflammatory proteins. Forexample, corticosteroids increase in several cells the synthesis ofannexin I (also called lipocortin-1), a 37-kDa protein that has an inhibitoryeffect on phospholipase A2(PLA2) and, thereby, inhibits the production of arachidonic acid-derived eicosanoids (i.e., prostaglandins, thromboxanes, prostacyclins, and leukotrienes). Other anti-inflammatory proteins induced by glucocorticoids include IL-1receptor antagonist (which inhibits the binding of IL-1 to its receptor), SLPI (which inhibits proteases, suchas tryptase), neutral endopeptidase (which degradesbronchoactive peptides such as kinins), CC-10 (animmunomodulatory protein), the inhibitor of NF-κB(IκB-α), and MAPK phosphatase I. Glucocorticoid-induced MAPK phosphatase 1 dephosphorylates and inactivates Jun N-terminal kinase, thereby inhibiting c-Jun-mediated transcription. Phosphorylated c-Jun homodimers and c-Jun-Fos heterodimers bind DNA sequences called activator protein 1 response elements and induce the transcription of inflammatory and immune genes.

### Induction and inhibition of cytokines.

Glucocorticoids are able to inhibit the transcription of many pro-inflammatory cytokines, such asIL-2andIL-12, INFγ and TNFα. These inhibitory effects are due, at least in part, to an inhibitory effect on the transcription factors that regulate induction of thesecytokine genes, including AP-1 and NF-κB. For example, eotaxin, which is important in selective attraction of eosinophils from the circulation into the airways, is regulated in part by NF-kB, and its expression in airway epithelial cells is inhibited by glucocorticoids. Furthermore, glucocorticoids inhibit IL-5, at least in part, by inhibiting the AP-1 component of NF-AT (nuclear transcription factor of activated T cells).

On the other side, glucocorticoids induce transforming growth factor (TGFβ) secretion, which is able to reduce lymphocyte T activation and cell proliferation, and the expression of IL-10 (an anti-inflammatory cytokine). Glucocorticoid-induced expression of IL-10 is important for the treatment of allergic diseases, since in macrophages from asthmaticpatients the expression of IL-10 is decreased, resulting in an increased expression of several inflammatory genes.

### Inhibition of adhesion molecules.

Theexpression of adhesion molecules such as vascular adhesion molecules (VCAM-1), intercellular adhesion molecules (ICAM), and E-selectin, which are important for the trafficking ofinflammatory cells to sites of inflammation, are inhibited by glucocorticoids at the level of gene transcription.

### Inhibition of inflammatory receptors.

A number of receptors involved in the regulation of inflammatory gene, are also inhibited by glucocorticoids. For example, the gene for the NK₁-receptor, which mediates the inflammatory effects of tachykinins in the airways, has an increased expression in asthma and is inhibited by corticosteroids, probably via an inhibitory effect on AP-1. Glucocorticoids also inhibit the transcription of the NK₂-receptor, which mediates the bronchoconstrictor effects of tachykinins. Furthermore, glucocorticoids inhibit the expression of the inducible bradykinin B₁-receptor and bradykinin B₂-receptor.

### Induction of apoptosis.

The immunosuppressive and anti-inflammatory effects of glucocorticoids are also based in part on the induction of apoptosis of certain inflammatory cells. For example, dexamethasone was shown to preferentially deplete CD4(+) effector cells while sparing Treg cells (Chen et al., 2004b). Dexamethasone-induced apoptosis was also observed in B cells (Andreau et al., 1998), preferentially in B-2 cells, while B-1 cells are spared (Chen et al., 2014). Furthermore, glucocorticoids induce apoptosis in eosinophils. Eosinophil survival is dependent on the presence of certain cytokines, such as IL-5 and GM-CSF. Exposure to glucocorticoids blocks the effects of these cytokines and leads to apoptosis. By contrast, glucocorticoids decrease apoptosis in neutrophils and thus extend their survival (for a review, see Barnes, 2001).

Promotion of peripheral Treg cell production and upregulation of FoxP3 and IL-10 expression in Treg cells.

For the method of the present invention it is important that glucocorticoids also promote or initiate differentiation of naive T cells toward regulatory T cells and upregulate FoxP3 and IL-10 expression in Treg cells (for a review, see Robinson, 2010).

In asthmatic patients receiving glucocorticoid treatment, FoxP3 mRNA expression was significantly increased and correlated tightly with IL-10 mRNA expression. The frequency of CD25(+) memory CD4(+) T cells and transient FoxP3 mRNA expression by CD4(+) T cells significantly increased after systemic glucocorticoid treatment (Karagiannidis et al., 2004).

Recently, it was shown that glucocorticoid-induced leucine zipper (GILZ) promotes peripheral Treg cell production (Bereshchenko et al., 2014). This function involves the regulation of TGF-βsignaling which is a requisite for the peripheral induction of FoxP3 in naive T cells and generation of peripheral Treg cells (Josefowicz et al., 2012;Yadav et al., 2013).Thus, GILZ mediates glucocorticoid-induced Treg generation by promoting TGF-βsignaling.

### IV.2.2. Selection of suitable glucocorticoids.

For the method of the present invention it is of interest, that the immunosuppressive and anti-inflammatory effects of glucocorticoids can be locally restricted to the site of allergen or autoantigen prersentation in order to minimize adverse systemic side effects. Therefore, a short plasma half-life is advantageous. Glucocorticoids exhibiting a short plasma half-life (ranging between 30 min and 2 hours) and a relatively short biological half-life of 8-12 hours include cortisone and hydrocortisone, glucocorticoids exhibiting an intermediate plasma half-life (ranging between 2.5 and 5 hours) and an intermediate biological half-life of 18-36 hours include prednisone, prednisolone, methylprednisolone and triamcinolone, and glucocorticoids exhibiting a long plasma half-life (up to 5 hours) and a relatively long biological half-life of 36-54 hours include dexamethasone, betamethasone and fludrocortisone (for a review, see Longui, 2007).

However, most important for the method of the present invention is the glucocorticoid potency, which defines the capacity to elevate glycemia and which is proportional to the anti-inflammatory potency. In this respect cortisone and hydrocortisone exhibit a rather low potency, prednisone, prednisolone, methylprednisolone and triamcinolone an intermediate potency, whereas dexamethasone and betamethasone exhibit a rather high potency, which is 25-30-fold higher than that of cortisone or hydrocortisone (for a review, see Longui, 2007). Therefore, glucocorticoids with a high anti-inflammatory potency such as dexamethasone are preferred for the the method of the present invention despite their relatively long plasma and biological half-lives.

### IV.3.Aptamer-based inhibitors of interleukins

In allergic and autoimmune diseases interleukins play an important role. For example, allergens are recognized and processed by dendritic cells that drive Th2 differentiation and secretion of multiple interleukins (IL) including but not limited to IL-4, IL-5, IL-9, IL-13, IL-17, and IL-25 family members. The many functions of these interleukins include induction of B cells by IL-4 and IL-13 to produce IgE, promotion of development, recruitment and survival of eosinophils by IL-5, activation of mast cells and induction of mucus hypersecretion by IL-9, and promotion of influx of lymphocytes and neutrophils by IL-17 family members. Furthermore, results of studies in mouse models and in humans have identified a key role of IL-17 family members in the pathogenesis of autoimmunity, including rheumatoid arthritis (RA) and multiple sclerosis (MS).

The demonstration that these interleukins contribute to local and systemic aspects of the pathogenesis of allergic and autoimmune diseases, suggest that local inhibition of these interleukins at the site of allergen/autoantigen presentation will improve the efficacy of allergen- or autoantigen-specific immunotherapy (for reviews, see; Catley et al., 2011; Miossec and Kolls, 2012; Petersen and Lukacz, 2012; Polosa and Casale, 2012).

However, considering the complicated interactions of multiple interleukins in allergic and autoimmune diseases, inhibition of one interleukin will not be sufficient for efficient support of allergen- or autoantigen-specific immunotherapy. There are multiple pro- and anti-inflammatory cytokines, which exhibit overlapping actions and redundancy, making the overall effect extremely difficult to predict (for a review, see Gibeon and Menzies-Gow; 2012). Therefore, a combined anti-interleukin therapy at the site of allergen/autoantigen presentation will be necessary to guarantee significant efficacy of this adjuvant approach.

For the application of interleukin inhibitors as additional immune modulators it is important that the interleukin inhibitors exhibit a short plasma half-life to minimize potential adverse effects mediated by interaction of the therapeutics with targets away from the site of allergen or autoantigen presentation. This requirement is even more important for a combined anti-interleukin therapy, since potential adverse effects mediated by interaction of the therapeutics with targets away from the site of allergen or autoantigen presentation are likely to be potentiated by a combination of anti-interleukin therapeutics staying in circulation for a long time.

### V. ADDITIONAL COMPOUNDS SUPPORTING THE THERAPEUTIC EFFICACY

In another embodiment, the present invention discloses additional compounds capable of supporting the therapeutic efficacy of the method of the present invention. Said supporting compounds include but are not limited to eat-me signals in addition to phosphatidylserine (PS) for efficient peripheral phagocytosis, tolerance-supporting liposomal surface ligands, and supporting mediators of macrophage-mediated immune suppression.

### V.1. Additional eat-me signals for efficient peripheral phagocytosis.

In one embodiment, one or more of these additional eat-me signals exposed on apoptotic cells, are attached to the surface of PS-containing liposomes or added to mixtures conmprising PS-containing liposomes in order to enhance tolerance-promoting phagocytosis.

Several studies have suggested that PS recognition is both necessary and sufficient for clearance of apoptotic cells. However, there is also evidence that apoptotic cells expose additional eat-me signals, the combination of which may enhance engulfment. Numerous eat-me signals exposed on apoptotic cells have been identified to date including but not limited to changes in glycosylation of surface proteins or changes in surface charge, expression of intercellular adhesion molecule 3 (ICAM3) and oxidized low-density lipoprotein particle (OxLDL), and exposure of certain intracellular proteins such as calreticulin and annexin I (for a review, see Hochreiter-Hufford and Ravichandran, 2013).

In one specific embodiment, annexin I (annexin A1, ANXA1) is employed as additional eat-me signal attached to PS-containing liposomes. ANXA1 is a 38 kDa protein that is recruited from the cytosol and exported to the outer plasma membrane leaflet, where it is required for efficient clearance of apoptotic cells. It binds to PS in a calcium-dependent manner, thereby mediating tethering of apoptotic cells to engulfing cells and internalization. ANXA1 is thought to bind to PS in a bivalent manner. Thereby, ANXA1 can act as a bridging protein between two PS molecules and is capable of pulling two membranes together. However, ANXA1 can also bind to other proteins such as S100 to produce complexes that can cross-link two membranes.Silencing of ANXA1 gene expression via siRNA has been shown to result in defective engulfment of apoptotic cells and the addition of purified soluble annexin I restored the observed engulfment defects observed in these cells (Arur et al., 2003). For the method of the present invention it is also advantageous that ANXA1 mediates various other anti-inflammatory functionsin addition to its tethering function. For example, ANXA1 mediates also the anti-inflammatory action of glucocorticoids and its N-terminal peptide can bind to the formyl peptide receptor on neutrophils, thereby preventing their trans-endothelial extravasation. Furthermore, exogenous administration of annexin I has been demonstrated to confer anti-inflammatory activity in animals (Goulding et al., 1998).

In another specific embodiment, calreticulin is employed as additional eat-me signal in cooperation with PS-containing liposomes. The endoplasmatic protein calreticulin (CRT) isa highly conserved 46 kDa protein. By still unknown mechanisms, CRT can be transported to the plasma membrane and is detectable on the cell surface. As a cell surface protein CRT has been implicated in antigen presentation and complement activation, immunogenicity of cancer cell death, wound healing, thrombospondin signaling, and clearance of apoptotic cells. Via trans-activation of the Low density lipoprotein Receptor-related Protein 1 (LRP1, also known as CD91 or the α2-macroglobulin receptor) on phagocytes, CRTcan act as a receptor for collectin family members (e.g., mannose binding lectin (MBL) and surfactant proteins A and D) and mediate uptake of apoptotic cells(Gardai et al., 2005). Most important for the method of the present invention is the fact that CRT does not need to be bound by a ligand to engage and stimulate LRP1 (Gardai et al., 2005). Thus, CRT and PS-containing liposomes can be administered as individual components and still act together to drive optimal phagocytosis. Also important for the method of the present invention is the observation that PS appears to drive the anti-inflammatory consequences of apoptotic cell-recognition, although LRP1 stimulation by CRT is known to be pro-inflammatory (Gardai et al., 2005). Thus, the combination of CRT and PS-containing liposomes can be assumed to drive optimal tolerance-promoting phagocytosis.

In another embodiment, one or more serum adaptor proteins capable of bridging PS to receptors on phagocytes, are employed in addition to eat-me signals on apoptotic cells in order to enhance phagocytosis. Suitable serum adaptor proteins include but are not limited to β2-glycoprotein I (β2GPI), the growth arrest-specific gene product 6 (Gas6), the milk-fat globule EGF-factor 8 (MFG-E8), and protein S.

In one specific embodiment, the milk-fat globule EGF-factor 8 (MFG-E8) is employed as additional eat-me signal in cooperation with PS-containing liposomes. MFG-E8 contains one or two EGF-like domains, two F5/8 type C domains, a PS-binding domain and an arginine-glycine-aspartic acid (RGD) motif, which enables the binding to integrins. Thereby, MFG-E8 bridges apoptotic cells or PS-containing liposomes to integrins on the surface of phagocytes (Hanayama et al., 2002). For the method of the present invention, recombinant human MFG-E8 can be produced in different eukaryotic systems. One MFG-E8 construct comprising 463 amino acid residues (apparent molecular weight 72 kDa) has been expressed in human 293T cells (Hanayama et al., 2002) and another MFG-E8 construct comprising 374 amino acid residues (apparent molecular weight 45 kDa) has been expressed in a baculovirus/insect cell system (e.g., Sino Biological Inc., Bejing, P-R. China).

In another specific embodiment, β2-glycoprotein I (β2-GPI) is employed as additional eat-me signal in cooperation with PS-containing liposomes. β₂-Glycoprotein I (also known as apolipoprotein H) is an anionic phospholipid-binding glycoprotein that belongs to the complement control protein (CCP) superfamily. β₂-GPI consists of 326 amino acids and contains four N-glycosylation sites (Arg143, Arg164, Arg174, and Arg234) and one O-linked sugar on Thr130. The glycans account for approximately 20% w/w of the total molecular mass (50 kDa). β₂-GPI is organized in five CCP domains. The first four domains have the regular, conserved sequences, but the fifth domain is aberrant. This domain contains a six-residue insertion, a 19-residue C-terminal extension, and an additional disulfide bond that includes a C-terminal cysteine. These additional amino acids in domain V constitute a large positively charged patch that mediates bindingto anionic phospholipids. Thereby, β₂-GPI serves as an intermediate for the interaction of PS-exposing apoptotic cells or PS-containing liposomes with macrophages (Balasubramanian and Schroit, 1998). β₂-GPI is a relatively abundant plasma protein and it circulates in blood at a level of 50-500 µg ml⁻¹(1-10 µM). For the method of the present invention, PS-containing liposomes are pre-incubated with β₂-GPI prior to administration at the site of antigen or allergen presentation. Recombinant human β₂-GPI can be produced in different eukaryotic systems. For example, a full-length cDNA coding a human β₂-GPI (MW 43000) has been produced in a baculovirus/insect cell system and purified from the culture supernatant by sequential cardiolipin-affinity column chromatography and gel filtration (Igarashi et al., 1993).

In another embodiment, one or more serum adaptor proteinsinvolved in the recognition of apoptotic cells, are employed in addition to eat-me signals on apoptotic cells in order to enhance phagocytosis. Suitable serum adaptor proteins include but are not limited to thrombospondin, complement protein C1q, c-reactive protein (CRP), Immunoglobulin M (IgM), and mannose binding lectin (MBL) (for a review, see Chaurio et al., 2009).

### V.2. Tolerance-supporting liposomal surface ligands

In another embodiment, PS-liposomes are modified by one or more additional surface-attached ligands capable of supporting the tolerance-promoting effect of PS. Such ligands include but are not limited to ligands of B cell inhibitory co-receptors.

B cell inhibitory co-receptors include but are not limited to CD22 and SIGLEC-10 (SIGLEC-G in mice), both members of the sialic acid binding Ig-like lectin immunoglobulin family which contain at least one ITIM on their cytoplasmic tail and are capable of inhibiting B cell receptor-mediated signaling. Ligands of these co-receptors are sialic acid-containing glycans of glycoproteins and glycolipids. CD22 exhibits a strict preference for α2-6-sialosides over α2-3-sialosides, whereas SIGLEC-G has the ability to bind α2-3-sialosides.

Liposomes displaying both antigen and CD22 glycan lipids have been shown to induce robust antigen-specific tolerance to protein antigens in mice (Macauley et al., 2013). Liposomes displaying both antigen and SIGLEC-G glycan lipids have also been shown to inhibit B cell receptor signaling and to induce robust tolerance toward T-independent and T-dependent antigens in mice (Pfrengle et al., 2013).

Preferred ethylamine-derivatized human CD22 glycan ligands include but are not limited to [9-biphenylcarboxyl-N-glycolylneuraminic acid-α2-6-galactose-β1-4-N-acetylglucosamine-β-ethylamine (6'-^{BPC}NeuGc)] . Preferred murine ethylamine-derivatized CD22 glycan ligands include but are not limited to [9-biphenylacetyl-N-glycolylneuraminic acid-α2-6-galactose-β1-4-N-acetylglucosamine-β-ethylamine (6'-^{BPA}NeuGc)], which binds to murine CD22 with 200-fold higher affinity than its natural ethylamine-derivatized ligand NeuGcα2-6Galβ1-4GlcNAc-β-ethylamine. Preferred murine ethylamine-derivatized SIGLEC-G glycan ligands include but are not limited to [9-biphenylacetyl-N-glycolylneuraminic acid-α2-3-galactose-β1-4-N-acetylglucosamine-β-ethylamine (3'-^{BPA}NeuGc)], and the natural ethylamine-derivatized ligand NeuGcα2-3Galβ1-4GlcNAc-β-ethylamine. Methods for coupling of these glycan ligands to lipd molecules are known to the person skilled in the art. In a preferred coupling procedure the ethylamine-derivatized glycan ligands are reacted with NHS-derivatized PEG-spacer molecules which are covalently attached to phosphatidylethanolamine molecules.

### V.3. Supporting mediators of macrophage-mediated immune suppression

In another embodiment, tolerizing PS-liposomes are modified by encapsulation of one or more additional mediators of macrophage-induced immune suppression including but not limited to microRNA-21 (miR-21) and resolvin D1.

MicroRNAs (miRs) are 19-22 nucleotides long and function as non-coding RNAs. Ingeneral, miRs negatively regulate gene expression post-transcriptionally by binding to the 3'-untranslated region (UTR) of the targeted messenger RNA (mRNA) to inhibit gene translation. Emerging evidence indicates that miR-21 regulates inflammatory responses. For example, in macrophages miR-21 silences the pro-inflammatory IL-12. Furthermore, upon phagocytosis of apoptotic cells (named efferocytosis) the expression of inducible miR-21 in macrophages is increased, leading to silencing of PDCD4 (programmed cell death protein 4) and favoring of c-Jun-AP-1 activity (c-Jun in combination with c-Fos forms the AP-1 early response transcription factor), which in turn results in elevated production of anti-inflammatory IL-10 (Das et al., 2014).

In one specific embodiment, tolerizing PS-liposomes are modified by encapsulation of miR-21 mimic which is a double strand sense mature miR-21. As demonstrated in a recent study, an increase of the cellular abundance of miR-21 levels in human monocyte-derive macrophages by transfection with miR-21 mimic (miRIDIAN mimic-miR-21; Dharmacon RNA Technologies) inhibited PDCD4 expression and resulted in significant suppression of LPS-induced TNF-α expression and NF-κB activation (Das et al., 2014).

In another embodiment, tolerizing PS-liposomes are modified by encapsulation of resolvin D1, an inducer of miR-21 (Das et al., 2014). Resolvins are compounds that are made by the human body from the omega-3 fatty acids eicosapentaenoic acid (EPA) and docosahexanenoic acid (DHA). They are produced by the COX-2 pathway especially in the presence of acetyl salicylic acid. Experimental evidence indicates that resolvins reduce cellular inflammation. Resolvin D1 is produced physiologically from the sequential oxygenation of DHA by 15- and 5-lipoxygenase.

In one specific embodiment, PS-liposomes are modified by encapsulation (ethanolic solution) or incorporation into the liposomal layers of commercially available 7(R)-Resolvin D1, an aspirin-triggered epimer of resolvin D1 that reduces human polymorphonuclear leukocyte (PMN) trans-endothelial migration, the earliest event in acute inflammation, with equipotency to resolvin D1 (EC₅₀ = ∼30 nM). 17 (R)-Resolvin D1 exhibits a dose-dependent reduction in leukocyte infiltration in a mouse model of peritonitis with maximal inhibition of ∼35% at a 100 ng dose. In contrast to resolvin D1, the aspirin-triggered form resists rapid inactivation by eicosanoid oxidoreductases (Sun et al., 2007).

### VI. FIELDS OF APPLICATION AND TREATMENT OPTIONS

In one embodiment, the present invention discloses allergic and autoimmune diseases for which the method of the present invention is beneficial. Allergic diseases include but are not limited to allergic conjunctivitis, allergic rhinitis, and allergic asthma. Autoimmune diseases include but are not limited to rheumatoid arthritis, type I diabetes, multiple sclerosis, and autoimmune uveoretinitis. For such diseases, the present invention disclosesmethods for restoring lasting immunological tolerance by peripheral administration of a matrix containing embedded tolerizing PS-liposomes with one or more encapsulated inhibitors of DC maturation and one or more encapsulated allergens/autoantigens or fragments thereof, one or more hydrogel-embedded eat-me signal for efficient peripheral phagocytosis of tolerizing PS-liposomes and, optionally, one or more hydrogel-embedded immune modulator capable of directly inhibiting inflammatory responses by effector T cells and other cells, thereby supporting the tolerizing efficacy. Preferably, all of these components are embedded in hydrogels capable of mediating the sustained local supply of these therapeutics. In the embodiment according to the invention, treatment is performed with thermo-sensitive copolymeric hydrogels composed of PLGA-PEG-PLGA.

The present invention aims for immune intervention by targeting the molecular mechanisms of allergen or autoantigen tolerance and reciprocal regulation of effector and regulatory T cells. The locally restricted therapeutic approach according to the method of the present invention is based on varying combinations of different therapeutic approaches, each of which addresses in a locally restricted manner the complex network of allergen- or autoantigen-specific Treg induction by the generation of tolerizing dentritic cells via tolerizing PS-liposomes, the induction of of macrophage-mediated immune suppression, the reciprocal regulation of effector T cells and other immune cells, and the inhibition of Th1- and Th2-promoting cytokines.

### VI.1. Treatment of allergy

Allergen-specific immunotherapy has been used for many decades as a desensitizing therapy for allergic diseases and represents the potentially curative method of treatment. Based on current knowledge, allergen tolerance is mediated by peripherally induced regulatory T cells as evidenced by a deficit of allergen-specific IL-10 producing T cells in the peripheral blood of allergic patients (for a review, see Schmidt-Weber et al., 2006). However, despite recent improvements the efficacy of allergen-specific immunotherapy needs to be optimized.Allergen-specific immunotherapies are efficient when patients are mono-sensibilized against seasonal allergens, but can be less or not efficient if the patient is atopic or if the patient reacts to perennial allergens.Therefore, improved approaches are needed.

The present invention discloses methods for restoring lasting immunological tolerance by allergen-specific immunotherapy using hydrogel-embedded tolerizing PS-liposomes containing one or more DC maturation inhibitors and one or more allergens or peptides derived thereof, one or more hydrogel-embedded find-me signals for efficient peripheral phagocytosis of tolerizing PS-liposomes and, optionally, one or more hydrogel-embedded immune modulator capable of supporting the tolerizing efficacy.

### VI.1.1. Selection of DC maturation inhibitors for the treatment of allergy

In one embodiment, the method utilizes tolerizing PS-liposomes loaded with one or more pharmacological DC maturation inhibitors selected from a) calcipotriol, b) glucocorticoids, and c) antisense oligonucleotides capable of gene silencing of different pro-inflammatory molecules including CD40, CD80, and CD86.

### VI.1.2. Selection of allergens for the treatment of allergy

In one embodiment, the method utilizes tolerizing PS-liposomes loaded with one or more allergens or peptides derived thereof from a variety of sources including but not limited to plant pollen (derived from e.g. grass (ryegrass, timothy-grass), weeds (ragweed, plantago, nettle, Artemisia vulgaris, Chenopodium album, sorrel), and trees (birch, alder, hazel, hornbeam, Aesculus, willow, poplar, Platanus, Tilia, Olea, Ashe juniper, Alstonia scholaris)), animal products (derived from e.g. dust mite excretion (feces and chitin), fur and dander, Fel d 1, cockroachcalyx, and wool), food (derived from e.g. legumes (peanuts, beans, peas, and soybeans), tree nuts (pecans, and almonds), fruit (e.g., pumpkin, egg-plant), eggs (typically albumen, the white), milk, fish (e.g., shellfish), wheat and their derivatives,sesame seeds,mustard, celery, celeriac, and corn or maize),insect venoms (derived from e.g., wasps, honey bees, mosquitos, and fire ants), airborne fungal allergens (e.g., the basidospore family whichproduces the dominant airborne fungal allergens and includes mushrooms, rusts, smuts, brackets, and puffballs), drugs (e.g., penicillin, sulfonamides, salicylates, neomycin),latex, metal, wood, and other allergens (e.g. nickel sulfate, Balsam of Peru, fragrance mix).

In another embodiment, the method utilizes tolerizing PS-liposomes loaded with one or more allergen extracts derived from said allergen sources and/or chemically modified allergen extracts with reduced IgE-reactivity. In a preferred embodiment, the method of the present invention utilizes tolerizing PS-liposomes loaded with one or more recombinant allergens derived from said allergen sources and/or derivatives of recombinant allergens with reduced IgE-reactivity. In another embodiment, the method utilizes tolerizing PS-liposomes loaded with one or more allergen-derived peptides.

### VI.1.3. Selection of additional immune modulators for the treatment of allergy

In one embodiment, the method of the present invention utilizes one or more additional immune modulators capable of supporting the tolerizing efficacy selected from a) calcipotriol, b) glucocorticoids, and c) antisense oligonucleotides capable of gene silencing of different pro-inflammatory molecules including CD40, CD80, and CD86.

### VI.2. Treatment of allergic asthma

Asthma is one of the most common chronic diseases worldwide. It is estimated that 150 million people around the world suffer from asthma. Mortality has reached over 180,000 annually. In Western Europe the incidence of asthma has doubled in ten years. Around 8% of the Swiss population suffers from asthma as compared with only 2%, 25-30 years ago. In the United States, there were an estimated 20.3 million asthmatics in 2001; the number of asthmatics has leapt by over 60% since the early 1980s and deaths have doubled to 5,000 a year. There are about 3 million asthmatics in Japan of whom 7% have severe asthma and 30% have moderate asthma. In Australia, one child in six under the age of 16 is affected. India has an estimated 15-20 million asthmatics and rough estimates indicate a prevalence of between 10% and 15% in 5-11 year old children.

According to a review of 75 trials covering a total of 3,188 patients with asthma, SCIT (subcutaneous immunotherapy) led to a significant reduction in asthma symptom scores, medication use and airway hyper-responsiveness, with evidence of a dose-related effect (Abramson et al., 2003). SCIT and SLIT (sublingual immunotherapy) also decrease the development of sensitization to new allergens and decrease the risk of new asthma in both adults and children with rhinitis. A recent retrospective cohort study of 322 subjects with allergic rhinitis showed that 53.1% of subjects who were not treated with SCIT developed asthma, whereas only 41.6% of subjects who received SCIT were diagnosed with asthma (for a review, see Holgate and Polosa, 2008).

Taken together, immunotherapy has been proven efficacious in treating mild asthma, as well as in preventing the progression to asthma in patients suffering from rhinoconjunctivitis (Moller et al., 2002; Jacobsen et al., 2007), but it is not yet recommended for the treatment of moderate to severe asthmatic patients (Rolland et al., 2009). Therefore, improved approaches are needed.

The present invention discloses methods for restoring lasting immunological tolerance by allergen-specific immunotherapy using hydrogel-embedded tolerizing PS-liposomes containing one or more DC maturation inhibitors and one or more allergens or peptides derived thereof, one or more hydrogel-embedded find-me signals for efficient peripheral phagocytosis of tolerizing PS-liposomes and, optionally, one or more hydrogel-embedded immune modulator capable of supporting the tolerizing efficacy.

### VI.2.1. Selection of DC maturation inhibitors for the treatment of allergic asthma

In one embodiment, the method utilizes tolerizing PS-liposomes loaded with one or more pharmacological DC maturation inhibitors selected from a) calcipotriol, b) glucocorticoids, and c) antisense oligonucleotides capable of gene silencing of different pro-inflammatory molecules including CD40, CD80, and CD86

### VI.2.2. Selection of allergens for the treatment of allergic asthma

In one embodiment, the method utilizes tolerizing PS-liposomes loaded with one or more allergens or peptides derived thereof from a variety of sources including but not limited to plant pollen (derived from e.g. grass (ryegrass, timothy-grass), weeds (ragweed, plantago, nettle, Artemisia vulgaris, Chenopodium album, sorrel), and trees (birch, alder, hazel, hornbeam, Aesculus, willow, poplar, Platanus, Tilia, Olea, Ashe juniper, Alstonia scholaris)), animal products (derived from e.g. dust mite excretion (feces and chitin),fur and dander, Fel d 1, cockroachcalyx, and wool), food (derived from e.g. legumes (peanuts, beans, peas, and soybeans), tree nuts (pecans, and almonds), fruit (e.g., pumpkin, egg-plant), eggs (typically albumen, the white), milk, fish (e.g., shellfish), wheat and their derivatives,sesame seeds,mustard, celery, celeriac, and corn or maize),insect venoms (derived from e.g., wasps, honey bees, mosquitos, and fire ants), and airborne fungal allergens (e.g., the basidospore family whichproduces the dominant airborne fungal allergens and includes mushrooms, rusts, smuts, brackets, and puffballs).

In another embodiment, the method utilizes tolerizing PS-liposomes loaded with one or more allergen extracts derived from said allergen sources and/or chemically modified allergen extracts with reduced IgE-reactivity. In a preferred embodiment, the method utilizes tolerizing PS-liposomes loaded with one or more recombinant allergens derived from said allergen sources and/or derivatives of recombinant allergens with reduced IgE-reactivity. In another embodiment, the method utilizes tolerizing PS-liposomes loaded with one or more allergen-derived peptides.

### VI.2.3. Selection of additional immune modulators for the treatment of allergic asthma

In one embodiment, the method utilizes one or more additional immune modulators capable of supporting the tolerizing efficacy selected from a) calcipotriol, b) glucocorticoids, and c) antisense oligonucleotides capable of gene silencing of different pro-inflammatory molecules including CD40, CD80, and CD86.

### VI.2.4. Selection of patients with allergic asthma for immunotherapy according to the method of the present invention.

The pathogenesis of asthma exhibits marked heterogeneity, and there is evidence that there are multiple phenotypes and mechanistically distinct groups called endotypes (Corren, 2013). The term endotype describes the underlying molecular, functional, or pathological mechanisms of disease. As a consequence, patient-tailored therapies are increasingly recognized as the future of asthma management.

Based on the eosinophil and neutrophil proportions in sputum, subjects have been categorized into four inflammatory subtypes: eosinophilic asthma: i.e., sputum eosinophils >1.0%; neutrophilic asthma: i.e., sputum neutrophils >61%; mixed granulocytic asthma: both increased eosinophils and neutrophils; and paucigranulocytic asthma: i.e., normal levels of both eosinophils and neutrophils (Porsbjerk et al., 2009).

Furthermore, based on gene expression of interleukin (IL)-13, IL-5, and IL-4 in broncho-alveolar lavage and in bronchial biopsy specimens from asthma patients with mild-to-moderate asthma, two signature clusters have been identified: Th2-high and Th2-low asthma (Woodruff et al., 2009). Patients considered to be Th2-high had high levels of bronchial tissue IL-13 and IL-5 mRNA, higher airway hyper-responsiveness, greater numbers of eosinophils, higher IgE levels, and increased reticular basement membrane thickness. These patients had significant responses to inhaled corticosteroids (ICSs). Patients considered Th2-low had no cytokine expression difference compared with healthy individuals, normal basement membrane thickness, and less response to ICSs.

Combining molecular findings with clinical phenotypes, preliminary asthma endo/phenotypes are now emerging (Wenzel, 2012).
a) Th2-associated early-onset allergic asthma typically with childhood onset and a history of allergy or atopy. Patients in this category commonly have a family history of asthma.Biomarkers are not specific between atopic and nonatopic asthma. Generally, this phenotype responds to ICSs and anti-immunoglobulin-E monoclonal antibodies.
b) Th2-associated late-onset persistent eosinophilic asthma includes adult-onset, usually severe, asthma with persistent sputum eosinophilia ≥2% but shows less clinical allergic responses than early-onset asthma. Aspirin-exacerbated airway disease is thought to be a subset of this subtype. Family history is rare. Lung eosinophilia is a distinct biomarker for this endotype. Generally, this subtype requires high-dose ICSs to overcome steroid resistance, and subsets can respond to leukotriene modifiers.
c) Th2-associated exercise-induced bronchospasm occurs primarily after exercise. In general, affected individuals experience mild asthma and reactive bronchoconstriction in response to exercise. No specific biomarkers or genetic factors have been identified.
d) Non-Th2-associated obesity-related asthma is generally women with late-onset, minimally allergic, mild asthma. Patients are highly symptomatic, but no specific biomarkers or genetic factors have been identified. This subgroup responds relatively poorly to ICSs.
e) Non-Th2-associated neutrophilic asthma has neutrophilic airway inflammation, but limited data are available to characterize it in detail. This subgroup responds relatively less well to ICSs.
f) Non-Th2-associated smoking asthma is sometimes considered to be a subtype of neutrophilic asthma, as smoking is sometimes associated with neutrophilia in lung tissue. At present, there is little information on the role of genetics or biomarkers in this subtype.

In one embodiment, the composition of the present invention are used in the treatment of patients with asthma endo/phenotypesa-f.

In a preferred embodiment, the composition of the present invention are used in the treatment of asthmatic patients with a) Th2-associated early-onset allergic asthma, and b) Th2-associated late-onset persistent eosinophilic asthma.

In a more preferred embodiment, the composition of the present invention are used in the treatment of asthmatic patients with Th2-associated early-onset allergic asthma (endo/phenotypea).

In a specific embodiment, treatment of asthma patients with lung eosinophilia (endo/phenotypeb) includes at least an anti-IL-5 aptamer as optional hydrogel-embedded immune modulator (see VI.2.3.).

In another specific embodiment, treatment of asthma patients which exhibit clearly increased IL-13 levels in sputum or biopsy specimens and express IL-13-sensitive genes in their airway epithelial cells (Th2-high endotype; endo/phenotypes a andb), according to the method of the present invention includes as least an anti-IL-13 aptamer as optional hydrogel-embedded immune modulator (see VI.2.3.).

### VI.3. Treatment of rheumatoid arthritis (RA)

RA affects between 0.5 and 1% of adults in the developed world with between 5 and 50 per 100,000 people newly developing the condition each year (Scott et al., 2010). In 2010 it resulted in about 49,000 deaths globally (Lozano et al., 2012). Women are affected three to five times as often as men.

The age at which the disease most commonly starts is in women between 40 and 50 years of age, and for men somewhat later (Alamanos et al., 2006). Onset is uncommon under the age of 15 and from then on the incidence rises with age until the age of 80.

Autoantigen-specific immunotherapy with the 15-mer synthetic peptide dnaJp1 has been proven efficacious in treating RA, but the majority of autoantigen-specific immunotherapeutic approaches showed in placebo-controlled trialslittle or no evidence of clinical efficacy. Therefore, improved autoantigen-specific immunotherapeutic approaches are needed.

The present invention discloses methods for restoring lasting immunological tolerance in RA patients by autoantigen-specific immunotherapy using hydrogel-embedded tolerizing PS-liposomes containing one or more DC maturation inhibitors and one or more autoantigens or peptides derived thereof, one or more hydrogel-embedded find-me signals for efficient peripheral phagocytosis of tolerizing PS-liposomes and, optionally, one or more hydrogel-embedded immune modulator capable of supporting the tolerizing efficacy.

### VI.3.1. Risk factors of RA.

Half of the risk for RA is believed to be genetic (Scott et al., 2010). It is strongly associated with a) the inherited tissue type major histocompatibility complex (MHC) antigen HLA-DR4 (most specifically DR0401 and 0404), and b) the genes PTPN22 and, only in Asia, PADI4 (Plenge et al., 2007; Goeldner et al., 2010). Inheriting the PTPN22 gene has been shown to double a person's susceptibility to RA. PADI4 has been identified as a major risk factor in people of Asian descent, but not in those of European descent. First-degree relatives prevalence rate is 2-3% (Silman et al., 1993; Bellamy et al., 1992).

Smoking is the most significant non-genetic risk (Scott et al., 2010) with RA being up to three times more common in smokers than non-smokers, particularly in men, heavy smokers, and those who are rheumatoid factor positive (Sugiyama et al., 2010).

Epidemiological studies have confirmed a potential association between RA and two herpesvirus infections: Epstein-Barr virus (EBV) and Human Herpes Virus 6 (HHV-6) (Alvarez-Lafuente et al., 2005). Individuals with RA are more likely to exhibit an abnormal immune response to EBV and have high levels of anti-EBV antibodies (Balandraud et al., 2004).

Vitamin D deficiency is common in those with RA and may be causally associated (Wen and Baker, 2011). Some trials have found a decreased risk for RA with vitamin D supplementation while others have not (Wen and Baker, 2011). Whether vitamin D deficiency is a cause or a consequence of the disease remains unclear (Guillot et al., 2010).

### VI.3.2. Diagnosis of RA.

a) Rheumatoid factor (RF): autoantibodies to IgGFc (IgG and IgM classes). During the first year of illness, rheumatoid factor is more likely to be negative with some individuals converting to seropositive status over time. RF is also seen in other illnesses, for example Sjögren's syndrome, hepatitis C, systemic lupus erythematosus, chronic infections and in approximately 10% of the healthy population, therefore the test is not very specific. A negative RF does not rule out RA; rather, the arthritis is called sero-negative. This is the case in about 15% of patients (Nishimura et al., 2007).
b) Anti-citrullinated protein antibodies (ACPAs). The most common tests for ACPAs are the anti-CCP (cyclic citrullinated peptide) test and the anti-MCV assay (antibodies against mutated citrullinated Vimentin). Tests for presence of anti-citrullinated protein (ACP) antibodies are highly specific (88-96%) for rheumatoid arthritis (RA). Recently a serological point-of-care test (POCT) for the early detection of RA has been developed. This assay combines the detection of rheumatoid factor and anti-MCV for diagnosis of RA and shows a sensitivity of 72% and specificity of 99.7% (Luime et al., 2009). Like RF, these tests are positive in only a proportion (67%) of all RA cases, but are rarely positive if RA is not present, giving it a specificity of around 95% (Nishimura et al., 2007).
   As with RF, there is evidence for ACPAs being present in many cases even before onset of clinical disease. ACP antibodies may predate the onset of clinical RA by up to 15 years (van de Stadt et al., 2011).Pre-clinical positivity of anti-CCP and/or 2 or more RF isotypes was >96% specific for future RA (Deane et al., 2010).
c) Serum Cytokines and Chemokines.
   Assays for multiple serum cytokines and chemokines have been shown to enhance the specificity for prediction of RA disease onset (Deane et al., 2011; Sokolove et al., 2012).
   In pre-clinical RA, levels of the following cytokines and chemokines were positive in a significantly greater proportion of RA cases versus controls: interleukin (IL)-1α, IL-1β, IL-6, IL-10, IL-12p40, IL-12p70, IL-15, fibroblast growth factor-2, Flt-3 ligand, tumor necrosis factor-α, interferon gamma induced protein-10, granulocyte macrophage colony-stimulating factor, and CRP. Also, increasing numbers of elevated cytokines/chemokines were present in cases nearer to the time of diagnosis. RA cases ≥40 years-old at diagnosis had a higher proportion of samples positive for cytokines/chemokines 5-10 years prior-to-diagnosis, compared to cases <40 at diagnosis (Deane et al., 2010).
d) Other Blood Tests.
   Also, several other blood tests are usually done to allow for other causes of arthritis, such as lupus erythematosus. The erythrocyte sedimentation rate (ESR), C-reactive protein, full blood count, renal function, liver enzymes and other immunological tests (e.g., antinuclear antibody/ANA) are all performed at this stage. Elevated ferritin levels can reveal hemochromatosis, a mimic of RA, or be a sign of Still's disease, a seronegative, usually juvenile, variant of rheumatoid arthritis.

### VI.3.3. Selection of DC maturation inhibitors for the treatment of RA

In one embodiment, the method of the present invention utilizes tolerizing PS-liposomes loaded with one or more pharmacological DC maturation inhibitors selected from a) calcipotriol, b) glucocorticoids, and c) antisense oligonucleotides capable of gene silencing of different pro-inflammatory molecules including CD40, CD80, and CD86.

### VI.3.4. Selection of autoantigens for the treatment of RA

In one embodiment, the method of the present invention utilizes tolerizing PS-liposomes loaded with one or more autoantigens selected from type II bovine or chicken collagen, HCgp39, lyophilised Escherichia coliextract, the 15-mer synthetic peptide dnaJp1, and citrullinated proteins including but not limited to cit-vimentin, cit-fibrinogen, cit-fibrinogen, and cit-collagen type II, or peptides derived from these citrullinated proteins (see 1.4.4.).

In a preferred embodiment, the method of the present invention utilizes tolerizing PS-liposomes loaded with one or more autoantigens selected from the 15-mer synthetic peptide dnaJp1, and citrullinated proteins including but not limited to cit-vimentin, cit-fibrinogen, cit-fibrinogen, and cit-collagen type II, or peptides derived from these citrullinated proteins.

In a more preferred embodiment, the method of the present invention utilizes tolerizing PS-liposomes loaded with one or more autoantigens selected from citrullinated proteins including but not limited to cit-vimentin, cit-fibrinogen, cit-fibrinogen, and cit-collagen type II, or peptides derived from these citrullinated proteins.

### VI.3.5. Selection of additional immune modulators for the treatment of RA

In one embodiment, the method of the present invention utilizes one or more additional immune modulators capable of supporting the tolerizing efficacy selected from a) calcipotriol, b) glucocorticoids, and c) antisense oligonucleotides capable of gene silencing of different pro-inflammatory molecules including CD40, CD80, and CD86.

### VI.3.6. Selection of patients with RA for immunotherapy according to the method of the present invention.

In one embodiment, the method of the present invention is used for a preventive approach targeting individuals or families at genetic risk (see VI.3.1.).

In another embodiment, the compositions of the present invention are used in the treatment of patients with early established RA.

In a preferred embodiment, the composition of the present invention are used in the treatment of RA patients, especially including those patients at genetic risk, prior to onset of clinical RA, but after the emergence of one or more diagnostic indicator of RA. Most important selection criteria of the targeted patient population include a) the presence of anti-citrullinated protein (ACP) antibodies prior to onset of clinical RA (ACP antibodies may predate the onset of clinical RA by up to 15 years), and b) a high number of elevated cytokines and chemokines, since the number of elevated cytokines and chemokines predicts in the preclinical period of autoantibody positive cases the time of diagnosis of future RA in an age-dependent manner (see VI.3.1.). Women at age 40-50 represent another important selection criterium of the targeted patient population, since women are affected three to five times as often as men. Additional helpful selection criteria of the targeted patient population include a) abnormal immune response to EBV, b) smoking, and c) vitamin D deficiency. Individuals with RA are more likely to exhibit an abnormal immune response to EBV and have high levels of anti-EBV antibodies, in smokers RA is up to three times more common than in non-smokers, a Vitamin D deficiency is common in those with RA and may be causally associated.

### VI.4. Treatment of type 1 diabetes (T1D)

Type 1 diabetes causes an estimated 5-10% of all diabetes cases or 11-22 million worldwide (2). Within the United States the number of affected persons is estimated at one to three million. The development of new cases varies by country and region. The lowest rates appears to be in Japan and China with approx. 1 person per 100,000 per year, the highest rates are found in Scandinavia where it is closer to 35 new cases per 100,000 per year. In Finland the incidence is 57 per 100,000 per year. The United States and northern Europe fall somewhere in between with 8-17 new cases per 100,000 per year.

In 2006 it affected 440,000 children under 14 years of age and was the primary cause of diabetes in those less than 10 years of age. It is estimated that about 80,000 children develop the disease each year. The incidence of type 1 diabetes has been increasing by about 3% per year (Aanstoot et al., 2007). Autoantigen-specific immunotherapy with immunomodulatory peptide DiaPep277 (derived from hsp60 protein) resulted in preservation of stimulated C-peptide for 10 months posttreatment. Furthermore, therapy with GAD-alum (glutamic acid decarboxylase formulated in alum) in subjects with T1D within 6 months from diagnosis also showed preservation of stimulated C-peptide levels. However, phase 2 and 3 trials of GAD-alum therapy failed to confirm the preliminary observation. Therefore, improved autoantigen-specific immunotherapeutic approaches are needed.

The present invention discloses methods for restoring lasting immunological tolerance in T1D patients by autoantigen-specific immunotherapy using hydrogel-embedded tolerizing PS-liposomes containing one or more DC maturation inhibitors and one or more autoantigens or peptides derived thereof, one or more hydrogel-embedded find-me signals for efficient peripheral phagocytosis of tolerizing PS-liposomes and, optionally, one or more hydrogel-embedded immune modulator capable of supporting the tolerizing efficacy.

### VI.4.1. Risk factors of T1D.

T1D is a disease that involves many genes. Depending on locus or combination of loci, they can be dominant, recessive, or somewhere in between. The strongest gene, IDDM1, is located in the MHC Class II region on chromosome 6, at staining region 6p21. Certain variants of this gene increase the risk for decreased histocompatibility characteristic of type 1. Such variants which are common in North Americans of European ancestry and in Europeans include DRB1 0401, DRB1 0402, DRB1 0405, DQA 0301, DQB1 0302 and DQB1 0201 (for a review, see Bluestone et al., 2010).

The family-related risk of a child developing T1D is about 10% if the father has it, about 10% if a sibling has it, about 4% if the mother has T1D and was aged 25 when the child was born, and about 1% if the mother was over 25 years old when the child was born.

One theory proposes that T1D is a virus-triggered autoimmune response in which the immune system attacks virus-infected cells along with the beta cells in the pancreas (Faiweather and Rose, 2002). The Coxsackie virus family or rubella is implicated, although the evidence is inconclusive. However, not everyone infected by the suspected virus develops type 1 diabetes. This has suggested presence of a genetic vulnerability and there is indeed an observed inherited tendency to develop type 1. It has been traced to particular HLA genotypes, though the connection between them and the triggering of an autoimmune reaction is still poorly understood.

Strong evidence of a vitamin D effect on T1D risk comes from experiments in the non-obese diabetic (NOD) mouse. The NOD mouse experiences disease pathogenesis similar to the human, including autoimmune destruction of β cells. When 1,25-dihydroxyvitamin D [1,25(OH)₂D], the active form of the vitamin, was administered to NOD mice in pharmacologic doses, it prevented the development of diabetes. More recently, NOD mice raised in a vitamin D deficient state were shown to develop diabetes at an earlier age than non-deficient NOD controls (Harris, 2005). Limited data from human observational studies suggest that early supplementation with 10 µg/d (400 IU/d) or less of vitamin D may not reduce the risk for T1D, but that doses of 50 µg/d (2000 IU/d) and higher may have a strong protective effect (Harris, 2005).

### VI.4.2. Diagnosis of T1D

By definition, the diagnosis of diabetes type 1 can be made first at the appearance of clinical symptoms and/or signs, but the emergence of autoantibodies may itself be termed "latent autoimmune diabetes". Not everyone with autoantibodies progresses to diabetes type 1, but the risk increases with the number of antibody types, with three to four antibody types giving a risk of progressing to diabetes type 1 of 60%-100% (Knip et al., 2005). The time interval from emergence of autoantibodies to frank diabetes type 1 can be a few months in infants and young children, but in some people it may take years - in some cases more than 10 years (Knip et al., 2005).

The main autoantibodies include a) islet cell autoantibodies (detectable by conventional immunofluorescence), b)insulin autoantibodies, c) autoantibodies against the 65-kDa isoform of glutamic acid decarboxylase (GAD), d) autoantibodies against the phosphatase-related IA-2 molecule, and e) autoantibodies against the zinc transporter autoantibodies (ZnT8) (Knip et al., 2005).

Furthermore, about a quarter of people with new T1D have developed some degree of diabetic ketoacidosis (a type of metabolic acidosis which is caused by high concentrations of ketone bodies, formed by the breakdown of fatty acids and the deamination of amino acids) by the time T1D is recognized.

### VI.4.3. Selection of DC maturation inhibitors for the treatment of T1D

In one embodiment, the method of the present invention utilizes tolerizing PS-liposomes loaded with one or more pharmacological DC maturation inhibitors selected from a) calcipotriol, b) glucocorticoids, and c) antisense oligonucleotides capable of gene silencing of different pro-inflammatory molecules including CD40, CD80, and CD86.

### VI.4.4. Selection of autoantigens for the treatment of T1D

In one embodiment, the method of the present invention utilizes tolerizing PS-liposomes loaded with one or more autoantigens selected from insulin, proinsulin, GAD65 (glutamic acid decarboxylase), IA-2 (islet antigen 2; tyrosine phosphatase), and the ZnT8 transporter (zink transporter 8, localized on the membrane of insulin secretory granules), the immunomodulatory peptide DiaPep277 (derived from hsp60 protein), and other HSP60-derived peptides (see 1.4.6).

In a preferred embodiment, the method of the present invention utilizes tolerizing PS-liposomes loaded with one or more autoantigens selected from proinsulin, GAD65 and HSP60-derived peptides.

In a more preferred embodiment, the method of the present invention utilizes tolerizing PS-liposomes loaded with one or more autoantigens selected from proinsulin and GAD65.

### VI.4.5. Selection of additional immune modulators for the treatment of T1D

In one embodiment, the method of the present invention utilizes one or more additional immune modulators capable of supporting the tolerizing efficacy selected from a) calcipotriol, b) glucocorticoids, and c) antisense oligonucleotides capable of gene silencing of different pro-inflammatory molecules including CD40, CD80, and CD86.

### VI.4.6. Selection of patients with T1D for immunotherapy accordingto the method of the present invention.

In one embodiment, the method of the present invention is used for a preventive approach targeting individuals or families at genetic risk (see VI.4.1.).

In another embodiment, the composition of the present invention are used in the treatment of patients with early established T1D.

In a preferred embodiment, the composition of the present invention are used in the treatment of T1D patients, especially including those patients at genetic risk, prior to onset of clinical T1D, but after the emergence of one or more diagnostic indicator of T1D. Most important selection criteria of the targeted patient population include the presence of at least one type of autoantibodies, preferably at least two different types of autoantibodies, more preferably at least three different types of autoantibodies, and most preferably at least four different types of autoantibodies (three to four antibody types give a risk of progressing to T1D of 60%-100%, see VI.4.2.). An additional helpful selection criterium of the targeted patient population includes individuals with vitamin D deficiency, since the risk for T1D by age 15 was reduced by about a third in vitamin D-supplemented compared with vitamin D-unsupplemented children (odds ratio 0.67) (The EURODIAB Substudy 2 Study Group,1999).

### VI.5. Treatment of multiple sclerosis (MS)

As of 2010, the number of people with MS was 2-2.5 million (approximately 30 per 100,000) globally, with rates varying widely in different regions. It is estimated to have resulted in 18,000 deaths that year. In Africa rates are less than 0.5 per 100,000, while they are 2.8 per 100,000 in South East Asia, 8.3 per 100,000 in the Americas, and 80 per 100,000 in Europe. Rates surpass 200 per 100,000 in certain populations of Northern European descent. The number of new cases that develop per year is about 2.5 per 100,000. Rates of MS appear to be increasing, but this may be explained simply by better diagnosis.

MS usually appears in adults in their late twenties or early thirties but it can rarely start in childhood and after 50 years of age. The primary progressive subtype is more common in people in their fifties.

The disease is more common in women, and the trend may be increasing. As of 2008, globally it is about two times more common in women than in men. In children, it is even more common in females than males, while in people over fifty, it affects males and females almost equally.

Autoantigen-specific immunotherapy has been proven efficacious in treating patients with relapsing-remitting MS. In one study, however, transdermal application of myelin peptides via skin patches had to be continued for a period of one year to achieve improvement(Walczak et al., 2013).In anotherstudy, myelin peptides were coupled to the surface of autologous, apoptotic peripheral blood monocytes and then reinjected into MS patients (Lutterotti et al., 2013). Although both studies showed promising results, there is a need for improved autoantigen-specific immunotherapeutic approaches.

The present invention disclosesmethods for restoring lasting immunological tolerance in Ms patients by autoantigen-specific immunotherapy using hydrogel-embedded tolerizing PS-liposomes containing one or more DC maturation inhibitors and one or more autoantigens or peptides derived thereof, one or more hydrogel-embedded find-me signals for efficient peripheral phagocytosis of tolerizing PS-liposomes and, optionally, one or more hydrogel-embedded immune modulator capable of supporting the tolerizing efficacy.

### VI.5.1. Risk factors of MS.

Specific genes that have been linked with MS include differences in the human leukocyte antigen (HLA) system - a group of genes on chromosome6 that serves as the MHC. Genome-wide association studies have discovered at least twelve other genes outside the HLA locus that modestly increase the probability of MS.

The most consistent finding is the association between multiple sclerosis and alleles of the MHC defined as DR15 and DQ6 (Compston and Coles, 2008).Other loci have shown a protective effect, such as HLA-C554 and HLA-DRB1*11.It has been estimated that HLA changes account for between 20 and 60% of the genetic predisposition (Baranzini, 2011).

The family-related risk is significant.In relatives of an affected person, the probability of MS is significantly higher, with a greater risk among those more closely related (Compston and Coles, 2002). If both parents are affected, the risk in their children is 10 times that of the general population (Milo and Kahana, 2010). In identical twins both are affected about 30% of the time, while around 5% for non-identical twins and 2.5% of siblings are affected with a lower percentage of half-siblings (Compston and Coles, 2008).

Low vitamin D and elevated immunoreactivity against Epstein-Barr virus before first clinical manifestation of multiple sclerosis was reported recently in 25 individuals (Decard et al., 2012). These data are in line with a prospective case-control study among US military personnel, showing that 25-OH-D levels are inversely correlated with the risk of MS later in life (Munger et al., 2008).

Many microbes have been proposed as triggers of MS, but none have been confirmed. The prevalence hypothesis proposes that the disease is due to an infectious agent more common in regions where MS is common and where in most individuals it causes an ongoing infection without symptoms. Only in a few cases and after many years does it cause demyelination. Evidence for a virus as a cause include: the presence of oligoclonal bands in the brain and cerebrospinal fluid of most people with MS, the association of several viruses with human demyelination encephalomyelitis, and the occurrence of demyelination in animals caused by some viral infection (Gilden, 2005). Human herpes viruses are a candidate group of viruses. Individuals having never been infected by the Epstein-Barr virus are at a reduced risk of getting MS, whereas those infected as young adults are at a greater risk than those having had it at a younger age. Other diseases that may be related include measles, mumps and rubella (Compston and Coles, 2008).

Smoking has been shown to be an independent risk factor for MS (Ascherio and Munger, 2007).

### VI.5.2. Diagnosis of MS

Multiple sclerosis is typically diagnosed based on the presenting signs and symptoms, in combination with supporting medical imaging and laboratory testing. The McDonald criteria, which focus on clinical, laboratory, and radiologic evidence of lesions at different times and in different areas, is the most commonly used method of diagnosis.

The most commonly used diagnostic tools are neuroimaging, analysis of cerebrospinal fluid and evoked potentials. Magnetic resonance imaging of the brain and spine may show areas of demyelination (lesions or plaques).

Testing of cerebrospinal fluid obtained from a lumbar puncture can provide evidence of chronic inflammation in the central nervous system. The cerebrospinal fluid is tested for oligoclonal bands of IgG on electrophoresis, which are inflammation markers found in 75-85% of people with MS.

At the current time, there are no laboratory investigations that can predict prognosis. Several promising approaches have been proposed including: interleukin-6, nitric oxide and nitric oxide synthase, osteopontin, and fetuin-A (Harris and Sadig, 2009). Since disease progression is the result of degeneration of neurons, the roles of proteins showing loss of nerve tissue such as neurofilaments, tau, and N-acetylaspartate are under investigation (Harris and Sadig, 2009). Antibodies against the Kir4.1 potassium channel appear to be related to MS (Methner and Zipp, 2013).

### VI.5.3. Selection of DC maturation inhibitors for the treatment of MS

In one embodiment, the method of the present invention utilizes tolerizing PS-liposomes loaded with one or more pharmacological DC maturation inhibitors selected from a) calcipotriol, b) glucocorticoids, and c) antisense oligonucleotides capable of gene silencing of different pro-inflammatory molecules including CD40, CD80, and CD86.

### VI.5.4. Selection of autoantigens for the treatment of MS

In a preferred embodiment, the method of the present invention utilizes tolerizing PS-liposomes loaded with the 7 myelin peptides including MBP₁₃₋₃₂, MBP₈₃₋₉₉, MBP₁₁₁₋₁₂₉, MBP₁₄₆₋₁₇₀, MOG₁₋₂₀, MOG₃₅₋₅₅, and PLP₁₃₉₋₁₅₄ (see I.4.2.).

### VI.5.5. Selection of additional immune modulators for the treatment of MS

In one embodiment, the method of the present invention utilizes one or more additional immune modulators capable of supporting the tolerizing efficacy selected from a) calcipotriol, b) glucocorticoids, and c) antisense oligonucleotides capable of gene silencing of different pro-inflammatory molecules including CD40, CD80, and CD86.

### VI.5.6. Selection of patients with MS for immunotherapy according to the method of the present invention.

In one preferred embodiment, the method is used for a preventive approach targeting individuals or families at genetic risk (see VI.5.1.). Women represent a preferred patient population, since globally the disease is about two times more common in women than in men. Additional important selection criteria of the targeted patient population for a preventive approach include a)low vitamin D level and b) elevated immunoreactivity against Epstein-Barr virus. Other helpful selection criteria of the targeted patient population include a) elevated immuno-reactivity against other viruses including measles virus, mumpsvirus, and rubella virus, and b) smoking (see VI.5.1.).

In a more preferred embodiment, the composition of the present invention are used in the treatment of patients with early established relapsing-remitting MS subtype (RRMS). The RRMS subtype is characterized by unpredictable relapses followed by periods of months to years of relative quiet (remission) with no new signs of disease activity. Deficits that occur during attacks may either resolve or leave problems, the latter in about 40% of attacks and being more common the longer a person has had the disease. This describes the initial course of 80% of individuals with MS. The RRMS subtype usually begins with a clinically isolated syndrome (CIS). In CIS, a person has an attack suggestive of demyelination, but does not fulfill the criteria for multiple sclerosis. 30% to 70% of persons experiencing CIS later develop MS (Miller et al., 2005).

In another embodiment, the method of the present invention is used for the treatment of patients with secondary progressive MS, which occurs in around 65% of those with initial relapsing-remitting MS, who eventually have progressive neurologic decline between acute attacks without any definite periods of remission. Occasional relapses and minor remissions may appear. The most common length of time between disease onset and conversion from relapsing-remitting to secondary progressive MS is 19 years.

In still another embodiment, the composition of the present invention are used in the treatment of patients with primary progressive MS and progressing relapsing MS. The primary progressive subtype occurs in approximately 10-20% of individuals, with no remission after the initial symptoms. It is characterized by progression of disability from onset, with no, or only occasional and minor, remissions and improvements. Progressive relapsing MS describes those individuals who, from onset, have a steady neurologic decline but also have clear superimposed attacks. This is the least common of all subtypes.

### VII. COMPOSITIONS FOR THERAPEUTIC APPLICATIONS

In one embodiment, the present invention discloses compositions for the treatment of allergic and autoimmune diseases according to the claims.

In a preferred specific embodiment, a biodegradable thermo-gelling polymer (hydrogel) solution is used for sustained local delivery of tolerizing agents. The quantity of each component in the biodegradable thermogelling polymer is balanced in a way that a) upon injection into the body the polymer forms a non-flowing gelin which the tolerizing agents are embedded, and b) upon gelation of the polymer composit at body temperature the amount of released tolerizing components is sufficient for the therapeutic aims of the method of the present invention. In a most preferred specific embodiment, a biodegradable PLGA-PEG-PLGA (PLGA: poly(lactic-co-glycolic acid); PEG: poly(ethylene glycol)) thermo-gelling triblock hydrogel solution is used for sustained local delivery of tolerizing agents.

In another preferred specific embodiment, at least two, more preferably at least three different allergens or autoantigens or fragments thereof are encapsulated in PS-liposomes. Most autoantigen-specific immunotherapy trials so far have simply failed to work. In 2009, a trial of a myelin peptide antigen involving 612 people with MS showed no benefit over a placebo (Freedman et al., 2011). One likely reason is that the immune response in most autoimmune diseases can shift from one antigen to another as tissue damage progresses, known as epitope spreading (McRae et al., 1995). The failed 2009 trial used a single antigen (Freedman et al., 2011). Therefore, new therapies all incorporate multiple antigens to anticipate epitope spreading.

In another preferred specific embodiment, one or two different DC maturation inhibitors are encapsulated in PS-containing liposomes, wherein said DC maturation inhibitors are selected from a) calcipotriol, b) glucocorticoids, and c) antisense oligonucleotides capable of gene silencing of different pro-inflammatory molecules including CD40, CD80, and CD86.

In another preferred specific embodiment, one or two different additional immune modulators are added to the hydrogel composition, wherein said immune modulators are selected from a) calcipotriol, b) glucocorticoids, and c) antisense oligonucleotides capable of gene silencing of different pro-inflammatory molecules including CD40, CD80, and CD86.

In still another preferred specific embodiment, find-me signals are added the hydrogel composition, wherein said find-me signals are ATP and UTP.

### VIII. PHARMACEUTICAL FORMULATIONS

In one embodiment, the therapeutic compositions of the present invention are incorporated into pharmaceutical compositions suitable for administration. Such compositions typically comprise the therapeutic compositions of the present invention and a pharmaceutically acceptable carrier. As used herein, a pharmaceutically acceptable carrier is intended to include any and all solvents, dispersion media, coatings, antibacterial and antifungal agents, isotonic systems, and the like, compatible with the components of the therapeutic compositions of the present invention and pharmaceutical administration. The use of such media and agents for pharmaceutically active substances is well known in the art. Supplementary active compounds can also be incorporated into the composition.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents, antioxidants such as ascorbic acid or sodium bisulfite, chelating agents such as ethylenediaminetetraacetic acid, buffers such as acetates, citrates or phosphates and agents for the adjustment of toxicity such as sodium chloride or dextrose. The pH can be adjusted with acids or bases, such as hydrochloric acid or sodium hydroxide. In many cases, it will be preferable to include isotonic agents, for example, sugars, polyalcohols such as mannitol, sorbitol, sodium chloride in the composition.

The composition should be fluid to the extent that easy syringability exists. The proper fluidity can be maintained, for example, by the use of a coating such as lecithin, by the maintenance of the required particle size in the case dispersion and by use of surfactants. The composition must be stable under the conditions of manufacture and storage and must be preserved against the contaminating action of microorganisms such as bacteria and fungi. Prevention of the action of microorganisms can be achieved by various antibacterial and antifungal agents such as parabens, chlorobutanol, phenol, ascorbic acid, thimoseral, and the like. In all cases, the composition must be sterile. Sterile injectable solutions can be prepared by filtered sterilization. The preparation can be enclosed in ampoules, disposable syringes or multiple dose vials made of glass or plastic.

In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

### IX. THERAPEUTIC METHODS

In one embodiment, the present disclosure discloses therapeutic methods including information about suitable therapeutically effective doses of PS-liposomes (selected from those listed in section 1.5.), DC maturation inhibitors (selected from those listed in section I.2.),allergens or autoantigens or peptides derived thereof (selected from those listed in section I.4.), find-me signals for efficient peripheral phagocytosis of tolerizing PS-liposomes (selected from those listed in section III.), immune modulator capable of supporting the tolerizing efficacy (selected from those listed in section IV.), and modes of administration for the induction of allergen or autoantigen tolerance using the compositions of the present invention.

Determination of a therapeutically effective dose is well within the capability of those skilled in the art. The therapeutically effective dose can be estimated initially in animal models, usually mice, rats, rabbits, dogs, pigs, or non-human primates. The animal model also can be used to determine the appropriate concentration range and route of administration. Such information can then be used to determine useful doses and routes for administration in humans.

Dosage regimens may be adjusted to provide the optimum therapeutic response. The quantity of the matrix-embedded components depends on the release kinetics of the biodegradable thermo-gelling polymer and is adjusted to a level that guarantees the continuous release of therapeutically effective doses over a period of 3 to 5 days. The quantity of embedded components will vary according to factors such as the weight and the age of the individual, and the ability of the composition to induce an effective immune response in the individual.

The following data of this section provide useful guidelines for the determination of a therapeutically effective dose for those skilled in the art.

### IX.1. Therapeutic effective doses of tolerizing PS-liposomes.

PS-liposomes have been studied in a variety of animal models in the recent past. Results obtained from these studies provide useful information for the application of PS-liposomes in humans.

For example, using BALB/cAnN mice (body weight approx. 20 g), the effect of subcutaneously (s.c.) administered PS-liposomes on immune responses upon subsequent injection of ovalbumin (OVA) or keyhole limpet hemocyanine (KLH) in complete Freund's adjuvant (CFA) has been investigated (Hoffman et al., 2005). In this study, PS-containing liposomes comprising a 30:30:40 molar ratio of PS to PC to cholesterol, were first injected s.c. in one flank of 6-8 weeks old BALB/cAnN mice (100 µl, corresponding to 0.5 mg of total lipid), followed after one hour by another s.c. injection of 150 µl of an emulsion containing 50 µg of OVA or 150 µg of KLH in CFAin the same region. As evident from the data, subcutaneously administered PS-liposomes specifically inhibited responses to antigens. Numbers of total leukocytes and antigen-specific CD4+ T cells were reduced as well as the level of antigen-specific IgG in blood. There was also a decrease of draining lymph node tissue mass and the size of germinal centers in spleen and lymph nodes (Hoffman et al., 2005).

Using a rat model of acute myocardial infarction (MI) in another study, the effects of intraveneously (i.v.) administered PS-liposomes on cardiac macrophages has been investigated (Harel-Adar et al., 2011) . In this study, 150 µl of a 0.06 M solution of PS-liposomes comprising a 30:30:40 (1:1:1.33) molar ratio of PS to PC to cholesterol (9 pmol lipid or approx. 5.4 mg lipid; compare Example 1.1), were injected i.v. through the femoral vein of female Sprague-Dawley rats (body weight approx. 150 g) 48 hours after MI induction. As evident from the data, i.v.administered PS-containing liposomes promoted angiogenesis, preservation of small scars, and prevented ventricular dilatation and remodeling. Following uptake of PS-liposomes by macrophages, the cells secreted high levels of the anti-inflammatory cytokines TGF-β and IL-10 and upregulated the expression of the mannose receptor CD206, concomitant with downregulation of proinflammatory markers such as TNF-α and the surface marker CD86 (Harel-Adar et al., 2011).

In one embodiment, PS-liposomes are used as carriers of encapsulated allergens/autoantigens or fragments thereof. Methods for encapsulation of proteins or fragments thereof in liposomes are known to the person skilled in the art. Various formulations of liposomes containing encapsulated allergens (including allergen extracts), autoantigens or fragments thereof have been prepared and studied in a variety of animal models (e.g., Ishii et al., 2010; Meechan et al., 2012; Belogurov et al., 2013) and in clinical trials (e.g., Basomba et al., 2002). Furthermore, clinical trials have demonstrated that subcutaneously administered liposomes containing encapsulated allergen extracts are safe and well tolerated (e.g., Galvain et al., 1999; Basomba et al., 2002).

In another embodiment, PS-liposomes are used as carriers of encapsulated DC maturation inhibitors. Methods for encapsulation of DC maturation inhibitors in liposomes are known to the person skilled in the art. For example, the lipohilic DC maturation inhibitors (NF-κB inhibitors curcumin, quercetin and Bay11-7082) have been encapsulated in phosphatidylcholine liposomes in the presence of methylated BSA (mBSA) used as model antigen (Capini et al., 2009). In this study, the final concentration of liposomal mBSA was 2.5 mg/ml, of liposomal Bay11-7082 0.5 mM (103.6 ug/ml;MW 207.3), of liposomal curcumin 2 mM (736.8 ug/ml;MW 368.4), and of liposomal quercetin also 2 mM (604.4 ug/ml;MW 302.2). In subsequent experiments, liposomal preparations containing mBSA and one of the NF-κB inhibitors were used for the treatment of established antigen-induced inflammatory arthritis (methylated BSA in CFA) in C57BL/6 mice. After a single s.c.injection of 0.1 ml of each liposomal preparation at the tail base, the mean clinical disease score was reduced within four days to approximately 50% as compared to control mice (Capini et al., 2009).

### IX.2. Therapeutic effective doses of vitamin D3.

Interesting vitamin D3 molecules include 25-OH-D3 (calcidiol), its biologically active metabolite 1,25-(OH)₂D3 (calcitriol), and vitamin D3 analog calcipotriol. These molecules have been studied in a variety of animal models and evaluated in clinical trials (for reviews, see Plum and DeLuca, 2010; Fletcher et al., 2012).

### IX.2.3. Calcipotriol

Calcipotriol (or calcipotriene) is a synthetic derivative of calcitriol, which has similar VDR binding properties as compared to calcitriol, but has low affinity for the vitamin D binding protein (DBP) (for a review, see Trémezaygues and Reichrath; 2011).In vivo studies in rats showed that effects of calcipotriol on calcium metabolism are 100-200 times lower as compared to calcitriol, while in vitro effects on proliferation and differentiation on human keratinocytes are comparable (Reichrath and Holick, 2010; Binderup et al., 1991). The half-life of calcipotriol in circulation is measured in minutes (Kragballe, 1995). The rate of clearance (serum half-life of 4 min in rats) is approximately 140 times higher for calcipotriol than for calcitriol.Furthermore, calcipotriol is rapidly metabolized and effects of the metabolites have been demonstrated to be 100 times weaker than those of the parent compound (Kissmeyer and Binderup, 1991).

Single dose toxicicity studies of calcipotriol administered to rats by subcutaneous injection revealed LD₅₀ values of 2.19 mg/kg in males (approx. 440 µg/200-g male rat) and 2.51 mg/kg in females (approx. 380 µg/150-g female rat) (Imaizumi et al., 1996). Rats died probably due to the circulatory and renal disturbance. According to this study, no death occurred up to a single s.c. dose of 540 pg/kg body weight (approx. 81 µg/150-g female rat), and no loss of body weight could be observerd two weeks after administration.

Calcipotriol has been used clinically for more than 10 years for topical treatment of psoriasis without systemic toxicity (for a review, see Plum and DeLuca, 2010). Clinical studies with radiolabeled ointment indicate that approximately 6% of the applied dose of calcipotriene is absorbed systemically when the ointment is applied topically to psoriasis plaques or 5% when applied to normal skin.

### IX.2.4. Animal studies

In animal studies, vitamin D molecules and analogs thereof have been used for the treatment of OVA-induced allergy in mice (Ghoreishi et al., 2009), OVA-induced allergic asthma in mice (Taher et al., 2008), insulin-dependent diabetes mellitus in NOD mice (Zella et al., 2003), Lyme arthritis and collagen-induced arthritis in mice (Cantorna et al., 1998), and experimental autoimmune encephalomyelitis (EAE) in mice (Branisteanu et al., 1995).

In the allergy model, mice were treated on their shaved dorsal skin with 30 mg/day of calcipotriol ointment (contains 50 µg calcipotriol/g; 1.5µg calcipotriol/30 mg) (Donovex, Leo Pharma) for three days followed by transcutaneous immunization with OVA in the presence of CpG adjuvant. This treatment abolished antigen-specific CD8+ T cell priming and induced CD4+CD5+ Tregs, thereby promoting antigen-specific tolerance (Ghoreishi et al., 2009).

In the allergic asthma mode, OVA-sensitized mice were subjected to allergen-specific immunotherapy by three s.c. injections of 100 µg OVA in the presence of 10 ng 1,25-(OH)₂D3. This treatment significantly inhibited airway hyper-responsiveness and caused a significant reduction of serum OVA-specific IgE levels (Taher et al., 2008).

In the murine model of type 1 diabetes, a diet containing 50 ng 1,25-(OH)₂D3/mouse/day was administered three times/week. This treatment prevented diabetes onset in NOD mice as of 200 days (Zella et al., 2003).

In the arthritis models, mice received a daily diet supplemented with 20 ng 1,25-(OH)₂D3/mouse/day. This dose was found to be effective in inhibiting the progression of arthritis without producing hypercalcemia (Cantorna et al., 1998).

In the EAE model, i.p.injection of 5 µg of 1,25-(OH)₂D3/kg body weight (200 ng calcitriol/20-g mouse) every 2 days prevented the appearance of paralysis in 70% of the treated mice (Branisteanu et al., 1995).

### IX.2.5. Clinical trials

Evaluation of vitamin D supplementation in clinical trials support some observations made in animal models. It should be noted, however, that only low doses of vitamin D3 or analogs thereof were administered to avoid adverse side effects such as hypercalcemia.

A recent prospective randomized, double-blind study including 48 children from 5 to 18 years of age with newly diagnosed asthma (only sensitive to house dust mites) has demonstrated that after six months of treatment with the glucocorticoid budesonide (800 µg/d, administered as dry powder) and cholecalciferol (500 IU) a significantly lower number of children experienced asthma exacerbation (17%) as compared to the group receiving only glucocorticoids (46%) (Majak et al., 2011).

For the prevention of type 1 diabetes, vitamin D supplementation in infants with 10 µg/day (400 IU/d) does not appear to be sufficient, but doses of 50 µg/day (2000 IU/d) and higher may have a strong protective effect (for a review, see Harris, 2005). A prospective study of vitamin D supplementation in infants and type 1 diabetes including 12,055 pregnant women in Northern Finland showed that regular vitamin D supplements during infancy at doses of over 50 µg/day (2000 IU/d) reduced the relative risk of type 1 diabetes over the subsequent 30 years to 0.14, and at doses of exactly 50 µg/day to 0.22 (Hyppönen et al., 2001). Current U.S. recommendations are in the range of 5-25 µg/day (200-1000 IU/d).

In one open label study, 19 patients with rheumatoid arthritis on methotrexate therapy were treated with 2 µg/day of alfacalcidiol (1(OH)D3) for a period of 3 months. After three months of therapy, 89% of patients experienced an improvement of disease activity, with 45% (9/19) going into remission (Andjelkovic et al., 1999).

Until 2012 seven vitamin D intervention studies have been performed in patients with multiple sclerosis, six of them being performed with inactive 25-OH-D3 and one with active 1,25-(OH)₂D3 (for a review, see Fletcher et al., 2012). Some of these explorative trials did show a trend to improvement, but significant clinical effects were not reported. For example, after oral administration of 4,000-40,000 IU/day of 25-OH-D3 for 28 weeks, a significant decrease in the number of new lesions as assessed by magnetic resonance imaging (MRI) was observed, but disease progression and relapse activity were not affected (Kimball et al., 2007). However, despite the limited clinical efficacy of published vitamin D intervention studies, the studies indicate that systemic vitamin D therapy is well tolerated since the trials (most of which administered doses of 1,000 to 40,000 IU/day of 25-OH-D3) recorded no adverse effects (for a review, see Fletcher et al., 2012). Only the study performed with active 1,25-(OH)₂D3 reported hypercalcemia in those individuals who did not adhere to the recommended dietary restriction of 2.5 µg/day (Wingerchuk et al., 2005).

### IX.3. Therapeutic effective doses of glucocorticoids.

Most preferred glucocorticoids for the method of the present invention are those which exhibit a high anti-inflammatory potencywhich is proportional to their glucocorticoid potencyestablished for their capacity to elevate glycemia. Glucocorticoids with a high anti-inflammatory potenca include but not limited to dexamethasone and betamethasone (for a review, see Longui, 2007). However, glucocorticoids with a moderate anti-inflammatory potency such as prednisone, prednisolone, methylprednisolone, and triamcinolone, as well as those with a lower anti-inflammatory potency such as cortisone and hydrocortisone are also applicable for the method of the present invention.

A dexamethasonedoseof0.25 mg/m²/daycorresponds to 2.5 mg/m²/day ofprednisolone and hydrocortisone 10mg/m²/day (for a review, see Gupta and Bhatia, 2008).

All of these glucocorticoids have been studied in a variety of animal models and evaluated in clinical trials. For example, in mice dexamethasone has been administered by i.p. injection at doses of 10-40 µg/20-g mouse (0.5-2.0 mg/kg) for 7 days, leading to a 30% decrease in the number of intestinal VDRs (Hirst and Feldman, 1982a). In rats, dexamethasone has been administered at doses of 0.15-7.5 mg/150-g female rat (1.0-50.0 mg/kg) for 7 days (Hirst anfd Feldman, 1982b).

In clinical trials, different glucocorticoids and varying combinations thereof have been evaluated. For example, several randomized controlled trials comparing dexamethasone with prednisolone in the treatment of acuteasthma exacerbations in children have been published. One study compared emergency department (ED) treatment with an initial dose of oralprednisolone 2 mg/kg (max. 60 mg) followed by 1 mg/kgdaily for four days with oral dexamethasone 0.6 mg/kg(max. 16 mg) daily for two days (Qureshi et al., 2001).

Another study compared ED treatment with an initial dose of oral prednisolone 1 mg/kg (max. 30 mg) followed by 1 mg/kgtwice daily for five days with a single dose of oral dexamethasone 0.6 mg/kg (max. 18 mg) (Altamimi et al., 2006).

Still another study compared ED treatment with a single dose of prednisolone 2 mg/kg (max. 80 mg) followed by 1 mg/kg (max.30 mg) twice daily for five days with a single dose of0.6 mg/kg oral dexamethasone (max. 16 mg) followed by one dose of 0.6 mg/kg oral dexamethasone to take the next day (Greenberg et al., 2008).

### IX.7. Routes of administration of therapeutic compositions

Routes of administration of the compositions of the present invention by injection or by implantation include but are not limited to subcutaneous, intradermal, intramuscular, nasal, transbucal, transmucosal, sublingual, rectal, vaginal, intraocular, or topical administration.

For mucosal immunization, the physicochemical characteristics of the administered components and the delivery vehicle have to be adjusted to stimulate their uptake through the various mucosal routes. For example, alum salts are ineffective when administered by the oral or nasal route. In contrast, cationic chitosan derivatives are of special interest in nasal delivery because of their excellent biocompatibility and mucoadhesive nature (Hagenaars et al., 2010). For example, a thermal-sensitive hydrogel which was formulated as intranasal vaccine with N[(2-hydroxy-3-trimethylammonium)propyl] chitosan chloride (HTCC) and α,β-glycerophosphate, was shown to significantly prolong the antigen residence time in the nasal cavity and to enhance the transepithelial transport via the paracellular routes (Wu et al., 2012).

For allergen-specific immunotherapy the subcutaneous route represents the gold standard. The sublingual-swallow route has emerged as a promising alternative, although less effective than the subcutaneous route. Local side effects of itching and swelling in the mouth are common although, in general, trivial and require no treatment and only rarely result in discontinuation of therapy. The intranasal route has also been shown to be effective, although this route is less attractive for patients and local side effects may require pre-treatment with antihistamines or cromoglycate. For these reasons, the intranasal route has not been widely taken up. The oral route is not currently recommended, although there has been a recent resurgence of interest in the use of microencapsulated extracts which may avoid gastric digestion and facilitate uptake within the small bowel. The inhaled route is not recommended on account of unacceptable side effects.

For autoantigen-specific immunotherapy the subcutaneous route represents also one of the most promising routes. As demonstrated in a recent study, the s.c.route of administration proved to be more effective than the intranasal route for administration of the nine-residue N-terminal peptide of MBP (MBP Ac1-9) fo the treatment of EAE, with a 1,000-fold lower dose of antigen being effective for anergy induction when compared with previous studies (Burton et al., 2014).

Preferred routes of administration of the compositions of the present invention include intradermal and subcutaneous administration. Both routes allow administration of therapeutically sufficient quantities of hydrogel-embedded tolerizing PS-lipopsomes and target peripheral dendritic cells (DCs) including epidermal Langerhans cells (LCs) and dermal DCs which provide a superior ability to generate Tregs in vivo as compared to lymphoid-resident DCs (Idoyaga et al., 2013).

The DC lineage is heterogeneous and can be classified on the basis of phenotype and origin. Lymphoid tissues, i.e., spleen and tissue-draining lymph nodes (LNs), contain lymphoid-resident DCs that arise from blood-borne precursors and can be loosely categorized as CD8⁺ and CD8⁻ DCs, expressing DEC205 (DEC) and DCIR2, respectively. These lymphoid-resident DCs rapidly take up antigens from the lymph and bloodstream for presentation to T cells. A second group of DCs are migratory DCs, which traffic from peripheral tissues to the draining LN charged with tissue self antigens. The nature of migratory DCs depends on the site of LN drainage. In skin draining LNs (sLNs), migratory DCs include epidermal Langerhans cells (LCs) and dermal DCs, which consist of two main subsets, CD103⁺ and CD11b⁺ DCs. Lymphoid-resident DCs and migratory DCs have different roles in the induction of immune responses (for a review, see Villadangos and Schnorrer, 2007). As demonstrated in a recent study (Idoyaga et al., 2013), Langerin⁺ migratory DCs (both dermal migratory CD103⁺ DCs and LCs co-express Langerin) induce antigen-specific Foxp3⁺ Tregs more potently than lymphoid-resident DCs in vivo.Furthermore, in vivo experiments in bone marrow chimeric mice have demonstrated that LCs and dermal CD103⁺ migratory DCs generate Tregs to an equivalent degree.It should be noted, however, that the Treg-inducing ability of Langerin⁺ migratory DCs is not restricted to skin DCs and is not determined by any particular receptor, as delivery of a self antigen, myelin oligodendrocyte glycoprotein, to lung Langerin⁺ migratory DCs also generated antigen-specific Tregs.Most important, the study also demonstrates that targeting a self antigen to Langerin⁺ migratory DCs, but not lymphoid-resident DCs, provides a very effective strategy for the treatment of autoimmune diseases.

### IX.8. Therapeutic protocols

In one embodiment, the present invention discloses therapeutic protocols including dose escalation protocols and injection protocols.

### IX.8.1. Dose escalation protocols.

Allergen-specific immunotherapy typically involves administration of escalating doses of allergen in the early phase of treatment, before a high maintenance dose is reached, resulting in allergic desensitization. It is widely accepted that use of dose escalation strategies minimizes the risk of adverse effects associated with allergen-specific immunotherapy, which may range from mild symptoms to anaphylaxis.

Same considerations apply to autoantigen-specific immunotherapy of autoimmune diseases, since dose escalation permits administration of larger antigen doses. Antigen dose plays a critical role in determining the efficacy of immunotherapy, and a dose escalation protocol is imperative to allow safe s.c. administration of the high antigenic doses required for efficient tolerance induction.

In light of these considerations, a dose escalation strategy for efficient self-antigen-specific tolerance induction has been developed recently using the subcutaneous (s.c.) route (Burton et al., 2014), since mucosal routes of administration have proven safe and effective in animal models of allergy and autoimmunity, but have not translated well to the clinic.

Tolerance induced by escalating dose immunotherapy (EDI) was effective in EAE models whether administered prophylactically or therapeutically (Burton et al., 2014).Using the Tg4 T-cell receptor (TCR) transgenic model of EAE (where >90% of CD4⁺ T cells recognize the nine-residue N-terminal peptide of MBP;MBP Acl-9), the authors show that self-antigen-specific tolerance can be effectively induced via the subcutaneous (s.c.) route, eliciting IL-10-secreting CD4⁺ T cells with an anergic, regulatory phenotype. At each consecutive stage of EDI the sequential modulation of CD4⁺ T-cell phenotype has been characterized. EDI minimized CD4⁺ T-cell activation and proliferation during the early stages of immunotherapy, preventing excessive systemic cytokine release. Furthermore, the s.c.route of administration proved to be more effective than the intranasal route, with a 1,000-fold lower dose of antigen being effective for anergy induction when compared with previous studies. The ability of cells to secrete IL-10, which serves as a promising mediator of effective antigen-specific immunotherapy (Sabatos-Peyton et al., 2010), and to suppress EAE increased in a dose-dependent manner (Burton et al., 2014).

Most importantly, the dose escalating approach has been successfully used in recent clinical trials (for a review, see Garber, 2014). In one trial, nine patients with MS received a single injection of manipulated immune cells, in escalating doses. The four patients receiving the highest doses showed a reduction in the number of T cells targeting self-antigens (Lutterotti et al., 2013).The most promising trial included 30 patients with relapsing-remitting MS (Walczak et al., 2013), randomized in 1 of 3 arms to receive placebo (n=10 patients), a mixture of 1 mg of PLP₁₃₉₋₁₅₁, 1 mg of MOG₃₅₋₅₅, and 1 mg of MBP₈₅₋₉₉ (n=16), or a mixture of 10 mg of PLP₁₃₉₋₁₅₁, 10 mg of MOG₃₅₋₅₅, and 10 mg of MBP₈₅₋₉₉ (n=4). Myelin antigens were dissolved in phosphate-buffered saline and were applied transdermally in an adhesive skin patch placed on the right upper arm that was changed once per week for 4 weeks and then once per month for 11 months.The effect of myelin peptides was detected at as early as 3 months of treatment, as demonstrated by the reduction in the cumulative number of Gd⁺ lesions vs the placebo group at this point of the study. In this trial, however, the higher dose of myelin peptides (10 mg) was less efficacious than the lower dose, but this result may be due to the low number of patients in the high-dose myelin peptide treatment group.

### IX.8.2. Injection protocols

In one embodiment, hydrogel-embedded tolerizing PS-liposomes are administered by at least one s.c. injectionsusing atherapeutically optimal dosis of liposome-ecapsulated allergens or autoantigens based on the results of a prior dose escalation study as described (Burton et al., 2014). Subsequent s.c. injections are performed at one-week or two-weeks intervals. Preferred are two-weeks intervals to allow for a complete release of hydrogel-embedded tolerizing PS-liposomes.

The number of required s.c.injections is determined for each patient individually by analysis (combination of microarray analyses and real-time PCR including RT-PCR)of the sequential modulation of CD4⁺ T-cell phenotype towards IL-10-secreting CD4⁺ T cells with an anergic, regulatory phenotypeafter each injection step (Burton et al., 2014).In some cases, a single injection of hydrogel-embedded tolerizing PS-liposomes may also be sufficient to induce IL-10-secreting CD4⁺ T cells with an anergic, regulatory phenotype to an extent that allows suppression of allergic or autoimmune disease symptoms. For example, a single s.c.injection of tolerizing liposomes loaded with antigen and NF-κB inhibitors into mice suffering from antigen-induced inflammatory arthritis has been demonstrated to reduce the mean clinical score by approximately 50% within four days (Capinni et al., 2009) .However, in the majority of cases repeated s.c. injections of hydrogel-embedded tolerizing PS-liposomes may be necessary to induce tolerance via a gradual establishment of a regulatory CD4⁺ T-cell phenotype.

The gradual establishment of a regulatory CD4⁺ T-cell phenotype is characterized by expression of specific negative co-stimulatory molecules and transcription factors, in addition to the regulatory cytokine IL-10, all of which are used surrogate markers for allergen/autoantigen-specific tolerance induction according to the method of the present invention. Transcription factors previously associated with IL-10 expression includeMaf, Ahr and Nfil3 (Pot et al., 2009; Motomura et al., 2011; Apetoh et al., 2010) . The induction of IL-21 expression is also noteworthy, as IL-21 contributes to the IL-27-driven production of IL-10 in murine T cells (Pot et al., 2009). The most notable correlation with effective immunotherapy is the induction of a set of negative costimulatory molecules including PD-1, LAG-3, TIM-3 and TIGIT. Some of these have previously been associated with T cell exhaustion (Wherry, 2011), while others have been described as markers of IL-10-secreting Tr1 cells (Gagliani et al., 2013; Okamura et al., 2009). Recently, a positive correlation between IL-10 production and the expression of LAG-3, TIGIT, PD-1 and TIM-3 was demonstrated (Burton et al., 2014). However, expression of these markers is not uniquely restricted to the IL-10⁺ population; only 11% of LAG-3⁺ cells are IL-10⁺ and ∼50% of Tim-3⁺ or TIGIT⁺ cells are IL-10⁺ (Burton et al., 2014). These results suggest that while LAG-3 and PD-1 are good markers of the anergic CD4⁺ T-cell population induced by EDI, TIGIT and TIM-3 are better discriminators of IL-10-secreting cells induced by immunotherapy (Burton et al., 2014).CD49b was also found to correlate with IL-10 expression in CD4⁺ T cells from autoantigen-treated mice; however, within the LAG-3⁺CD49b⁺ population, only 33% of cells were found to express IL-10(Burton et al., 2014).

Although the necessary extent of established regulatory CD4⁺ T-cell phenotypes for the induction of tolerance may vary for different patient and also for different allergic and autoimmune diseases, a recent successful, escalating dose immunotherapeutic approach for the treatment of EAE provides valuable cornerstones (Burton et al., 2014). In this study, mice were treated every 3-4 days six times s.c. with an escalating dose of autoantigen (escalatingfrom 0.08 µg to 0.8 µg and then to4 x 8 µg, or escalating from 0.08 µg to 0.8 µg and then to 8 µg and finally to 3 x 80 µg). Induction of tolerance was associated with a percentage of CD4⁺ T cells expressing IL-10, c-Maf or LAG-3 in at least 50% of the cells.A rising percentage of TIGIT⁺ cells also accumulated during autoantigen-specific immunotherapy (20% of activated CD4⁺ T cells). The proportion of cells expressing TIM-3 remained relatively stable throughout the treatment, while the percentage of PD-1⁺ cells increased upon initial CD4⁺ T-cell activation and further increased during the later stages of the treatment.

In another embodiment, hydrogel-embedded tolerizing PS-liposomes are administered analogous to allergen-specific immunotherapy protocols by one-weekly or two-weekly s.c. injections for at least three weeks during an updosing phase (escalating dosing phase), followed by monthly maintenance injections. At each consecutive stage of the escalating dose immunotherapy the sequential modulation of CD4⁺ T-cell phenotype towards IL-10-secreting CD4⁺ T cells with an anergic, regulatory phenotype isanalyzed as described above.

Allergen-specific immunotherapy protocols, in general, involve weekly injections via the subcutaneous route 8-16 weeks during an updosing phase, followed by monthly maintenance injections (empirically this has been extended in some centres to 6-8 weeks) for a period of 3-5 years. Cluster immunotherapy updosing schedules may involve repeated injections at each clinic visit. Rush protocols which may involve repeated updosing injections in order to achieve maintenance doses within several hours are applicable to venom sensitive patients, although are unsuitable for patients with inhalant allergies in view of the marked increased occurrence of side effects.

Autoantigen-specific immunotherapy protocols, in general, involve also repeated administrations of autoantigens. For example, immunotherapy of RA patients with recombinant human cartilage glycoprotein-39 (HC gp-39) has been performed by intranasal administration of 30, 150, 300 or 600 µg of HC gp-39 once a week for 13 weeks (Landewe et al., 2010) .In another study, immunotherapy of MS patients has been performed by transdermal administration of 3 myelin antigens, each at a dosage of 1 or 10 mg in an adhesive skin patch placed on the right upper arm that was changed once per week for 4 weeks and then once per month for 11 months (Walczak et al., 2013).

### EXAMPLES

The following examples are intended to illustrate the present invention.

### EXAMPLE 1: TOLERIZING PS-LIPOSOMES

This example describes the synthesis of PS-liposomes containing one of the following compounds including calcipotriol, dexamethasone phosphate (DexP), ovalbumin (OVA), methylated BSA (mBSA), myelin oligodendrocyte glycoprotein (MOG)-derived peptide 35-55 (MOG(35-55)), and combinations of these compounds.

### 1.1.Synthesis of PS-liposomes

This example describes the synthesis of unilamellar PS-liposomes from a lipid mixture of phosphatidyldserine (PS) (either1,2-dipalmitoyl-sn-glycero-3-phospho-L-serine sodium salt (Sigma-Aldrich), 1-palmitoyl-2-oleoyl-sn-3-glycerophospho -L-serine (POP-L-S), or bovine brain phosphatidyldserin (Avanti Polar Lipids)), phosphatidylcholine (PC) (either1,2-dipalmitoyl-sn-glycero-3-phosphocholine (DMPC; Sigma-Aldrich), 1-palmitoyl-2-oleoyl-sn-3-glycerophosphocholine (POPC; Avanti Polar Lipids), or egg phosphatidylcholine (egg-PC; Avanti Polar Lipids)), and cholesterol (CH; Avanti Polar Lipds) at a ratio of 30:30:40 (PS to PC to CH)according to Hoffmann et al. (2005).

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask and dried by rotary evaporation to prepare a thin lipd film. Thereafter, the flask is placed in a desiccator for at least one hour to completely remove the solvent. Then, 1.7 ml of phosphate-buffered saline (PBS) is added (approx. 35 mg total lipid/ml) and multilamellar vesicles are generated by intense vortex dispersion.For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmole (approx. 35mg) of lipd/ml (59 mM liposomal suspension). Unilamellar PS-liposomes prepared by this procedure have been shown to disperse uniformly in physiological medium at a concentration of 60 mM total lipid due to repulsion forces (Harel-Adar et al., 2011).

The degree of PS exposure on liposomes is assessed by binding of FITC-annexin V to surface-exposed PS and subsequent analysis by FACS.

### 1.2.Synthesis of PS-liposomes containing encapsulated allergen or antigen

This example describes the synthesis of unilamellar PS-liposomes containing either ovalbumin (OVA), methylated BSA (mBSA), or the myelin oligodendrocyte glycoprotein (MOG)-derived peptide 35-55 (MOG(35-55)).

OVA is used as model allergen for the induction and treatment of OVA-induced allergy and airway inflammation in mice (Heine et al., 2014), mBSA is used as model antigen for the induction and treatment of antigen-induced arthritis (AIA) in mice (Capini et al., 2009), and MOG(35-55) is used as model antigen for the induction and treatment of EAE in mice (Schweingruber et al., 2011; Wüst et al., 2008). The sequences of murine and rat MOG(35-55) are identical, whereas human MOG (35-55) contains one different amino acid residue. For this example, the murine MOG(35-55) peptide (MEVGWYRSPFSRVVHLYRNGK; MW 2582; purity >95%; AnaSpec, USA) is used.

### 1.2.1. Solubility of OVA, mBSA nd MOG (35-55).

In water, the solubility of OVA is about one gram per ml of water. Methylated BSA (mBSA) has been used for immunization at a concentration of 50 mg/ml of 0.9% NaCl (Gent et al., 2014). The murine MOG(35-55) has been used for the induction of EAE at a concentration of 3 mg/ml PBS (Sharp et al., 2008). In water, the solubility of murine MOG(35-55) is approx. 2 mg/ml (product information of Abbiotec).

### 1.2.2. Entrapment efficiency of soluble protein.

Factors affecting the liposomal entrapment efficiency of soluble protein allergens/antigens include liposome size and zeta potential. For example, the entrapment efficiency of OVA in anionic liposomes was found to be significantly lower (21%-35%) than in cationic liposomes (43%-69%) (Brgles et al., 2008).In order to achieve sufficient entrapment of OVA, mBSA and MOG(35-55), resuspension of thin lipid films produced by evaporation is performed with a PBS solution containing relatively high concentrations of the proteins.

### 1.2.3. Synthesis of PS-liposomes containing encapsulated OVA, mBSA, or MOG(35-55)

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask and dried by rotary evaporation to prepare a thin lipd film. Thereafter, the flask is placed in a desiccator for at least one hour to completely remove the solvent. Then, 1.7 ml of phosphate-buffered saline (PBS) containing 17 mg OVA (Sigma-Aldrich), or 17 mg mBSA (Sigma-Aldrich), or 3.4 mg murine MOG(35-55) (2.0 mg/ml; AnaSpec, USA) is added. Multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.7 ml of PBS and vortexed to resuspend the liposomes.Optionally, residual unincorporated OVA, mBSA, or MOG(35-55) that has not been removed by the centrifugation step, may be removed by subsequent size exclusion chromatography on a Sephadex G50 column.

### 1.2.4. Analysis of entrapment efficiency.

The concentration of encapsulated allergen or antigen is determined by subjecting different volumes of PS-liposomes to SDS-PAGE electrophoresis in parallel with known amounts of allergen/antigen and visualizing the protein by Coomassie blue staining. The density of the bands is determined by gel scanning and densitometry analysis. The concentration of encapsulated MOG(35-55) is determined by ELISA using rabbit anti-MOG(35-55) polyclonal anibodies (AnaSpec, USA) after dissolution of the liposomes in 1% (v/v) Triton X-100. The phospholipid content is determined with a phosphate assay in the organic phase after extraction of the liposomal preparations with chloroform (Rouser et al., 1970).

Based on the reported entrapment efficiency of albumin in anionic liposomes (Brgles et al., 2008), encapsulation of approx. 2-2.5 mg of OVA or mBSA/ml PS-liposome suspension (20 pmol (12 mg) of lipd/ml)is a realistic assumption using the conditions of Example 1.2.3 (encapsulation efficiency of 20-25%). This concentration is in accordance with values reported for the entrapment of OVA in tolerizing PC-liposomes (2-2.5 mg of OVA or mBSA/ml PS-liposome suspension) loaded with lipophilic NF-κB inhibitors (Capini et al., 2009).

As demonstrated in a recent study (Belogurov et al., 2013), liposomal encapsulation of three peptides derived from the myelin basic protein (MBP) at a peptide to lipid ratio /w/w) of 1:330 (0.15 mg peptides/ml:50 mg lipid/ml) provided an encapsulation efficiency of more than 90%. Since in this example a significantly higher peptide to lipid ratio of 1:18 (2 mg peptide/ml: 35 mg/lipid/ml) is used (approx. 6-fold higher if related to one MBP-derived peptide), the encapsulation efficiency of MOG(35-55) can be assumed to be in the range of 30-50%.

The final liposomal suspension contains approx. 59 µmole (approx. 35 mg) of lipd/ml (59 mM liposomal suspension), and approx. 2-2.5 mg OVA or mBSA/ml (based on 20-25% encapsulation efficiency), or approx. 0.6-1.0 mg MOG(35-55)/ml (based on 30-50% encapsulation efficiency).

### 1.3.Synthesis of PS-liposomes containing lipid bilayer-incorporatedcalcipotriol

This example describes the synthesis of unilamellar PS-liposomes containing the vitamin D3 derivative calcipotriol (Tocris Bioscience, UK).

### 1.3.1. Incorporation of calcipotriol into lipid bilayers.

Calcipotriol molecules are incorporated into the lipid bilayer and intercalate between the hydrocarbon chains of phospholipid molecules (Merz and Sternberg, 1994).Using calcipotriol for incorporation into liposomes made of DMPPC or egg-PC in a molar ratio of calcipotriol (MW 412.6) to lipd of 0.03 to 1, incorporation rates of more than 80%have been reported (Merz and Sternberg, 1994).

Since in this example, a two-fold lower molar ratio of calcipotriol to lipd of 0.015 to 1 is used, an incorporation rate of at least 85% can be assumed.

### 1.3.2. Synthesis of PS-liposomes containing calcipotriol

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask, mixed with a stock solution of calcipotriol in methanol(10 mg/ml) in a molar ratio of calcipotriol to lipd of 0.015 to 1.0 (620 µg calcipotriol corresponding to approx. 1.5 µmole), and dried by rotary evaporation to prepare a thin lipd film.Thereafter, the flask is placed in a desiccator for at least one hour to completely remove the solvent. Then, 1.7 ml of phosphate-buffered saline (PBS) is added and multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.7 ml of PBS and vortexed to resuspend the liposomes.

### 1.3.3. Analysis of incorporation efficiency

The calcipotriol concentration in the liposomal suspensions is determined by UV absorption at 252 nm (molar extinction coefficient of 42,000; Plum et al., 2004) after dissolution of the liposomes in ethanol. Alternatively, the calcipotriol concentration in the liposomal suspensions can be determined by reversed phase HPLC using a C18-column and acetonitrile:water(77:23) as elution agent (Cirunay et al., 1998). Calcipotriol is detected by UV absorption at 263 nm. The phospholipid content is determined as described in Example 1.2.

The final liposomal suspension contains approx. 59 µmole (approx. 35 mg) of lipd/ml (59 mM liposomal suspension), and approx. 310 µg (751 nmole) calcipotriol/ml liposomal suspension, based onan incorporation rate of 85%.

### 1.4.Synthesis of PS-liposomes containing calcipotriol and encapsulatedallergen or antigen

This example describes the synthesis of unilamellar PS-liposomes with lipid bilayer-incorporated calcipotriol, containing either encapsulated ovalbumin (OVA), methylated BSA (mBSA), or MOG(35-55) (for more information, see Example 1.2.).

### 1.4.1. Synthesis of PS-liposomes containing calcipotriol and OVA, mBSA, or MOG(35-55)

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask, mixed with a stock solution of calcipotriol in methanol(10 mg/ml) in a molar ratio of calcipotriol to lipd of 0.015 to 1.0 (620 µg calcipotriol corresponding to approx. 1.5 µmole), and dried by rotary evaporation to prepare a thin lipd film. Thereafter, the flask is placed in a desiccator for at least one hour to completely remove the solvent. Then, 1.7 ml of phosphate-buffered saline (PBS) containing 17 mg OVA (Sigma-Aldrich), or 17 mg mBSA (Sigma-Aldrich), or 3.4 mg murine MOG(35-55) (2.0 mg/ml; AnaSpec, USA) is added. Multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.7 ml of PBS and vortexed to resuspend the liposomes. Optionally, residual unincorporated OVA, mBSA, or MOG(35-55) that has not been removed by the centrifugation step, may be removed by subsequent size exclusion chromatography on a Sephadex G50 column.

### 1.4.2. Analysis of entrapment efficiencies.

The concentration of encapsulated allergen or antigen is determined by subjecting different volumes of PS-liposomes to SDS-PAGE electrophoresis in parallel with known amounts of allergen/antigen and visualizing the protein by Coomassie blue staining. The density of the bands is determined by gel scanning and densitometry analysis. The concentration of encapsulated MOG(35-55) is determined by ELISA using rabbit anti-MOG(35-55) polyclonal anibodies (AnaSpec, USA) after dissolution of the liposomes in 1% (v/v) Triton X-100. The calcipotriol concentration in the liposomal suspensions is determined by UV absorption at 252 nm (molar extinction coefficient of 42,000; Plum et al., 2004) after dissolution of the liposomes in ethanol. Alternatively, thecalcipotriol concentration in the liposomal suspensions is determined by reversed phase HPLC using a C18-column and acetonitrile/water (77:23) as elution agent (Cirunay et al., 1998). Calcipotriol is detected by UV absorption at 263 nm. The phospholipid content is determined as described in Example 1.2.

The final liposomal suspension contains approx. 59 µmole (approx. 35 mg) of lipd/ml (59 mM liposomal suspension), approx. 310 µg (751 nmol) calcipotriol/ml liposomal suspension (based on 85% incorporation rate), and approx. 2-2.5 mg OVA or mBSA/ml (based on 20-25% encapsulation efficiency), or approx. 0.6-1.0 mg MOG(35-55)/ml(based on 30-50% encapsulation efficiency).

### 1.5.Synthesis of PS-liposomes containing water-soluble dexamethasone sodium phosphate(DexP).

This example describes the synthesis of unilamellar PS-liposomes containing DexP (liposomal DexP).

### 1.5.2. Concentration range of liposomal DexP

In the study of Hegeman et al. (2011), liposomal DexP has been administered i.v. at a concentration of 11.2 µg DexP/20-g mouse (adult male C57BL/6 mice with a body weight of 20-24g). The DexP to lipid ratio was 28 µg DexP/µmole lipid (comprising PC, cholesterol and PE at a molar ration of 55:40:5). In another study (Anderson et al., 2010), liposomal DexP has been administered i.v. at a concentration of 1 mg DexP/kg body weight for 3 days, corresponding to three injections of 20 µg DexP/20-g mouse. The DexP to lipid ratio was 40 µg DexP/µmole lipid (comprising DPPC, DPPG and cholesterol at a molar ration of 50:10:40).A more than three-fold higher amount of liposomal DexP (3.75 mg liposomal DexP/kg body weight, corresponding to 75 µg/20-g mouse or 563 µg/150-g female rat) has been administerd i.v to rats 6, 24 and 48 hours after induction of antigen-induced arthritis (US20060147511A1).

For comparison, non-liposomal DexP has been administered i.v. in the study of Hegeman et al. (2011) at a concentration of 20 µg DexP/20-g mouse. In the study of Hirst and Feldman (1982a), non-liposomal DexP has been administered by i.p. injection in mice at doses of 10-40 µg/20-g mouse (0.5-2.0 mg/kg) for 7 days.

### 1.5.1. Solubility of DexP.

In water DexP (MW 516.4) is soluble at a concentration of 50 mg/ml (product information of Santa Cruz Biotechnology), in PBS at a concentration of at least 25 mg/ml (Anderson et al., 2010), and in 10 mM HEPES and 135 mM NaCl, pH 6.7, at a concentration of at least 50 mg/ml (Koning et al., 2006).

### 1.5.3. Liposomal encapsulation efficiency of DexP

The liposomal encapsulation efficiency of DexP depends on the concentration of DexP, high DexP concentrations result in low encapsulation efficiencies and vice versa. For example, the addition of 1000 mg DexP in 10 ml of sterilized water with 1093 mg of a lipid film comprising PC, PE and cholesterol, resulted in capsulation efficiencies ranging from 4.8% to 17.6% depending on the kinds of lipids used ifor the preparation of unilammelar liposomes (US20090226509A1).

A comparable liposomal encapsulation efficiency of DexP has been reported by Koning et al. (2006) . In this study, a lipid film (comprising PC, cholesterol, and PE in a molar ratio of 1.85:1:0.15) was hydrated in 10 mM HEPES, 135 mM NaCl, pH 6.7, containing 50 mg/ml DEXP at a ratio of 1 mg DEXP/µmole total lipid.Liposomal DEXP contents (detected via absorbance at 254 nm) varied between 30 and 60 µg DEXP/pmole total liposomal lipid, representing an encapsulation efficiency of 3-6%.

Based on the study of Koning et al. (2006), a liposomal encapsulation efficiency for DexP in the range of approx. 10% is assumed, since in this example a five-fold lower DexP:lipid ratio of 0.2 mg DexP/µmoletotal lipd is used for hydration of the lipid film.

### 1.5.4. Synthesis of PS-liposomes containing encapsulated DexP

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask and dried by rotary evaporation to prepare a thin lipd film. Thereafter, the flask is placed in a desiccator for at least one hour to completely remove the solvent. Then, 1.7 ml of PBS containing 20mg DexP (Dex-ratio-pharm, Ratiopharm) is added. Multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.7 ml of PBS and vortexed to resuspend the liposomes.Optionally, residual unincorporated DexP that has not been removed by the centrifugation step, may be removed by subsequent size exclusion chromatography on a Sephadex G50 column.

### 1.5.5.Analysis of entrapment efficiencies.

For analysis of the liposomal entrapment efficiency, the phospholipid content is determined as described in Example 1.2. The aqueous phase after extraction is used to determine DexP content using a spectrophotometric method (Singh and Verma, 2008). The method involves oxidation of the corticosteroid by iron (III) and subsequent complexation of iron (II) with potassium hexacynoferate (III), forming a bluish green coloured complex with maximum absorbance at 780 nm (Beer's law range: 10-50 µg/ml; molar absorptivity (M⁻¹cm⁻¹) : for DexP 0.55 x 10⁴). Alternatively, the DexP content is determined by reversed-phase HPLC at 254 nm using a C18-column and methanol:water (1:1) as solvent (Kwak and D'Amico, 1995). Furthermore, anti-DexP polyclonal antibodies (MyBioSource) are commercially available for the determination of DexP by Elisa.

The final liposomal suspension contains approx. 59 µmole (approx. 35 mg) of lipd/ml (59 mM liposomal suspension), and approx. 1.2 mg liposomal DexP/ml(based on 10% encapsulation efficiency), corresponding to 20 µgDexP/µmole lipid which is comparable to 40 µg DexP/µmole lipid reported by Anderson et al. (2010), and 28 µg DexP/µmole lipid reported by Hegeman et al. (2011).

### 1.6.Synthesis of calcipotriol-loaded PS-liposomes containing encapsulated DexP

This example describes the synthesis of unilamellar PS-liposomes containing lipid blilayer-incorporated calcipotriol and encapsulated dexamethasone sodium phosphate (DexP). Encapsulation of dexamethasone sodium phosphate (DexP; Dex-ratio-pharm, Ratiopharm)in PS-liposomes loaded with calcipotriol (Tocris Bioscience, UK)is performed according to sections 1.3. and 1.5.

### 1.6.1. Synthesis of calcipotriol-loaded PS-liposomes containingencapsulated DexP

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask, mixed with a stock solution of calcipotriol in methanol(10 mg/ml) in a molar ratio of calcipotriol to lipd of 0.015 to 1.0 (620 µg calcipotriol corresponding to approx. 1.5 µmole), and dried by rotary evaporation to prepare a thin lipd film. Thereafter, the flask is placed in a desiccator for at least one hour to completely remove the solvent. Then, 1.7 ml of PBS containing 20 mg DexP (Dex-ratio-pharm, Ratiopharm) is added. Multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.7 ml of PBS and vortexed to resuspend the liposomes. Optionally, residual unincorporated DexP that has not been removed by the centrifugation step, may be removed by subsequent size exclusion chromatography on a Sephadex G50 column.

### 1.6.2.Analysis of liposomal entrapment efficiencies.

For analysis of the liposomal entrapment efficiency, the phospholipid content is determined as described in Example 1.2. The aqueous phase after extraction is used to determine the DexP content as described in Example 1.5. The calcipotriol concentration in the liposomal suspensions is determined as described in Example 1.3.

The final liposomal suspension contains approx. 59 µmole (approx. 35 mg) of lipd/ml (59 mM liposomal suspension), approx. 310 µg (751 nmol) calcipotriol/ml liposomal suspension (based on 85% incorporation rate), and approx. 1.2 mg liposomal DexP/ml (based on 10% encapsulation efficiency), corresponding to 20 µgDexP/µmole lipid

### 1.7.Synthesis of PS-liposomes containing encapsulated DexP and allergen or antigen

This example describes the synthesis of unilamellar PS-liposomes containing encapsulated dexamethasone sodium phosphate (DexP)) and encapsulated ovalbumin (OVA), methylated BSA (mBSA), or MOG(35-55) (for more information, see Examples 1.2. and 1.5.).

### 1.7.1. Synthesis of PS-liposomes containing DexP and OVA, or DexP and mBSA, or DexP and MOG(35-55)

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask and dried by rotary evaporation to prepare a thin lipd film. Thereafter, the flask is placed in a desiccator for at least one hour to completely remove the solvent. Then, 1.7 ml of phosphate-buffered saline (PBS) is added containing 17 mg OVA (Sigma-Aldrich) and 20 mg DexP (Dex-ratio-pharm, Ratiopharm), or 17 mg mBSA (Sigma-Aldrich) and 20 mg DexP (Dex-ratio-pharm, Ratiopharm), or 3.4 mg murine MOG(35-55) (2.0 mg/ml; AnaSpec, USA) and 20 mg DexP (Dex-ratio-pharm, Ratiopharm). Multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.7 ml of PBS and vortexed to resuspend the liposomes. Optionally, residual unincorporated OVA, mBSA, or MOG(35-55) that has not been removed by the centrifugation step, may be removed by subsequent size exclusion chromatography on a Sephadex G50 column.

### I.7.2. Analysis of entrapment efficiencies.

For analysis of the liposomal entrapment efficiency, the phospholipid content is determined as described in Example 1.2. The aqueous phase after extraction is used to determine the DexP content as described in Example 1.5. The concentration of encapsulated OVA, mBSA or MOG(35-55) is determined as described in Example 1.2.

The final liposomal suspension contains approx. 59 µmole (approx. 35 mg) of lipd/ml (59 mM liposomal suspension), approx. 1.2 mg liposomal DexP/ml (based on 10% encapsulation efficiency), corresponding to 20 µgDexP/µmole lipid, and approx. 2-2.5 mg OVA or mBSA/ml (based on 20-25% encapsulation efficiency), or approx. 0.6-1.0 mg MOG(35-55)/ml (based on 30-50% encapsulation efficiency).

### 1.8.Synthesis of PS-liposomes containing calcipotriol, DexP, and allergen or antigen

This example describes the synthesis of unilamellar PS-liposomes with lipid bilayer-incorporated calcipotriol, containing encapsulated dexamethasone sodium phosphate (DexP) and encapsulated ovalbumin (OVA), methylated BSA (mBSA), or MOG(35-55). Encapsulation of dexamethasone sodium phosphate (DexP; Dex-ratio-pharm, Ratiopharm), OVA (Sigma-Aldrich), mBSA (Sigma-Aldrich) or MOG(35-55) (AnaSpec, USA) in PS-liposomes loaded with calcipotriol (Tocris Bioscience, UK)is performed according to Examples 1.3. and 1.7.

### 1.8.1. Synthesis of calcipotriol-loaded PS-liposomes containingDexP and OVA, or DexP and mBSA, or DexP and MOG(35-55)

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask, mixed with a stock solution of calcipotriol in methanol(10 mg/ml) in a molar ratio of calcipotriol to lipd of 0.015 to 1.0 (620 µg calcipotriol corresponding to approx. 1.5 µmole), and dried by rotary evaporation to prepare a thin lipd film. Thereafter, the flask is placed in a desiccator for at least one hour to completely remove the solvent. Then, 1.7 ml of phosphate-buffered saline (PBS) is added containing 17 mg OVA (Sigma-Aldrich) and 20 mg DexP (Dex-ratio-pharm, Ratiopharm), or 17 mg mBSA (Sigma-Aldrich) and 20 mg DexP (Dex-ratio-pharm, Ratiopharm), or 3.4 mg murine MOG(35-55) (2.0 mg/ml; AnaSpec, USA) and 20 mg DexP (Dex-ratio-pharm, Ratiopharm). Multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.7 ml of PBS and vortexed to resuspend the liposomes. Optionally, residual unincorporated OVA, mBSA, or MOG(35-55) that has not been removed by the centrifugation step, may be removed by subsequent size exclusion chromatography on a Sephadex G50 column.

### 1.8.2. Analysis of entrapment efficiencies.

For analysis of the liposomal entrapment efficiency, the phospholipid content is determined as described in Example 1.2. The aqueous phase after extraction is used to determine the DexP content as described in Example 1.5. The calcipotriol concentration in the liposomal suspensions is determined as described in Example 1.3.The concentration of encapsulated OVA, mBSA or MOG(35-55) is determined as described in Example 1.2.

The final liposomal suspension contains approx. 59 µmole (approx. 35 mg) of lipd/ml (59 mM liposomal suspension), approx. 310 µg (751 nmol) calcipotriol/ml liposomal suspension (based on 85% incorporation rate), approx. 1.2 mg liposomal DexP/ml (based on 10% encapsulation efficiency), corresponding to 20 µgDexP/µmole lipid, and approx. 2-2.5 mg OVA or mBSA/ml (based on 20-25% encapsulation efficiency), or approx. 0.6-1.0 mg MOG(35-55)/ml (based on 30-50% encapsulation efficiency).

### EXAMPLE 2:HYDROGEL/PS-LIPOSOME COMPOSITS

This example describes the synthesis and chacterization of thermogelling PLGA-PEG-PLGA hydrogels containing phosphatidylserine (PS)-liposomes.

### 2.1. Synthesis of PLGA-PEG-PLGA hydrogels.

The biodegradable triblock polymer described in this example has a PLG/PEG weight ratio of 2.3 (70/30), and a lactide/glycolide molar ratio of approx. 15/1. Synthesis of the triblock copolymer is performed according to published protocols (Qiao et al., 2005).

### 2.1.1.Copolymer synthesis

Polyethylene glycol (PEG 1000) was purchased from Fluka, poly(DL-lactide) from Sigma, glycolide (1,4-Dioxane-2,5-dione) from Sigma, and stannous 2-ethylhexanoate from Aldrich.

A total of 25 g of DL-lactide, glycolide and PEG are used for polymerization (16.6 g DL-lactide, 0.9 g glycolide, 7.5 g PEG 1000) (PLG/PEG weight ratio of 70/30 (2.3)). Under nitrogen atmosphere, PEG 1000 is dried under vacuum and stirring at 120°C for 2 h in a vigorously dried Erlenmeyer reaction flask. Then the reaction flask is filled with dry argon. DL-lactide and gycolide monomers are added under stirring followed by the addition of Stannous 2-ethylhexanoate (0.2% w/w). Then the tube is sealed under argon. The sealed flask is immersed and kept in an oil bath thermostated at 130°C. After approx. 16 h the flask is cooled to room temperature, and the product is dissolved in cold water. After completely dissolved, the copolymer solution is heated to 80°C to precipitate the copolymer and to remove the water-soluble low molecular weight copolymers and unreacted monomers. The supernatant is decanted, the precipitated copolymer is again dissolved in cold water followed by heating to induce precipitation. This process of dissolution followed by precipitation is repeated three times. Alternatively the polymer can be dissolved in acetonitrile, sterile filtred, and precipitated by mixing with sterile water and heating. Finally, the copolymer is dried under vacuum at room temperature until constant weight.

### 2.1.2.Molecular weight determination

The molecular weight of the copolymer is determined by gel permeation chromatography using polystyrene standards as described by Qiao et al. (2005).

### 2.1.3.Measurement of gelation temperature

The gelation temperature is determined as described by Qiao et al. (2005). A 2 ml transparent vial is filled with 200 µl water solution of the copolymer (20% w/w and 25% w/w), is placed in a water bath. The solution is heated in 1°C steps beginning at 26°C in a thermomixing device (Eppendorf). At each temperature step the gelation is checked by careful inversion of the tube. When the solution is not free-flowing, gelation of the solution occurred, the temperature read from the thermometer is determined as gelation temperature.

### 2.2. Preparation of hydrogel/PS-liposome composits

Different concentrations of the PLGA-PEG-PLGA copolymer of Example 2.1 (22.5% w/w, and 30% w/w) in water are mixed with liposomal suspensions in PBS of Example 1 (1.1.-1.8.)at a ratio of two volumes hydrogel solution to one volume of liposomal suspension.The final concentration of the hydrogel is 15% (w/w) or 20% (w/w) containing liposomes at a concentration of approx. 20 µmole (12 mg) of lipd/ml, and combinations of approx. 100 µg (242 nmole; MW 412.6) calcipotriol/ml, approx. 400 µg liposomal DexP (775 nmole; MW 516.4)/ml, approx. 0.7-0.8 mg OVA or mBSA/ml, and approx. 0.2-0.3 mg MOG(35-55)/ml.

### 2.3. Gelation characteristics of hydrogel/PS-liposome composits

The gelation temperature of hydrogel/PS-liposome composits of Example 2.2is determined as described by Qiao et al. (2005). Transparent vials are filled with 200 µl water containing different concentrations of the copolymer of Example 2.1. (22.5% w/w, and 30% w/w), cooled to 4°C and mixed with 100 µl PBS containing liposomes of Example 1 or 100 µl PBS containing no liposomes. The final concentrations of the copolymer are 15% (w/w) and 20% (w/w) containing liposomes at a concentration of approx. 20 µmole (12 mg) of lipd/ml. The vials are placed in a water bath and each solution is heated in 1°C steps beginning at 20°C in a thermomixing device (Eppendorf). At each temperature step the gelation is checked by careful inversion of the tube. When the solution is not free-flowing, gelation of the solution occurred, the temperature read from the thermometer is determined as gelation temperature.

### 2.4. In vitro degradation of hydrogel/PS-liposome composits

The in vitro degradation behavior of hydrogel/PS-liposome composits of Example 2.3 is evaluated by the mass loss and/or the molecular weight reduction with time upon incubation in PBS.

Samples (0.2 ml) are incubated in PBS, pH 7.4, at 37°C under mild agitation in a water bath. The solid residues are removed from the incubation medium at scheduled time intervals and lyophilized. The samples are weighted and the weight loss is calculated. For determination of the molecular weight reduction, the solid residues are solved in cold water and analyzed by gel permeation chromatography using polystyrene standards as described by Qiao et al. (2005).

### EXAMPLE 3: RELEASE OF PS-LIPOSOMES FROM HYDROGELS

This example describes the in vitro release characteristics of PS-Liposomes with encapsulated FITC-BSA from thermogelling PLGA-PEG-PLGA hydrogels.

### 3.1. Synthesis of thermogelling PLGA-PEG-PLGA hydrogels

The biodegradable triblock polymer described in this example has a PLG/PEG weight ratio of 2.3 (70/30), and a lactide/glycolide molar ratio of approx. 15/1. Synthesis of the triblock copolymer is performed as described in Example 2.1.

### 3.2.Synthesis of FITC-BSA-containing PS-liposomes

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask and dried by rotary evaporation to prepare a thin lipd film. Thereafter, the flask is placed in a desiccator for at least one hour to completely remove the solvent. Then, 1.5 ml of PBS containing 1.5 mg FITC-labeled bovine serum albumin (FITC-BSA, Sigma-Aldrich) is added and multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.5 ml of PBS and vortexed to resuspend the liposomes.The final liposomal suspension contains approx. 66.7 µmol (40.1 mg) of lipd/1.0 ml.

The amount of encapsulated FITC-BSA in liposomes is determined by dissolving the lipid vesicles with 1% (v/v) Triton X-100 and monitoring the absorbance of FITC-BSA at 495 nm. Using the conditions of this example, the encapsulation efficacy is 22% (220 µg FITC-BSA/ml PS-liposome suspension).

### 3.3.In vitro release of FITC-BSA-containing PS-liposomes from hydrogel/liposome composits

The in vitro release of FITC-BSA-containing PS-liposomes from hydrogel/PS-liposome composits is determined after gelling of the hydrogel/PS-liposome composits at 37°C by monitoring the supernatant for the development of absorbance at 495 nm in the presence of Triton X-100.

Vials are filled with 200 µl water containing different concentrations of the copolymer of Example 2.1. (22.5% w/w, and 30% w/w), cooled to 4°C and mixed with 100 µl PBS containing liposomes of Example 3.2. The final concentrations of the copolymer are 15% (w/w) and 20% (w/w) containing liposomes with encapsulated FITC-BSA at a concentration of 22.2µmol lipid/ml (13.3 mg/ml). The reaction mixtures are incubated at 37°C under mild agitation in a water bath until gelling. Thereafter, 1.7 ml PBS, pH 7.4, is added to each sample and incubation at 37°C is continued. At specified sample collection times, 0.5 ml aliquots of the supernatant are withdrawn and replaced by an identical volume of PBS, pH 7.4, to maintain release conditions. The amount of released PS-liposomes is determined by measuring encapsulated FITC-BSA via absorbance at 495 nm in the supernatant after dissolving the lipid vesicles with 1% (v/v) Triton X-100 (Cohen et al., 1991)or by fluorescense detection in suitable detection systems.

Using the experimental conditions of Example 3, approx. 7% of the PS-liposomes are released from the hydrogel within the first 5 hours, approx. 15% after 24 hours and approx. 35% after 48 hours.

### EXAMPLE 4: RELEASE OF FIND-ME SIGNALS FROM HYDROGELS

This example describes the release of find-me signal ATP for peripheral phagocytosis of PS-liposomes from thermogelling PLGA-PEG-PLGA hydrogels.

### 4.1. Synthesis of thermogelling PLGA-PEG-PLGA hydrogels

The biodegradable triblock polymer described in this example has a PLG/PEG weight ratio of 2.3 (70/30), and a lactide/glycolide molar ratio of approx. 15/1. The synthesis is performed as described in Example 2.1.

### 4.2. In vitro release of ATP from PLGA-PEG-PLGA hydrogels

An aliquot of 20 µl of a 10 mM solution of ATP is combined with 160 µl of 25% gel solution of Example 4.1 and 20 µl of 10x PBS (final concentration of ATP is 1 mM). The mixture is incubated for 2 minutes at 37°C to induce gelling and overlayed with 1 ml of 1x PBS. At frequent timepoints the supernatant is removed by pipetting and stored at 4°C. The removed supernatant is replaced by fresh 1 ml of 1x PBS. After 48 hours the samples are measured spectrometrically at 260 nm and the amount of released ATPis calculated as percentage of a reference sample containing a concentration of ATP equaling 100% release.

Using the experimental conditions of Example 4, approx. 50% of the hydrogel-embedded ATP is released within the first 5 hours in an initial burst, followed by another 10% with the next 20 hours. After 48 hours approx. 75% of the hydrogel-embedded ATP is released.

### 4.3. Loading of hydrogels with therapeutically suitable concentrations of ATP and UTP

For the method of the present invention it is important to restrict the concentration of released ATP and/or UTP to the nanomolar range since extracellular nucleotides at concentrations of more than 1 µM are considered pro-inflammatory (Kono and Rock, 2008). Therefore, for therapeutic applications PLGA-PEG-PLGA hydrogels are loaded with ATP and/or UTP at a 1000-fold lower concentration of approx. 1 µM. Thereby, the concentration of released nucleotides will not exceed the critical limit of 1 µM, since within the first hour only approx. 20% of embedded nucleotides are released, followed by another 10% with the next hour and decreasing percentages during the following hours. Furthermore, triphosphate nucleotides released from the hydrogel into the extracellular space are rapidly degraded by extracellular enzymes to di- and mono-phosphate nucleotides.

### EXAMPLE 5: RELEASE OF IMMUNE MODULATORS FROM HYDROGEL

This example describes the release of hydrogel-embedded low molecular weight immune-modulators suitable for supporting the induction of tolerance by suppressing effector T cell responses and by enhancing the suppressive function of regulatory T cells, including calcitriol(1α,25-dihydroxyvitamin D3; 1,25-(OH)₂D3) and dexamethasone sodium phosphate (DexP).

### 5.1. Release of calcitriol from PLGA-PEG-PLGA hydrogels

This example describes the release of calcitriol from the hydrogel of Example 2.1.

### 5.1.1. Solubility of calcitriol

The solubility of calcitriol (MW 416.65) in ethanol is approx. 50 mg/ml, in a 1:5 solution of ethanol: PBS, pH 7.2, approx. 0.15 mg/ml.

### 5.1.2. Synthesis of hydrogel/calcitriol composits

The biodegradable PLGA-PEG-PLGA triblock polymer described in this example has a PLG/PEG weight ratio of 2.3 (70/30), and a lactide/glycolide molar ratio of approx. 15/1. Synthesis of the triblock copolymer is performed as described in Example 2.1. An aliquot of 20 µl of a stock solution of calcitriol (2 mg/ml; 4.8 mM) in absolute ethanol (Cayman/Biomol GmbH) is combined with 160 µl of 25% gel solution and 20 µl of 10x PBS (final concentration of calcitriol: 0.2 mg/ml or 0.48 mM; final concentration of ethanol: 10%).

### 5.1.3. Release assay.

The mixture of 5.1.1 is incubated for 2 minutes at 37°C to induce gelling and overlayed with 200 µl of 1x PBS. At frequent time points the supernatant is removed by pipetting and stored at 4°C. The removed supernatant is replaced by fresh 200 µl of 1x PBS. Controls of the same concentration of calcitriol in 1x PBS without gel are incubated and sampled in parallel.

### 5.1.4. Analysis of calcitriol release

After 48 hours the samples and controls are analysed using a Vitamin D ELISA kit, according to the manual of the manufacturer (Euroimmun AG, Luebeck). The assay is applicable for the determination of 25-OH vitamin D3 and other hydroxylated vitamin D3 molecules, and sufficiently linear from 4.0 to 120 ng/ml of 25-OH vitamin D3 (cross reactivity with calcitriol: 45%).

In brief, 20 µl of diluted samples are mixed with 230 µl of sample buffer containing a biotin-labed 25-OH vitamin D derivative. 200 µl of the mixture are transferred to an ELISA well of an 8-well strip and incubated at room temperature for 2 hours. The wells are washed with 3x 300 µl washing buffer. Peroxidase labelled steptavidin-conjugate (100 µl) is added and incubated for a further hour. The well is again washed 3x with 300 µl washing buffer. Tetramethyl-benzidine (100 µl) substrate solution is added and developed until sufficient coloring. The reaction is stopped by addition of 100 µl 0.5 M sulphuric acid and measured spectrometrically at 420 nm with reference 650 nm.

Using the experimental conditions of Example 5.1, approx. 15% of the hydrogel-embedded calcitriol is released within the first 2 hours, approx. 30% after 6 hours, approx. 45% after 12 hours, approx. 65% after 24 hours, and approx. 85% after 48 hours.

### 5.1.5. Discussion of the results

For the disclosed method, the concentration of hydrogel-embedded calcitriol is restricted to the low µM range. Toxicity occurs when serum levels of calcidiol rise to 500 ng/ml (comparable to 1.20 µM calcitriol; MW 416.6) or above (Shephard and DeLuca, 1980). Based on experiments in the rat, serum levels of calcitriol should be kept below 250 ng/ml to avoid toxicity (Shephard and DeLuca, 1980).However, doses needed for achieving the immune effects exceed this level. For example, Langerhans cells (LCs) and dermal dendritic cells (DDCs) primed under in vitro conditions, required the presence of 2.5 µM calcitriol (1.04 µg/ml) in order to effectively induce FoxP3(+)Tregs and FoxP3(-)IL-10(+)T_{R}1 cells, respectively (van der Aar et al., 2011). Although this concentration is approx. 4-fold higher than the recommended concentration, local administration of calcitriol may allow the application of a therapeutically effective concentration of calcitriolsince the release of calcitriol from the hydrogel occurs gradually over two days (30% within the first 6 hours and then significantly less) and released calcitriol is rapidly entering cells in the neighborhood of the hydrogel. Thereby, released calcitriol has only limited access to intestinal cells, which limits the danger of hypercalcemia. Furthermore, application of the calcitrol derivative calcipotriol for the method of the present invention further minimizes the risk of side effects due to the extremely short plasma half-life of calcipotriol and its low effects on calcium metabolism.

### 5.2. Release of DexP from PLGA-PEG-PLGA hydrogels

Example 5.2 describes the release of dexamethasone sodium phosphate (DexP) from the hydrogel of Example 2.1.

### 5.2.1. Synthesis of hydrogel/DexPcomposits

The biodegradable PLGA-PEG-PLGA triblock polymer used in this example has a PLG/PEG weight ratio of 2.3 (70/30), and a lactide/glycolide molar ratio of approx. 15/1. Synthesis of the triblock copolymer is performed as described in Example 2.1. A20 mM stock solution of DexP in PBS (10.3 mg/ml; MW 516.4; Sigma-Aldrich) is diluted 1:40 in PBS (final concentration: 0.5 mM), and 20 µl of the diluted stock solution is combined with 160 µl of 25% gel solution and 20 µl of 10x PBS (final concentration of DexP: 0.05 mM).

### 5.2.2. Release assay.

The mixture of 5.2.1 is incubated for 2 minutes at 37°C to induce gelling and overlayed with 200 µl of 1x PBS. At frequent time points the supernatant is removed by pipetting and stored at 4°C. The removed supernatant is replaced by fresh 200 µl of 1x PBS. Controls of the same concentration of DexP in 1x PBS without gel are incubated and sampled in parallel.

### 5.2.3. Analysis of DexP release

Released DexP is determined with a spectrophotometric method (Singh and Verma, 2008). The method involves oxidation of the corticosteroid by iron (III) and subsequent complexation of iron (II) with potassium hexacynoferate (III), forming a bluish green coloured complex with maximum absorbance at 780 nm (Beer's law range: 10-50 µg/ml; molar absorptivity (M⁻¹cm⁻¹): for DexP 0.55 x 10⁴).

Using the experimental conditions of Example 5.2, between30 and 50% of hydrogel-embedded DexP is released within the first 6 hours, followed by a continuously decreasing release per hour, until 80-90% of embedded DexP is released after 2 days.

### 5.2.4. Discussion of the results

In this example, an aliquot of 100 µl hydrogel/DexP composit contains 5 nmole DexP (2.58 µg DexP). Subcutaneous injection of this amount into mice generates a DexP concentration of 2.94 µM (2.94 nmole/ml; 1.7 ml blood volume/mouse) upon complete release of DexP from the injected hydrogel composit.

Therapeutically suitable concentrations of Dex for the generation of tolerogenic DCs and suppression of autoimmune diseases have been evaluated in various studies. For example, 50% inhibition of DC maturation under in vitro conditions has been observed at concentrations of 2-30 nM Dex and 100% inhibition at 500 nM Dex (Metasic et al., 1999). In another study, monocyte-derived tolerogenic DCs obtained from patients with primary Sjögren's syndrome have been generated under in vitro conditions at concentrations of 1 µM Dex and 0.1 nM calcitriol (Volchenkov et al., 2013).

A concentration of 1 µM Dex (392.4 ng/ml; MW of Dex: 292.4) under in vitro conditions corresponds to a concentration of approx. 670 ng Dex/mouse with a blood volume of 1.7 ml, which is approx. three-fold lower than that of Example 5.2generated in mice by injection of 100 µl hydrogel/DexP composit. However, the gradual release of hydrogel-embedded DexP (approx. 30-50% within the first 6 hours, followed by a continuously decreasing release per hour, until 80-90% of embedded DexP is released after 2 days) generates DexP levels which are comparable with those applied under in vitro conditions for complete inhibition of DC maturation.

Much higher concentrations of Dex have been applied in clinical trials. For example, in patients with severe idiopathic thrombocytopenic purpura, intravenous administration of 40 mg/dayDex (102µmole (MW 392.4); corresponding to approx. 35 µM in 2.7-3.0 liters of plasma) for 4 consecutive days (4-6 cycles every 28 days) has been demonstrated to induce a response rate of approx. 90% and was well tolerated (Mazzucconi et al., 2007). In patients with rheumatoid arthritis, even 5-fold higher amounts of Dex (200 mg/day; approx. 175 µM) have been administered intraveneouslyon three alternating days, leading to a rapid, clinically beneficial effect which was accompanied by upregulation of IL-10 (Verhoef et al., 1999).In the latter study, however, the plasma concentration of Dex around day 7 after i.v.administration was still in the range of 100-1000 nM and only after 6 weeks (at day 42) the concentration had leveled off to 1 nM.

In summary, tolerizing effects can be achieved apparently with relatively low local concentrations of Dex in the range of 0.5-1 µM. As demonstrated in a clinical trial, however, higher dosages of Dex(up to 35 µM; 13.7 pg) are not harmful, if the higher plasma concentrationsof Dex are not maintained for more than 4 days (Mazzucconi et al., 2007). Even 5-7 day treatments have shown few side effects and quick recovery (for a review, see Longui, 2007).

### EXAMPLE 6: IN VIVO UPTAKE OF PS-LIPOSOMES

Example 6 describes the analysis of in vivo trafficking and uptake of fluorescently labeled PS-liposomes in mice by macrophages and DCs after subcutaneous injection at the tail base of PLGA-PEG-PLGA hydrogel/PS-liposome composits containingnanomolar concentrations of ATP and UTP.

### 6.1. Marker molecules used for the analysis of subsets of DCs and macrophages

Several phenotypic and functional dendritic cell (DC) subsets have been characterized including conventional (classical; myeloid) DCs (cDCs), Langerhans cells, inflammatory/monocyte-derived DCs (moDCs), and plasmacytoid DCs (pDCs).

Mouse lymphoid-resident conventionalDCs (cDCs) are found in the thymus, spleen, lymph nodes, and Peyer's patches. Multiple cDC subsets have been identified in mouse including CD4⁻CD8α⁺ cDCs, CD4⁺CD8α⁻ cDCs, CD4⁻CD8α⁻ cDCs, Integrin alpha E (CD103⁺) cDCs, and Integrin alpha M (ITGAM; CD11b⁺) cDCs.CD11b (also known as CR3A) is one protein subunit that forms the heterodimericintegrin alpha-M beta-2 (α_{M}β₂) molecule, also known as macrophage-1 antigen (Mac-1) or complement receptor 3 (CR3).Integrin alpha-M beta-2 (α_{M}β₂) is expressed on the surface of many leukocytes involved in the innate immune system, including monocytes, granulocytes, macrophages, and natural killer cells. Mouse cDCs also express CD11c and B220. CD11c is an integrin alpha X chain protein, a type I transmembrane protein, found at high levels on most humandendritic cells, but also on monocytes, macrophages, and neutrophils. The mouse B220 antigen(CD45R) represents a subset of mouse CD45 isoforms that is predominantly expressed on all B lymphocytes, but also on DCs.Subsets of conventional migratory DCs express CD103, CD11b, CD11c, and Langerin (CD207).

In contrast to cDCs, moDCs develop from monocytes at sites of inflammation and are identified in mice by their expression of Ly-6C (lymphocyte antigen 6C). Ly-6Cis a glycoprotein which is expressed on macrophage/dendritic cell precursors in midstage.

Langerhans cells can be identified in both human and mouse by the presence of Langerin (CD207)-containing Birbeck granules. Langerin is a type II transmembrane, C-type lectin.

Plasmacytoid dendritic cells (pDCs) are a rare subset of dendritic cells. Unlike other DCs, pDCs are inefficient antigen-presenting cells and have low MHC class II expression. Mouse pDC express CD11c, B220, mPDCA-1 (mouse plasmacytoid dendritic cell antigen-1; also known as BST-2 (bone marrow stromal antigen 2) or CD317)), which is specifically expressed on mouse pDCs, and Gr-1 (granulocyte receptor 1).

Macrophages are a population of differentiated myeloid cells comprisinga) classically activated macrophages which secrete high levels of pro-inflammatory cytokines upon stimulation by the combination of IFN-γ, TNF, and LPS, b) wound-healing macrophages responding to IL-4, IL-13, and other signals released during injury, and c) a subset of activated macrophages representing regulatory macrophages.In this Example, F4/80 (also known as EMR1, a protein in the EGF-TM7 family of adhesion molecules), is used as marker molecule. It is primarily expressed on macrophages and dendritic cells in the mouse. Furthermore, CD11b is also used as marker for identification of macrophages.

### 6.2. Synthesis of thermogelling PLGA-PEG-PLGA hydrogels

The biodegradable triblock polymer described in this example has a PLG/PEG weight ratio of 2.3 (70/30), and a lactide/glycolide molar ratio of approx. 15/1. Synthesis of the triblock copolymer is performed as described in Example 2.1.

### 6.3.Synthesis of PS-liposomes

A chloroform/methanol (2:1, v/v) solution containing 30 µmole PS (approx. 22.7 mg), 30 µmole PC (approx. 22.0 mg) and 40 µmole CH (approx. 15.5 mg) is placed in a conical flask and dried by rotary evaporation to prepare a thin lipd film. Thereafter, the flask is placed in a desiccator for at least one hour to completely remove the solvent. Then, 1.5 ml of PBS is added and multilamellar vesicles are generated by intense vortex dispersion.For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.5 ml of PBS and vortexed to resuspend the liposomes. The final liposomal suspension contains approx. 66.7 µmole (40 mg) of lipd/ml. For fluorescent labeling the liposome suspension is diluted in PBS to a concentration of approx. 6.7 mg lipid/ml (dilution 1:6).

### 6.4. Post-formation fluorescent labeling of PS-liposomes.

Liposomes are labelled with Dil because of its strong fluorescence, low toxicity, and because it integrates with high stability into the liposome and remains fixed there even when in contact with other membranes (Claassen, 1992).

The PS-liposomes of Example 6.3 (6.7 mg lipid/ml) are labeled by adding 100 µl of stock solutions in ethanol (2.5 mg/ml of the lipophilic carbocyanine dye Dil (1,1'-dioctadecyl-3,3,3',3'-tetramethylindocarbocyanine perchlorate) (InvitrogenMolecular Probes, USA) to 1.90 ml of the liposome suspension containing 12.7 mg lipid (final concentrations: ethanol 5%; 6.35 mg lipid/ml; 125 µg dye/ml).

The ratio of dye/lipidin Example 6.4 is approx. 50 pg/2.5 mg according to the method of Claassen (1992).Using these conditions, the liposomal uptake of the extremely lipophilic dye is established within a few minutes and practically complete as indicated by an almost colourless supernatant upon centrifugation of the labeled liposomal suspension (Claassen, 1992).

### 6.5. Synthesis of hydrogel composits containing labeled PS-liposomes, ATP and UTP

An aliquotof 200 µl water containing the copolymer of Example 6.1 at a concentration of 22.5% (w/w), is cooled to 4°C and mixed with 100 µl of the labeled PS-liposome suspension of Example 6.4(0.635 mg lipid; 12.5 µg dye), 5 µl of 60 µM ATP in PBS (0.30 nmole), and 5µl of 60 µM UTP in PBS (0.30 nmole). The final concentrations of the copolymer is 15% (w/w) containing labeled PS-liposomes at a concentration of 2.05 mg lipid/ml (0.63 mg/310 µl), ATP at a concentration of 0.97 µM (0.3 nmol/310 µl), and UTPat a concentration of 0.97µM (0.3 nmol/310 µl).

According to Example 4, approx. 50% of the hydrogel-embedded nucleotides are released within the first 5 hours in an initial burst, followed by another 10% with the next 20 hours. Due to these release kinetics, the local concentration of released nucleotides stays below the critical concentration of 1 µM (compare Example 4.3 for the critical concentrations of ATP and UTP).

### 6.6. In vivo fate of hydrogel-emedded PS-liposomes

C57BL/6 mice are purchased from Harlan (Borchen, Germany) and used at an age of 8-12 weeks.

An aliquot of 100 µl of the hydrogel/PS-liposome composit of Example 6.5 containing labeled PS-liposomes (0.21mg lipid), ATP (0.1 nmole) and UTP (0.1 nmole),is injected subcutaneously at the tail base. After 48 hours (approx. 35% of the labeled PS-liposomes are released from the hydrogel during 48 hours), spleen, inguinal, mesenteric, and axillary lymph nodes (LNs) are removed and pressed through a cell strainer.

In a control experiment, 100 µl of labeled PS-liposomes from Example 6.4 (0.635 mg lipid/100 µl; 12.5 µg dye/100 µl) and non-labeled PS-liposomes from Example 6.3 (diluted 1.6 to 0.67 mg lipid/100 µl) are injected subcutaneously at the tail base and mice are analyzed as described for the hydrogel/PS-liposome composit.

### 6.7. Analysis

Cell suspensions of Example 6.6 are stained with different fluorescent labeled antibodies according to the manufacturer's instructions including anti-mouse MHC class II-FITC (eBioscience), anti-mouse CD8α-FITC (eBioscience), anti-mouse CD4-allophycocyanin (BioLegend), anti-mouse CD11b-FITC (eBioscience), anti-mouse CD11c-allophycocyanin(BD Pharmingen),anti-mouse CD45R(B220)-FITC (eBioscience), anti-mouse CD207-allophycocyanin (BioLegend), anti-mouse PDCA-1(BST-2; CD317)-allophycocyanin (eBioscience), and anti-mouse F4/80-allophycocyanin (eBioscience). After staining, cells are washed and subjected to flow cytometry. Dil is an orange-red-fluorescent dye, which is spectrally similar to tetramethylrhodamine. Therefore, for the analysis of phagocytosed PS-liposomes, red fluorescent cells are gated.

Several sets of stained cells are analyzed. Antigen presenting cells (APCs) are analyzed after staining with anti-mouse MHC class II-FITC, macrophages and DCs after double staining with anti-mouse CD11b-FITC and anti-mouse F4/80-allophycocyanin, cDCs and pCDs after double staining with anti-mouse CD45R(B220)-FITC and anti-mouse CD11c-allophycocyanin, cDCs after double staining with anti-mouse CD8α-FITC and anti-mouse CD4-allophycocyanin, pDCs after double staining with anti-mouse MHC class II-FITC and anti-mouse PDCA-1(BST-2; CD317)-allophycocyanin, and LCs after staining with anti-mouse CD207-allophycocyanin.

As evident from previous experiments with Dil-labeled PC-liposomes (Capini et al., 2009), subcutaneous injection of Dil-labeled liposomes at the tail base of C57BL/6 mice leads to an almost exclusive uptake of the liposomes by MHC class II⁺ APCs in the inguinal LNs, whereas liposome-containing APCs in non-draining axillary LNs are rare. Among the liposome-containing APCsin the inguinal LNs are F4/80⁺ and CD11b⁺ macrophages and DCs, and CD11c⁺ DCs including a) B220^{high}CD11c^{low} and B220^{low}CD11c^{high}DC subsets, b) PDCA-1⁺ and PDCA-1⁻DC subsets, and c) CD8⁺ and CD8⁻ DC subsets. Thus, the majority of Dil-labeled liposomes is phagocytosed by macrophages and conventional cDCs in the draining lymph nodes.

### EXAMPLE 7: IMMUNOTHERAPY OF ANTIGEN-INDUCED ARTHRITIS IN C57BL/6 MICE WITH TOLERIZING PS-LIPOSOMES

This example investigates the efficacy of immunotherapy of antigen-induced arthritis in C57BL/6 mice induced by intradermal injection of mBSA emulsified in complete Freund's adjuvant (CFA) and intraperitoneal injection of pertussis toxin, with hydrogel-embedded tolerizing PS-liposomes loaded with DC maturation inhibitors (calcipotriol and dexamethasone phosphate (DexP)) and mBSA or ovalbumin (OVA). PS-liposomes containing encapsulated mBSA or OVA are used for control. In this example, OVA is used as a non-related control antigen

### 7.1. Animals

For this example, female wild-type C57BL/6 mice 6 to 10 weeks of age are purchased from Charles River (Sulzfeld, Germany). The animals are kept under specific pathogen-free conditions.

### 7.2.Preparation of hydrogel composits containing tolerizing PS-liposomes or control PS-liposomes and ATP/UTP mixtures

This example describes the synthesis of hydrogel composits containing ATP/UTP as find-me signals and tolerizing PS-liposomes with encapsulated mBSA or OVA, or control PS-liposomes with encapsulated mBSA or OVA.

### 7.2.1. Synthesis of PS-liposomes containing encapsulated mBSA or OVA

Synthesis of PS-liposomes containingencapsulated mBSA or OVA is performed as described in Example 1.2.3. The final unilamellar PS-liposomes with a particle size of approx. 1 µm contain approx. 59 µmole (approx. 35 mg) of lipd/ml (59 mM liposomal suspension), and approx. 2-2.5 mg mBSA or OVA/ml (based on 20-25% encapsulation efficiency).

### 7.2.2. Synthesis ofcalcipotriol-loaded PS-liposomes with encapsulated mBSA or OVA

Synthesis of calcipotriol-loaded PS-liposomes containing mBSA or OVA is performed as described in Example 1.4.1. The final liposomal suspension contains approx. 59 µmole (approx. 35 mg) of lipd/ml (59 mM liposomal suspension), approx. 310 µg (751 nmol) calcipotriol/ml liposomal suspension (based on 85% incorporation rate), and approx. 2-2.5 mg mBSA or OVA/ml (based on 20-25% encapsulation efficiency).

### 7.2.3. Synthesis ofcalcipotriol-loaded PS-liposomes with encapsulatedDexP and mBSA or DexP and OVA

Synthesis of calcipotriol-loaded PS-liposomes containingdexamethasone phosphate (DexP) and mBSAor DexP and OVA is performed as described in Example 1.8.1.The final unilamellar PS-liposomes with a particle size of approx. 1 µm contain approx. 59 µmole (approx. 35 mg) of lipd/ml (59 mM liposomal suspension), approx. 310 µg (751 nmol) calcipotriol/ml liposomal suspension (based on 85% incorporation rate), approx. 1.2 mg liposomal DexP/ml (based on 10% encapsulation efficiency), corresponding to 20 µgDexP/µmole lipid, and approx. 2-2.5 mg mBSA or OVA/ml (based on 20-25% encapsulation efficiency).

### 7.2.4. Synthesis of hydrogel composits

The synthesis of thermogelling PLGA-PEG-PLGA hydrogels containing either PS-liposomes with encapsulated mBSA or OVA, or calcipotriol-loaded PS-liposomes with encapsulated mBSA or OVA, or calcipotriol-loaded PS-liposomes with encapsulated DexP and mBSA or DexP and OVA, is performed as described in Example 2.

The biodegradable triblock polymer used for this example has a PLG/PEG weight ratio of 2.3 (70/30), and a lactide/glycolide molar ratio of approx. 15/1. A 30% (w/w) solution of the PLGA-PEG-PLGA copolymer in water is mixed with the liposomal suspensions in PBS of Examples 7.2.1, 7.2.2 and 7.2.3at a ratio of two volumes hydrogel solution to one volume of liposomal suspension.ATP and UTP are added to the hydrogel composit to a final concentration of approx. 1 µM by the addition of 5 µl of 0.2 mM ATP (1 nmole) and 5 µl of 0.2 mM UTP(1 nmole) per ml of hydrogel composit.

The final concentration of the hydrogel is 20% (w/w) containing liposomes at a concentration of approx. 20 µmole (12 mg) of lipd/ml, various combinations of liposomal immune modulators including approx. 100 µg (242 nmole; MW 412.6) calcipotriol/ml, approx. 400 µg liposomal DexP (775 nmole; MW 516.4)/ml, and approx. 0.7-0.8 mg mBSA or OVA/ml, and approx. 1 nmole ATP/ml and approx. 1 nmole UTP/ml as find-me signals.

For control experiments with PS-liposomes which are non-embedded in a hydrogel, the liposomal suspensionsin PBS of Examples 7.2.1, 7.2.2 and 7.2.3arethree-fold diluted in PBS.

### 7.3. Induction of arthritis with mBSA and pertussis toxin

Three weeks before arthritis induction, C57BL/6 mice are immunized in each axilla by intradermal injection of 100 µg mBSA emulsified in complete Freund's adjuvant (CFA). Simultaneously, 400 ng of pertussis toxin are injected intraperitoneally (i.p.). Seven days later, a booster dose of 100 µg mBSA emulsified in CFA is injected intracutaneously (s.c.). Arthritis is induced by intra-articular injection of 60 µg mBSA into the right knee joint cavity (day 0), while the control left knee joint is treated with saline.Arthritis induced in this manner is chronic, antigen-specific, and T cell-dependent (Brackertz et al., 1977).

### 7.4. Immunotherapy with tolerizing PS-liposomes

In this example, the therapeutic efficacy of different liposomal formulations for the treatment of established arthritis is evaluated. Three sets of experiments are performed, a) comparison of hydrogel/liposome composits with non-embedded PS-liposomes, b) evaluation of liposomal calcipotriol as adjuvant, and c) evaluation of a combination of liposomal calcipotrioland DexP as adjuvant.

### 7.4.1. Comparison of hydrogel/liposome composits with non-embedded PS-liposomes

In this example, PS-liposomes containing encapsulated mBSA or OVA (see Example 7.2.1) are administered as hydrogel/liposome composit or as liposomal suspension without hydrogel.

### 7.4.2. Evaluation of liposomal calcipotriol as adjuvant

In this example, calcipotriol-loaded PS-liposomes containing encapsulated mBSA or OVA (see Example 7.2.2) are administered as hydrogel/liposome composit. Hydrogel-embedded PS-liposomes containing encapsulated mBSA (see Example 7.2.1.) are used for control.

### 7.4.3. Evaluation of a combination of liposomal calcipotriol and DexP as adjuvant

In this example, calcipotriol-loaded PS-liposomes containing encapsulated mBSA and DexP or OVA and DexP (see Example 7.2.3) are administered as hydrogel/liposome composit.Hydrogel-embedded PS-liposomes containing encapsulated mBSA (see Example 7.2.1.) and hydrogel-embedded calcipotriol-loaded PS-liposomes containing encapsulated mBSA (see Example 7.2.2.) are used for control.

### 7.4.4. Immunotherapy

On day 7, each group of mice (n = 10) is injected s.c. 100 µl of liposomal formulations at the tail base.Aliquots of 100 µl hydrogel/liposome composit or liposomal suspension without hydrogel contain:
- approx. 2 µmole (1.2 mg) of lipd
   (PS-liposomes according to 7.2.1, 7.2.2 and 7.2.3)
- approx. 0.07-0.08 mg mBSA or OVA
   (PS-liposomes according to 7.2.1, 7.2.2 and 7.2.3)
- approx. 10 µg (24.2 nmole) calcipotriol
   (PS-liposomes according to 7.2.2 and 7.2.3)
- approx. 40 µg (775 nmole)liposomal DexP
   (PS-liposomes according to 7.2.3)

Aliquots of 100 µl hydrogel/liposome composit contain in addition approx. 0.1 nmole ATP and 0.1 nmole UTP as find-me signals.

### 7.5. Evaluation of clinical signs of arthritis

From the day of arthritis induction, knee joint swelling is measured in each mouse every 3-4 days for up to two weeks with a Vernier caliper and then expressed as a percentage based on the difference between the diameters of the right and the left knee joint, where the maximum difference between two knees in each mouse is equal to 100%.

At day 14, mice are sacrificed and the knee skin is removed. Severity of knee joint swelling is compared between knees injected with antigen and saline and expressed as a clinical score rated from 1 to 5, where all scoring is performed without knowledge of the treatment group. 1 = no difference between saline and antigen knee; 2 = slight discoloration of joint; 3 = discoloration of joint and mild lateral swelling and discoloration; 4 = discoloration of joint and moderate lateral swelling and discoloration; 5 = severe discoloration of joint to the point where the ligament is no longer visible and severe lateral swelling and discoloration.

### 7.6 Discussion of results

In accordance with a previous immunotherapy study using liposomes loaded with mBSA and lipophilic NF-κB inhibitors in C57BL/6 mice with mBSA-induced arthritis (Capini et al., 2009), even a single s.c. injection of tolerizing liposomes (including those embedded in a hydrogel) with encapsulated mBSA reduces the clinical score of arthritis significantly, whereas liposomal formulations containing the control antigen OVA have no significant suppressive effect.

Treatment of mBSA-induced arthritis with PS-liposomes containing calcitriol, DexP and mBSA favors the development of tolerogenic APCs and, thereby, the induction of antigen-specific Tregs. This is in accordance with the observation that after injection of liposomes loaded with mBSA and lipophilic NF-κB inhibitors the development of antigen-specific FoxP3⁺ Tregs can be observed (Capini et al., 2009).As evident from this study, antigen-specific FoxP3⁺ Tregs have the ability to induce antigen-specific tolerance and to suppress full-blown antigen-induced arthritis in an antigen-specific manner.

### EXAMPLE 8: IMMUNOTHERAPY OF MOG (35-55) -INDUCED EAE IN C57BL/6 MICE WITH TOLERIZING PS-LIPOSOMES

This example investigates the efficacy of immunotherapy of experimental autoimmune encephalomyelitis (EAE) in C57BL/6 mice induced by subcutaneous (s.c.) injection of MOG(35-55) emulsified in complete Freund's adjuvant (CFA) and intraperitoneal injection of pertussis toxin, with hydrogel-embedded tolerizing PS-liposomes loaded with DC maturation inhibitors (calcitriol and dexamethasone phosphate (DexP)) and MOG(35-55). PS-liposomes containing encapsulated MOG(35-55) or the non-related antigen OVA are used for control.

### 8.1. Animals

Female wild-type C57BL/6 mice 6 to 10 weeks of age are purchased from Charles River (Sulzfeld, Germany). The animals are kept under specific pathogen-free conditions.

### 8.2. Preparation of hydrogel composits containing tolerizing PS-liposomes or control PS-liposomes and ATP/UTP mixtures

This example describes the synthesis of hydrogel composits containing ATP/UTP as find me signals and tolerizing PS-liposomes with encapsulated MOG(35-55) or control PS-liposomes with encapsulated MOG(35-55) or OVA. In this example, OVA is used as a non-related control antigen.

### 8.2.1. Synthesis of PS-liposomes containing encapsulated MOG(35-55) or OVA

Synthesis of PS-liposomes containingencapsulated MOG(35-55) or OVA is performed as described in Example 1.2.3. The final unilamellar PS-liposomes with a particle size of approx. 1 µm contain approx. 59 µmole (approx. 35 mg) of lipd/ml (59 mM liposomal suspension), approx. 0.6-1.0 mg MOG(35-55)/ml (based on 30-50% encapsulation efficiency),and approx. 2-2.5 mg OVA/ml (based on 20-25% encapsulation efficiency).

### 8.2.2. Synthesis ofcalcipotriol-loaded PS-liposomes with encapsulatedMOG(35-55) or OVA

Synthesis of calcipotriol-loaded PS-liposomes containingMOG(35-55) or OVA is performed as described in Example 1.4.1. The final liposomal suspension contains approx. 59 µmole (approx. 35 mg) of lipd/ml (59 mM liposomal suspension), approx. 310 µg (751 nmol) calcipotriol/ml liposomal suspension (based on 85% incorporation rate), approx. 0.6-1.0 mg MOG(35-55)/ml (based on 30-50% encapsulation efficiency),and approx. 2-2.5 mg OVA/ml (based on 20-25% encapsulation efficiency).

### 8.2.3. Synthesis ofcalcipotriol-loaded PS-liposomes with encapsulatedDexP and MOG(35-55) or DexP and OVA

Synthesis of calcipotriol-loaded PS-liposomes containingdexamethasone phosphate (DexP) and MOG(35-55) or DexP and OVA is performed as described in Example 1.8.1. The final unilamellar PS-liposomes with a particle size of approx. 1 µm contain approx. 59 µmole (approx. 35 mg) of lipd/ml (59 mM liposomal suspension), approx. 310 µg (751 nmol) calcipotriol/ml liposomal suspension (based on 85% incorporation rate), approx. 1.2 mg liposomal DexP/ml (based on 10% encapsulation efficiency), corresponding to 20 µgDexP/µmole lipid, approx. 0.6-1.0 mg MOG(35-55)/ml (based on 30-50% encapsulation efficiency),and approx. 2-2.5 mg OVA/ml (based on 20-25% encapsulation efficiency)).

### 8.2.4. Synthesis of hydrogel composits

The synthesis of thermogelling PLGA-PEG-PLGA hydrogels containing either PS-liposomes with encapsulated MOG(35-55) or OVA, or calcipotriol-loaded PS-liposomes with encapsulated MOG(35-55) or OVA, or calcipotriol-loaded PS-liposomes with encapsulated DexP and MOG(35-55) or DexP and OVA, is performed as described in Example 2.

The biodegradable triblock polymer used for this example has a PLG/PEG weight ratio of 2.3 (70/30), and a lactide/glycolide molar ratio of approx. 15/1. A 30% (w/w) solution of the PLGA-PEG-PLGA copolymer in water is mixed with the liposomal suspensions in PBS of Examples 8.2.1, 8.2.2 and 8.2.3 at a ratio of two volumes hydrogel solution to one volume of liposomal suspension.ATP and UTP are added to the hydrogel composit to a final concentration of approx. 1 µM by the addition of 5 µl of 0.2 mM ATP (1 nmole) and 5 µl of 0.2 mM UTP(1 nmole) per ml of hydrogel composit.

The final concentration of the hydrogel is 20% (w/w) containing liposomes at a concentration of approx. 20 µmole (12 mg) of lipd/ml, various combinations of liposomal immune modulators including approx. 100 µg (242 nmole; MW 412.6) calcipotriol/ml, approx. 400 µg liposomal DexP (775 nmole; MW 516.4)/ml, approx. 0.2-0.3 mg liposomal MOG(35-55)/ml, approx. 0.7-0.8 mg liposomal OVA/ml, and approx. 1 nmole ATP/ml and approx. 1 nmole UTP/ml as find-me signals.

For control experiments with PS-liposomes which are non-embedded in a hydrogel, the liposomal suspensions in PBS of Examples 8.2.1, 8.2.2 and 8.2.3 are three-fold diluted in PBS.

### 8.3. Induction of EAE with murine MOG(35-55) and pertussis toxin

C57BL/6 mice are immunized by subcutaneous injection into the flanks of 50 µg murine MOG(35-55) emulsified in an equal volume of complete Freund's adjuvant (CFA) containing *Mycobacterium tuberculosis* H37RA (Difco) at a final concentration of 1 mg/ml as described (Wüst et al., 2008). Immediately after immunization and 2 days after immunization pertussis toxin (List Biological Laboratories) is injected intraperitoneally (400 ng/mouse in total).

Animals are weighed and scored daily for clinical signs of disease on a scale from 0 to 10 depending on severity. 0 = normal; 1 = reduced tone of tail; 2 = limp tail, impaired righting; 3 = absent righting; 4 = gait ataxia; 5 = mild paraparesis of hind limbs; 6 = moderate paraparesis; 7 = severe paraparesis or paraplegia; 8 = tetraparesis; 9 = moribund; 10 = death (Wüst et al., 2008).

### 8.4. Immunotherapy with tolerizing PS-liposomes

In this example, the therapeutic efficacy of different liposomal formulations for the treatment of established EAE is evaluated.Three sets of experiments are performed, a) comparison of hydrogel/liposome composits with non-embedded PS-liposomes, b) evaluation of liposomal calcipotriol as adjuvant, and c) evaluation of a combination of liposomal calcipotrioland DexP as adjuvant.

### 8.4.1. Comparison of hydrogel/liposome composits with non-embedded PS-liposomes

In this example, PS-liposomes containing encapsulated MOG(35-55) or OVA (see Example 8.2.1) are administered as hydrogel/liposome composit or as liposomal suspension without hydrogel.

### 8.4.2. Evaluation of liposomal calcipotriol as adjuvant

In this example, calcipotriol-loaded PS-liposomes containing encapsulated MOG(35-55) or OVA (see Example 8.2.2) are administered as hydrogel/liposome composit. Hydrogel-embedded PS-liposomes containing encapsulated MOG(35-55) (see Example 8.2.1) are used for control.

### 8.4.3.Evaluation of a combination of liposomal calcipotriol and DexP as adjuvant

In this example, calcipotriol-loaded PS-liposomes containing encapsulated MOG(35-55) and DexP or OVA and DexP (see Example 8.2.3) are administered as hydrogel/liposome composit. Hydrogel-embedded PS-liposomes containing encapsulated MOG(35-55)(see Example 8.2.1.) and hydrogel-embedded calcipotriol-loaded PS-liposomes containing encapsulated MOG(35-55)(see Example 8.2.2.) are used for control.

### 8.4.4. Immunotherapy

Immunotherapy is started once the mice have reached an average clinical score of 2-3 (approx. 6 days after immunization). Each group of mice (n = 10) is injected s.c. 100 µl of liposomal formulations at the tail base.Aliquots of 100 µl hydrogel/liposome composit or liposomal suspension without hydrogel contain:
- approx. 2 µmole (1.2 mg) of lipd
   (PS-liposomes according to 8.2.1, 8.2.2 and 8.2.3)
- approx. 0.02-0.03 mg liposomal MOG(35-55)
   (PS-liposomes according to 8.2.1, 8.2.2 and 8.2.3)
- approx. 0.07-0.08 mg OVA
   (PS-liposomes according to 8.2.1, 8.2.2 and 8.2.3)
- approx. 10 µg (24.2 nmole) calcipotriol
   (PS-liposomes according to 8.2.2 and 8.2.3)
- approx. 40 µg (775 nmole)liposomal DexP
   (PS-liposomes according to 8.2.3)

Aliquots of 100 µl hydrogel/liposome composit contain in addition approx. 0.1 nmole ATP and 0.1 nmole UTP as find-me signals.

### 8.5 Discussion of results

In accordance with a previous immunotherapy study using liposomes loaded with glucocorticoids in C57BL/6 mice with MOG(35-55)-induced EAE (Schweingruber et al., 2011), even a single injection of glucocorticoid-loaded liposomes (including those embedded in a hydrogel) with encapsulated MOG(35-55) reduces the clinical score of EAE significantly, whereas liposomal formulations containing the control antigen OVA have no significant effect.

Treatment of MOG(35-55)-induced EAE with calcipotriol-loaded PS-liposomes containing DexP and MOG(35-55) favors the development of tolerogenic APCs. This is in accordance with the observation that after injection of prednisolone-loaded liposomesmacrophages are converted to the M2 phenotype which exert anti-inflammatory activity (Schweingruber et al., 2011).

The functions of effector T cells are only marginally affected by this treatment. This is also in accordance with the observation of Schweingruber et al. (2011) that liposome-encapsulated glucocorticoids target primarily APCs, whereas free glucocorticoids mainly target T lymphocytes.

### EXAMPLE 9: IMMUNOTHERAPY OF OVA-INDUCED ALLERGIC AIRWAY INFLAMMATION IN BALB/c MICE WITH TOLERIZING PS-LIPOSOMES

This example investigates the efficacy of immunotherapy of OVA-induced allergig airway inflammation in BALB/c mice with hydrogel-embedded tolerizing PS-liposomes loaded with DC maturation inhibitors (calcipotriol and dexamethasone phosphate (DexP)) and OVA. PS-liposomes containing encapsulated OVA or the non-related antigen mBSA are used for control.

### 9.1. Animals

For this example, female wild-type C57BL/6 mice 6 to 10 weeks of age are purchased from Charles River (Sulzfeld, Germany). The animals are kept under specific pathogen-free conditions and maintained on OVA-free diets.

### 9.2. Preparation of hydrogel composits containing tolerizing PS-liposomes or control PS-liposomes and ATP/UTP mixtures

This example describes the synthesis of hydrogel composits containing ATP/UTP as find me signals and tolerizing PS-liposomes with encapsulated OVA or control PS-liposomes with encapsulated OVA or mBSA. In this example, mBSA is used as a non-related control antigen.

### 9.2.1. Synthesis of PS-liposomes containing encapsulated OVA or mBSA

Synthesis of PS-liposomes containingencapsulated OVA or mBSA is performed as described in Example 1.2.3. The final unilamellar PS-liposomes with a particle size of approx. 1 µm contain approx. 59 µmole (approx. 35 mg) of lipd/ml (59 mM liposomal suspension), and approx. 2-2.5 mg OVA or mBSA/ml (based on 20-25% encapsulation efficiency).

### 9.2.2. Synthesis ofcalcipotriol-loaded PS-liposomes with encapsulatedOVA or mBSA

Synthesis of calcipotriol-loaded PS-liposomes containingOVA or mBSA s performed as described in Example 1.4.1. The final liposomal suspension contains approx. 59 µmole (approx. 35 mg) of lipd/ml (59 mM liposomal suspension), approx. 310 µg (751 nmol) calcipotriol/ml liposomal suspension (based on 85% incorporation rate), and approx. 2-2.5 mg OVA or mBSA/ml (based on 20-25% encapsulation efficiency).

### 9.2.3. Synthesis ofcalcipotriol-loaded PS-liposomes with encapsulatedDexP and OVA or DexP and mBSA

Synthesis of calcipotriol-loaded PS-liposomes containingdexamethasone phosphate (DexP) and OVA or DexP and mBSA is performed as described in Example 1.8.1. The final unilamellar PS-liposomes with a particle size of approx. 1 µm contain approx. 59 µmole (approx. 35 mg) of lipd/ml (59 mM liposomal suspension), approx. 310 µg (751 nmol) calcipotriol/ml liposomal suspension (based on 85% incorporation rate), approx. 1.2 mg liposomal DexP/ml (based on 10% encapsulation efficiency), corresponding to 20 µgDexP/µmole lipid, and approx. 2-2.5 mg OVA or mBSA/ml (based on 20-25% encapsulation efficiency).

### 9.2.4. Synthesis of hydrogel composits

The synthesis of thermogelling PLGA-PEG-PLGA hydrogels containing either PS-liposomes with encapsulated OVA or mBSA, or calcipotriol-loaded PS-liposomes with encapsulated OVA or mBSA, or calcipotriol-loaded PS-liposomes with encapsulated DexP and OVA or DexP and mBSA, is performed as described in Example 2.

The biodegradable triblock polymer used for this example has a PLG/PEG weight ratio of 2.3 (70/30), and a lactide/glycolide molar ratio of approx. 15/1. A 30% (w/w) solution of the PLGA-PEG-PLGA copolymer in water is mixed with the liposomal suspensions in PBS of Examples 9.2.1, 9.2.2 and 9.2.3 at a ratio of two volumes hydrogel solution to one volume of liposomal suspension.ATP and UTP are added to the hydrogel composit to a final concentration of approx. 1 µM by the addition of 5 µl of 0.2 mM ATP (1 nmole) and 5 µl of 0.2 mM UTP(1 nmole) per ml of hydrogel composit.

The final concentration of the hydrogel is 20% (w/w) containing liposomes at a concentration of approx. 20 µmole (12 mg) of lipd/ml, various combinations of liposomal immune modulators including approx. 100 µg (242 nmole; MW 412.6) calcipotriol/ml, approx. 400 µg liposomal DexP (775 nmole; MW 516.4)/ml, approx. 0.7-0.8 mg OVA or mBSA/ml, and approx. 1 nmole ATP/ml and approx. 1 nmole UTP/ml as find-me signals.

For control experiments with PS-liposomes which are non-embedded in a hydrogel, the liposomal suspensions in PBS of Examples 9.2.1, 9.2.2 and 9.2.3 are three-fold diluted in PBS.

### 9.3. Induction of allergig airway inflammation

First,100 µg of OVA are solved in 0.3 ml of PBS, pH 7.4, and mixed with 0.70 ml Imject Alum (Pierce/KMF).Mice are immunized three times by i.p. injection of 100 µl alum-adsorbed OVA (10 µg OVA in 100 µl of PBS/Imject Alum). The second i.p.injection is performed 7 days after the first injection and the third injection 14 days after the first injection.

One week after the last injection the mice are exposed in a Plexiglas chamber (approx. 10 x 15 x 25 cm) to 1% (w/v) aerosolized OVA in 0.9% saline (using a nebulizer with an airflow rate of 10 L/min), 30 min/day for 10 days.

### 9.4. Immunotherapy with tolerizing PS-liposomes

In this example, the therapeutic efficacy of different liposomal formulations for the treatment of established allergic airway inflammation is evaluated.

Three sets of experiments are performed, a) comparison of hydrogel/liposome composits with non-embedded PS-liposomes, b) evaluation of liposomal calcipotriol as adjuvant, and c) evaluation of a combination of liposomal calcipotrioland DexP as adjuvant.

### 9.4.1. Comparison of hydrogel/liposome composits with non-embedded PS-liposomes

In this example, PS-liposomes containing encapsulated OVA or mBSA (see Example 9.2.1) are administered as hydrogel/liposome composit or as liposomal suspension without hydrogel.

### 9.4.2. Evaluation of liposomal calcipotriol as adjuvant

In this example, calcipotriol-loaded PS-liposomes containing encapsulated OVA or mBSA (see Example 9.2.2) are administered as hydrogel/liposome composit. Hydrogel-embedded PS-liposomes containing encapsulated OVA(see Example 9.2.1.) are used for control.

### 9.4.3. Evaluation of a combination of liposomal calcipotriol and DexP as adjuvant

In this example, calcipotriol-loaded PS-liposomes containing encapsulated OVA and DexP or mBSA and DexP (see Example 9.2.3) are administered as hydrogel/liposome composit.Hydrogel-embedded PS-liposomes containing encapsulated OVA(see Example 9.2.1.) and hydrogel-embedded calcipotriol-loaded PS-liposomes containing encapsulated OVA(see Example 9.2.2.) are used for control.

### 9.4.4. Immunotherapy

Immunotherapy is started one week after the final aerosol exposure. Each group of mice (n = 10) is injected s.c. 100 µl of the liposomal formulations at the tail base. After 14 days, a second s.c. injection of 100 µl of the liposomal formulations is performed.Aliquots of 100 µl hydrogel/liposome composit or liposomal suspension without hydrogel contain:
- approx. 2 µmole (1.2 mg) of lipd
   (PS-liposomes according to 9.2.1, 9.2.2 and 9.2.3)
- approx. 0.07-0.08 mg OVA or mBSA
   (PS-liposomes according to 9.2.1, 9.2.2 and 9.2.3)
- approx. 10 µg (24.2 nmole) calcipotriol
   (PS-liposomes according to 9.2.2 and 9.2.3)
- approx. 40 µg (775 nmole)liposomal DexP
   (PS-liposomes according to 9.2.3)

Aliquots of 100 µl hydrogel/liposome composit contain in addition approx. 0.1 nmole ATP and 0.1 nmole UTP as find-me signals.

### 9.4.5. Challenge procedure

One week after completed immunotherapeutic treatment, mice are challenged in a Plexiglas chamber (approx. 10 x 15 x 25 cm) with 1% (w/v) aerosolized OVA in 0.9% saline (using a nebulizer with an airflow rate of 10 L/min), 30 min/day for 3 consecutive days.

### 9.5. Measurement of AHR: Lung resistance

Measurement of airway hyperreactivity (AHR), defined as bronchoconstriction in response to methacholine, is performed in two animals of each group. Three days after the final aerosol exposure, the mice are sacrificed by exsanguination after i.p. injection of a ketamine/xylazine overdose, and analyses of bronchoalveolar lavage (BAL), lung tissue, and blood samples are performed.

### 9.6. Measurements of pulmonary resistance

Measurements of pulmonary resistance (R_{L}) are performed in anesthetized, mechanically ventilated mice (Yiamouyiannis et al., 1999). After anesthetizing the animals with pentobarbital (75 mg/kg i.p. injection), the abdominal inferior vena cava is cannulated, and a tracheostomy catheter is placed. The chest is opened by a small anterior incision, and the animal is placed in a whole-body plethysmograph. Mechanical ventilation is established with a small rodent respirator delivering a 10 ml/kg tidal volume at 140 breaths/minute, with a positive end-expiratory pressure (PEEP) of 3 cm H₂O. Values for R_{L} are calculated by analysis of electrical signals proportional to lung volume, airflow, and transpulmonary pressure. Changes in lung volume are determined from the measured changes in plethysmographic pressure and are differentiated over time to obtain flow measurements. Transpulmonary pressure is obtained from the difference between measured pressures at the airway opening and within the plethysmograph. After the establishment of baseline lung function, the animal receives sequentially increasing intravenous doses of methacholine (Sigma; 3 to 3000 µg/ml in 1 ml/kg body weight increments). Maximal R_{L} responses are determined from measurements averaged over 6-second intervals. Pulmonary function is allowed to return to baseline values before each subsequent dose.

### 9.7. Bronchoalveolar lavage (BAL) analysis

The lungs from five animals of each group are lavaged in *situ* with five 1 ml aliquots of sterile saline, with 3 to 4 ml BAL fluid recovered from each animal. The BAL is centrifuged and resulting cell pellets are suspended in 250 µl saline. BAL protein concentrations are measured in the supernatants by the bicinchoninc acid (BCA) assay using the BCA™ Protein Assay Kit (Pierce, USA) and bovine serum albumin as standard. Total leukocytes are counted in a hemocytometer using trypan blue dye exclusion as a measure of viability. Cytospin slides are made and stained with May-Grunwald/Giemsa to determine the BAL cell differential. The remaining cells are analyzed by fluorescence flow cytometry. For these analyses, BAL samples are washed in phosphate-buffered saline (PBS) containing 0.2% bovine serum albumin and 0.1% NaN₃. Aliquots containing 10⁴ to 10⁵ cells are incubated with 100 µl of appropriately diluted antibodies for 30 min at 4°C. After staining, the cells are washed twice with the above PBS solution, and relative fluorescence intensities are determined on a 4-decade log scale by flow cytometric analysis using a FACScan (Becton Dickinson). Fluorescent monoclonal antibodies used for the fluorescence flow cytometric analyses are directed against B cell and T cell antigens including CD3ε, TCRβ, TCRδ, CD4, CD8, and CD45.

### 9.8. Analysis of serum levels of OVA-specific antibodies

Mice are bled at day 0 before immunization with OVA, one week after immunization with OVA (before starting immunotherapy), one week after the last immunotherapeutic treatment (before the aerosolic challenge), and three days after the final aerosol exposure (before exsanguination). Analyses include the determination of serum levels of OVA-specific antibodies and various cytokines including the TH2 cytokines IL-4, IL-5 and IL-13.

### 9.9. Tissue analysis

For tissue immunofluorescence, unmanipulated lungs (not exposed to BAL or methacholine) are excised, cut into small pieces, and are rapidly frozen in optimal cutting temperature embedding media. The pieces are then cut into 5 µm frozen sections using a Hacker cryostat, mounted onto microscope slides, and stored at -20°C. For immunofluorescence staining, the slides are fixed in acetone (-20°C) for 5 minutes, dried, and blocked with 1% ChromPure IgG solution (Jackson ImmunoResearch) for 30 min at room temperature. After two washes with PBS containing 1% NaN₃, specific fluorescent monoclonal antibodies are added to the tissue and incubated for 60 min in a humidity chamber. Slides are then washed twice with PBS containing 1% NaN₃, and then analyzed by fluorescence microscopy. For staining with hematoxylin and eosin (H&E), unmanipulated lungs (not exposed to BAL or methacholine) are excised, fixed with 10% buffered formalin, and stained with H&E according to standard protocols.

### 9.10. Discussion of the results

In accordance with the positive results of a recent vitamin D-supported immunotherapy study using a murine model of OVA-induced allergic airway inflammation (Heine et al., 2014), calcitriol-loaded PS-liposomes containing DexP and OVA reduce the allergic airway inflammation and responsiveness upon aerosolic OVA challenge significantly, whereas liposomal formulations containing the control antigen mBSA have no significant effect.

In this example, the administered amount of liposomal vitamin D3 derivative calcipotriol/mouse (approx. 10 µg liposomal calcipotriol/100 µl hydrogel/liposome composit) is 10-fold higher than the amount of calcidiol (50 µg calcidiol/kg body weight; corresponding to 1 µg calcidiol per female BALB/c mouse) used to support OVA-specific immunotherapy in the study of Heine et al. (2014). However, calcipotriol has a low effect effect on calcium metabolism, a very short half-life in circulation and is rapidly metabolized to relatively inactive compounds. Furthermore, liposomal calcipotriol and free calcidiol target different cell populations. Free vitamin D3 targets primarily effector T cells among other immune cells, whereas liposome-encapsulated vitamin D3 is phagocytosed by APCs. As a result of phagocytosis, vitamin D3-mediated adverse side effects are minimized and the higher liposomal concentration efficiently inhibits the proinflammatory transcription factor NF-κB in APCs in concert with the liposome-encapsulated DexP.

The amount of liposomal OVA administered in this example (70-80 µg OVA/100 µl hydrogel/liposome composit) is comparable to that used for specific immunotherapy in the study of Heine et al. (2014). In the latter study, successful immunotherapy was achieved with three weekly injections of 100 µg OVA.

### REFERENCES

Aanstoot H.J., et al. Pediatric diabetes. 8. Suppl 8: 1-44, 2007.
Abe M., et al. J. Immunol. 167: 4651-4660; 2001.
Adamy C., et al. J. Mol. Cell. Cardiology 43: 344-353; 2007.
Adler A., et al. Comb. Chem. High Throughput Screen 11: 16-23; 2008.
Alamanos Y., et al. Semin. Arthritis Rheum.36: 182-8; 2006.
Albani S., et al. Nat. Med. 1 : 448-452 ; 1995.
Alhalaweh A., et al.Eur. J. Pharm. Sci. 38: 206-214; 2009.
Altamimi S., et al. Pediatr. Emerg. Care 22: 786-793; 2006.
Alvarez-Lafuente R., et al. Ann. Rheum. Dis.64: 1357-1359, 2005.
Ames R.S., et al. J. Immunol. 166: 6341-6348; 2001. Andjelkovic Z., et al. Clin. Exp. Rheumatol. 17: 453-456; 1999.
Anderson P.H., et al.Mol. Cell. Endocrinol. 285: 1-9; 2008.
Anderson R., et al. Arthr. Res. Ther. 12 : R147 ; 2010.
Andreau K., et al. Immunopharmacology 40 : 67-76 ; 1998.
Anzilotti C, et al.Autoimmun Rev9:158-160; 2010.
Apetoh L. et al., Nat. Immunol. 11: 854-861; 2010.
Arumugam T.V., et al. Kidney Int. 63:134-142; 2003.
Arumugam T.V., et al. J. Hepatol. 40: 934-941; 2004.
Arur S., et al. Develop. Cell 4 : 587-598 ; 2003.
Ascherio A., Munger K.L.Ann. Neurol.61: 504-513; 2007.
Assier E., et al. Ann. Rheum. Dis. 69:A46-A47; 2010
Baelder R., et al. J. Immunol. 174: 783-789; 2005.
Balandraud N., et al. Autoimmun. Rev.3: 362-367; 2004.
Balasubramanian K., Schroit A.J., J. Biol. Chem. 273: 29272-29277; 1998.
Ballantyne, S.J., et al. J. Allergy Clin. Immunol. 120: 1324-1331; 2007.
Bao L., et al. J. Immunol. 175: 1947-1955; 2005a.
Bao L., et al. Eur. J. Immunol. 35: 2496-2506; 2005b.
Baranzini S.E.Genetics & Development21: 317-24; 2011.
Barlow J.L., McKenzie A.N.Biofactors, vol. 35: 178-182; 2009.
Barnes P.J. Allergy 56: 928-936; 2001.
Basomba A., et al. J. Allergy Clin. Immunol. 109: 943-948; 2002.
Bautsch W., et al. J. Immunol. 165: 5401-5405; 2000.
Beer T.M., et al. Clin. Cancer Res. 11: 7794-7799; 2005.
Bellamy N., et al. Ann. Rheum. Dis.51: 588-593; 1992.
BelogurovJr. A.A., et al. FASEB J. 27: 222-231; 2013.
Bereshchenko O., et al. Cell Rep.7: 464-475; 2014.
Biesecker G., et al. Immunopharmacol. 42: 219-320; 1999.
Bikle D.D., Curr. Osteoporos. Rep. 7: 58-63; 2009.
Binderup L., et al. Biochem Pharmacol. 42:1569-1575; 1991.
Blagg J., et al.Bioorg. Med. Chem. Lett. 18: 5601-5604; 2008.
Blass S, et al. Arthritis Rheum44:761-771.; 2001.
Bluestone J.A., Tang Q. Proc. Natl. Acad. Sci. USA101 Suppl. 2, 14622-14626; 2004.
Bluestone J.A., et al. Nature464: 1293-1300, 2010.
Boks M.A., et al. Clin. Immunol. 142: 332-342; 2012.
Bouillon R., et al.Endocrinol. Rev. 29: 726-775; 2008.
Brackertz D., et al. Arthr. Rheum. 20 : 841-850 ; 1977.
Branisteanu D.D., et al. J. Neuroimmunol. 61: 151-160; 1995.
Brgles M., et al. J. Liposome Res. 18 : 235-248 ; 2008.
Brodbeck R.M., et al. J. Pharmacol. Exp. Ther.327 : 898-909 ; 2008.
Burton B.R., et al. Nature Communications 5, article 4741; 2014.
Cantorna M.T., et al. J. Immunol. 160: 5314-5319; 1998.
CantornaM.T., et al. J. Nutr. 128: 68-72; 1998.
Capini C., et al. J. Immunol. 182: 3556-3565; 2009.
Carranza F., et al. PLOS One 7, p.e40356; 2012.
Carrol M.C., Nat. Immunol. 5: 981-986 ; 2004.
Catley M.C., et al. Pharmacol. Therap. 132: 333-351; 2011.
Chambers E.S., Hawrylowicz C.M., Curr. Allergy Asthma Rep. 11: 29-36; 2011.
Chaurio R.A., et al. Molecules 14 : 4892-4914 ; 2009.
Chauveau C., et al. Blood 106: 1694-1702; 2005.
Chekeni F.B., Ravichandran K.S. J. Mol. Med. (Berl.) 89: 13-22; 2011.
Chen A., et al. J. Immunol. 193: 35-39; 2014.
Chen L., et al. Osteoarthr. Cartilage 19: 711-718; 2011.
Chen X., et al. J. Immunol 173:2985-2994; 2004.
Chen X., et al. Eur. J. Immunol. 34: 859-869; 2004b.
Chen X., et al. J. Immunl. 179: 154-161; 2007.
Chen X., Oppenheim J.J. In: TNF pathophysiology. Molecular and cellular mechanisms. Curr. Dir. Autoimmun., pp. 119-134, Karger, Basel; 2010.
Chen Y., et al. J. Clin. Invest. 116: 1317-1326; 2006.
Choi, S., et al.Pharmaceut. Res. 20: 2008-2010; 2003.
Chorny A., et al. Proc. Natl. Acad. Sci. USA 102: 13562-13567; 2005.
Cianferoni A., et al. Blood 97: 1742-1749; 2001.
Cirunay J.J.N., et al. J. Chromatogr. Sci. 36: 417-421; 1998.
Claassen E. J. Immunol. Meth. 147: 231-240; 1992.
Clare-Salzler M.J., et al. J. Clin.Invest. 90: 741-748; 1992.
Cohen S., et al. Biochim. Biophys. Acta 1063: 95-102; 1991.
Comalada M., et al. Eur. J. Immunol. 35: 584-592; 2005.
Compston A., Coles A.Lancet359: 1221-31; 2002.
Compston A., Coles A. Lancet372: 1502-17; 2008.
Corren J. Discov. Med. 5:243-249; 2013.
Corrigan C.J., et al. J. Allergy Clin. Immunol. 128: 116-124; 2011.
D'Ambrosio A., et al. J. Leukoc. Biol. 84: 661-668; 2008.
Das A., et al. J. Immunol. 192: 1120-1129; 2014.
Deane K.D., et al. Arthritis Rheum. 62: 3161-3172; 2011.
Decard B.F., et al. J. Neurol. Neurosurg. Psychiatry 83:1170-1173; 2012.
Defer N., et al. J. Biol. Chem. 282: 35564-35573; 2007.
Denonne F., et al. Bioorg. Med. Chem. Lett. 17: 3258-3261; 2007a.
Denonne F., et al. Bioorg. Med. Chem. Lett. 17: 3262-3265; 2007b.
Dong Z., et al.J. Biol. Chem. 272: 9962-9970; 1997.
Drouin S.M., et al. J. Immunol. 169: 59-26-5933; 2002.
Drouin S.M., et al. Am. J. Respir. Crit. Care Med. 173: 852- 857; 2006.
Duan L., et al. Clin. Develop. Immunology. Article ID 924363; 2013
Elliott, M.R., et al. Nature 461 : 282-286 ; 2009.
Esensten J.H., et al. Nat. Rev. Rheumatol. 5: 560-565; 2009.
Eylar E., et al. Int. Immunol. 1: 97-101; 1993.
Fadok V.A., et al. J. Immunol. 148 : 2207-2216 ; 1992.
Fadok V.A., et al. J. Biol. Chem. 276 : 1071-1077 ; 2001.
Fairweather D., Rose N.R. Nat. Immunol.3: 338-40, 2002.
Fakih M.G., et al. Clin. Cancer Res. 13: 1216-1223; 2007.
Fang C., et al. Blood 114: 1005-1015; 2009.
Farahani R., et al. Adv Biomed Res. 2014; 3: 127. doi: 10.4103/2277-9175.133249.
Fazekasova H., et al. Transplantation 87: 1617-1628; 2009.
Fessler J., et al. BioDrugs 27: 281-291; 2013.
Fiane A.E., et al. Xenotransplantation 6: 52-65; 1999.
Finch A.M., et al. J. Med. Chem. 42: 1965-1974; 1999.
Fleili-Hariri M., et al. Diabetes 48: 2300-2308; 1999.
Fletcher J.M., et al. Recent Pat. Inflamm. Allergy Drug Discov. 6: 22-34; 2012.
Francois C., et al.Patent WO2009046198.
Freedman M.S., et al. Neurology77: 1551-1560; 2011.
Freireich E.J., et al. Cancer Chemother. Rep. 50: 219-244; 1966.
Fujita J., et al. Allergy 67 : 744-750 ; 2012.
Furst D.E., et al.J. Rheumatol. 14: 342-347; 1987.
Gaffen S.L. Nature Rev. Immunol. 9: 556-567; 2009.
Gagliani N.,et al. Nat. Med.19: 739-746; 2013.
Gale E.A., et al. Lancet, 363: 925-931; 2004.
Galvain S., et al. Current Therap. Res. 60 : 278-294 ; 1999.
Garber K.Nature507: 418-420; 2014
Gardai S.J., et al. Cell 123 : 321-334 ; 2005.
Geelen T., et al. J. Nanobiotechnology 10 : 37-48 ; 2012.
Gent Y.Y.J., et al. Arthr. Res. Ther. 16 : R70 ; 2014.
Gerard N.P., Gerard C. Curr. Opin. Immunol. 14: 705-708; 2002.
Germer K., et al. Int. J. Biochem. Mol. Biol. 4: 27-40; 2013.
Getts D.R., et al. J. Immunol. 187 : 2405-2417 ; 2011.
Ghoreishi M., et al. J. Immunol. 182: 6071-6078; 2009.
Giannoukakis N., et al. Diabetes Care 34: 2026-2032; 2011.
Gibeon D., Menzies-Gow A.N. Expert Rev. Respir. Med. 6: 423-39; 2012.
Gilbert J.C., et al. J. Control. Release 5: 113-118; 1987.
Gilbreath M.J., et al. J. Immunol. 134 : 3420-3425 ; 1985.
Gilden D.H. Lancet Neurology4: 195-202; 2005.
Goeldner I., et al. Rheumatology (Oxford)49: 1590-3; 2010.
Gogishvili T., et al. Int. Arch. Allergy Immunol. 142: 165-174; 2006.
Gohil U., et al. Global J. Pharmacol. 4 : 19-30 ; 2010.
Gombault A., et al. Frontiers Immunol. 3: article 414; 2013.
Gong, C.Y., et al. Int. J. Pharm. 365: 89-99; 2009a.
Gong C.Y., et al.BMC Biotechnol. 9: 8; 2009b.
Gonzalez-Rey E., et al. Blood 107: 3632-3638; 2006.
Gonzalez-Rey E., et al. Nature Rev. Immunol. 7: 52-63; 2007.
Goulding N.J., et al. Inflamm. Res. 3 : S158-S165 ; 1998.
Grant C.R., et al. Autoimmunity Rev. 14: 105-116; 2015.
Greenberg R.A., et al. Clin. Pediatr. (Phila.) 47: 817-823; 2008.
Gross C.C., Wiendl H. Curr. Opin. Rheumatol. 25: 268-274; 2013.
Grunewald S.M., et al. J. Immunl. 160: 404-4009; 1998.
Guillot X., et al. Joint Bone Spine77: 552-7; 2010.
Gupta P., Bhatia V. Indian J. Pediatr. 75: 1039-1044; 2008.
Hackstein H., et al. J. Immunol. 166: 7053-7062; 2001.
Hackstein H., et al. Blood 100: 1084-1087; 2002.
Hackstein H., et al. Blood 101: 4457-4463; 2003.
Hagenaars N., et al. J. Control. Release 144: 17-24; 2010.
Hanayama R., et al. Nature 417 : 182-187 ; 2002.
Harel-Adar T., et al. Proc. Natl. Acad. Sci. USA 108 : 1827-1832 ; 2011.
Harkin D.W., et al. J. Vasc. Surg. 39: 196-205; 2004.
Harris S. J. Nutr.135: 323-325, 2005.
Harris V.K., Sadiq S.A.Mol. Diagn. Ther.13: 225-244; 2009.
Hart P.H., et al. Nat. Rev. Immunol. 11: 584-596; 2011.
Hashimoto M., et al. J. Exp. Med. 207: 1135-1143; 2010.
Hegele R.A., et al. Lancet Diabetes Endocrinol. pii: S2213-8587 (13) 70191-8; 2013.
Hegeman M.A., et al. Brit. J. Pharmacol. 163: 1048-1058; 2011.
Heine G., et al. J. Immunol. 193: 1017-1023; 2014.
Herold K.C., et al. Nat. Rev. Immunol. 13: 243-256; 2013.
Hewison M. Mol. Cell. Endocrinol. 321: 103-111; 2010.
Hirst M., Feldman D. Biochem. Biophys. Res. Commun. 105: 1590-1596; 1982a.
Hirst M., Feldman D. Endocrinol. 111: 1400-1402; 1982b.
Hochreiter-Hufford A., Ravichandran,K.S. Cold Spring Harb. Perspect. Biol. 5: a008748; 2013.
Hoffmann, P.R., et al. J. Immunol. 174 : 1393-1404 ; 2005.
Hofstetter H.H., et al. Cell. Immunol. 237: 123-130; 2005.
Holgate S.T. J. Allergy Clin. Immunol. 128 : 495-505 ; 2011.
Huber-Lang M., et al. Am. J. Pathol. 161: 1849-1859; 2002.
Humbles A.A., et al. Nature 406: 998-1001; 2000.
Hyppönen E., et al. Lancet 358: 1500-1503; 2001.
Hyun H., et al. Biomacromolecules 8: 1093-1100; 2007.
Idoyaga J., et al. J. Clin. Invest. 123 : 844-854 ; 2013.
Igarashi M., et al. Clin. Exper. Immunol. 93 : 19-25 ; 1993.
Imaizumi T., et al. J. Toxicol. Sci. 21 (supplement II): 277-285; 1996.
Iruretagoyena M., et al. J. Pharmacol. Exp. Ther. 312: 366-372; 2005.
Ishii M., et al. Intern. Immunopharmacol.10: 1041-1046; 2010.
Iwasaki E. Clin. Chem. 33: 1412-1418; 1987.
Jaeckel E., et al.Diabetes54, 306-310; 2005.
Jayakumar T., et al. Evidence-Based Complement. Alternat. Med., Article ID 846740; 2013.
Jeannin P., et al. J. Exp. Med. 182: 1785-1792; 1995.
Jeffery L.E., et al.J. Immunol. 183: 5458-5467; 2009.
Jenkins M.K., Schwartz, R.H., J. Exp. Med. 165 : 202-319 ; 1987.
Jeong B., et al. Nature 388: 860-862, 1997.
Jiang H.-R., et al. Eur. J. Immunol. 42: 1804-1814; 2012.
Jirapongsananuruk O., et al.J. Allergy Clin. Immunol. 106: 981-985; 2000.
Jones G. Am. J. Clin. Nutr. 88(suppl.): 582S-586S; 2008.
Jones S.A., et al. Nature Immunol. 13: 1022-1025; 2012.
Josefowicz S.Z., et al. Annu. Rev. Immunol. 30: 531-564; 2012.
Kalantari T., et al. Clin. Exp. Immunol. 176: 180-189; 2014.
Kang Y.M., et al. Biomaterials 31: 2453-2460; 2010.
Karagiannidis C.,et al. J. Allergy Clin. Immunol. 114 : 1425-1433 ; 2004.
Katragadda M., et al. Med. Chem. 49: 4616-4622; 2006.
Kawamoto S., et al. J. Clin. Invest. 114: 399-407; 2004.
Kearley J., et al. J. Exp. Med. 202: 1539-1547; 2005.
Keefe A.D., et al. Nature Rev. Drug Discov. 9 : 537-550 ; 2010.
Kelchtermans H., et al. Ann. Rheum. Dis. 68: 744-750; 2009.
Kimball S.M., et al. Am. J. Clin. Nutr. 86: 645-651; 2007.
Kissmeyer A.-M., Binderup, L., Biochem. Pharmacol.41: 1601-1606; 1991.
Knip M., et al. Diabetes54: S125-S136, 2005.
Ko H., et al. Transpl. Immunol. 20: 99-105; 2008.
Köhl J., et al.J. Clin. Invest. 116: 783-796; 2006a.
Köhl J., Curr. Opin. Mol. Ther. 8: 529-538; 2006b.
Koenders M.I., et al. Arthritis Rheum.52: 3239-3247; 2005.
Koffeman E.C., et al. Arthritis Rheum. 60: 3207-3216; 2009.
Kohm A. P., et al. J. Immunol.169, 4712-4716; 2002.
Komai-Koma M., et al. Eur J Immunol. 2007;37:2779-86; 2007.
Komiyama Y., et al. J. Immunol. 177: 566-573; 2006.
Kondo C., et al. Clin. Exp. Immunol. 124: 323-329; 2001.
Kong N., et al. Arthritis Rheum. 64: 2548-2558; 2012.
Koning G.A., et al. Arthritis Rheum. 54: 1198-1208; 2006.
Kono H., Rock, K.L., Nature Rev. Immunol. 8 : 279-289 ; 2008.
Konteatis Z.D., et al.J. Immunol. 153: 4200-4205; 1994.
Koop E., Ghosh S., Science 265: 956-959; 1994.
Kornbluth R.S., Immunol. Lett.43 : 125-132 ; 1994.
Kragballe K., Pharmacol. Toxicol. 77: 241-246; 1995.
Kretschmer K., et al. Nat. Immunol. 6: 1219-1227 ; 2005.
Kurowska-Stolarska M., et al. J Immunol. 181:4780-90; 2008.
Kwak H.W., D'Amico D.J. Korean J. Ophthalmol. 9 : 79-83 ; 1995.
Landewe R.B.N., et al. Ann. Rheum. Dis. 69: 1655-1659; 2010.
Lappegard K.T., et al.Ann. Thorac. Surg. 79: 917-923; 2005.
Lappegard K.T., et al. J. Biomed. Mater. Res. A87: 129-135; 2008.
Law S.C., et al. Arthritis Res Ther14:R118; 2012.
Lee H., et al. Immunol. Cell Biol. 86 : 153-160 ; 2008.
Lee H.Y., et al. Exp. Lung Res. 40: 66-76; 2014.
Lenmark A., LarssonH.E. Nature Rev. Endocrinology 9: 92-103; 2013.
Li R., et al. J. Transl. Med. 10: p.19; 2012.
Li X., et al. Am. J. Respir. Cell Mol. Biol. 42: 190-199; 2010.
Liang C.M., et al. J. Biol. Chem. 264: 13519-13523; 1989.
Liang S.M., et al. J. Immunol. 146: 1909-1913; 1991.
Linker R.A., et al. Exp. Neurol. 211: 397-406; 2008.
Longui C.A., J. Pediatr. (Rio J.) 83: S163-171; 2007.
Lozano, R. et al.Lancet380: 2095-128; 2012.
Lu L., et al. Adv. Exp. Med. Biol. 417:275-282; 1997.
Lubberts E., et al. J. Immunol. 167: 1004-1013; 2001.
Lubberts E. Curr. Opin. Investig. Drugs 4: 572-7; 2003.
Lubberts E., et al. Arthritis Rheum. 50: 650-659; 2004.
Ludvigsson J., et al. N. Engl. J. Med. 359: 1909-1910; 2008.
Ludvigsson J., et al. N. Engl. J. Med. 366: 433-442; 2012.
Luime J.J., et al. Ann Rheum Dis69: 337-44; 2009.
Lutterotti A.,et al. Sci. Transl. Med.5: 188ra75; 2013.
Ma Q., et al. Bone Marrow Transplantation49: 972-976; 2014.
Macauley M.S., et al. J. Clin. Invest. 123: 3074-3083; 2013.
Machen J., et al. J. Immunol. 173: 4331-4341; 2004.
Mackern-Oberti J.P., et al. Autoimmun. Rev. 14: 127-139; 2015.
Mahon B.D., et al.J. Cell. Biochem. 89: 922-932; 2003.
Majak P., et al. J. Allergy Clin. Immunol. 127 : 1294-1296 ; 2011.
Mantel P.-Y. et al.PLoS biology 5, e329: 2847-2861; 2007.
March D.A., et al. Mol. Pharmacol.65 : 868-879 ; 2004.
Markiewski M.M., et al. Nat. Immunol. 9 : 1225-1235 ; 2008.
Martin E., et al. Arthritis Rheum. 56: 2255-2266; 2007.
Masson-Bessiere C., et al. J Immunol166:4177-4184; 2001.
Matasic R., et al. J. Leukocyte Biol. 66: 909-914: 1999.
Matsuda R., et al. Invest. Ophthalmol. Vis. Sci. 53: 7235-7245; 2012.
Mazzeo D., et al.Eur. J. Immunol. 28: 3205-3213; 1998.
Mazzucconi M.G., et al. Blood 109: 1401-1407; 2007.
McRae B.L., et al. J. Exp. Med.182: 75-85; 1995.
Meechan P., et al. Int. Arch. Allergy Immunol. 61 : 351-362 ; 2013.
Menges M., et al. J. Exp. Med. 195: 15-21; 2002.
Merz K., Sternberg B. J. Drug Target. 2: 411-417; 1994.
Metasic R., et al. J. Leukocyte Biol. 66: 909-914; 1999.
Methner A., Zipp F. Nature Rev. Neurol.9: 72-73; 2013.
Metselaar J.M., et al. Ann. Rheum. Dis. 63: 348-353; 2004.
Meyer C., et al. J. Nucleic Acids, Article ID 904750; 2011.
Miller D., et al. Lancet Neurol.4: 281-288; 2005.
Milo R., Kahana E. Autoimmun. Rev.9: A387-394; 2010.
Miossec P. Arthritis Rheum.48: 594-601; 2003.
Miossec P., Kolls, J.K. Nature Rev. Drug Discovery 11: 763-776; 2012.
Mizutani N., et al. J. Immunol. 188: 5694-5705; 2012.
Mizutani N., et al. J. Immunol. 192: 1372-1384; 2014.
Mollnes T.E., et al.Trends Immunol. 23: 61-64; 2002.
Morgan B.P., Gasque, P., Clin. Exp. Immunol. 107: 1-7 ; 1997.
Morgan M.E., et al. Arthritis Rheum. 52: 2212-2221; 2005.
Morikis D., et al. Protein Science 7: 619-627; 1998.
Motomura Y., et al. Nat. Immunol.12:450-459; 2011.
Mottet C., et al. J. Immunol. 170: 3939-3943; 2003.
Muindi J.R., et al. Oncology 66: 62-66; 2004.
Munger K.L., et al. JAMA 296: 2832-2838; 2008.
Nakae S., et al. J. Immunol. 171: 6173-7; 2003.
Nakano Y., et al. J. Allergy Clin. Immunol. 112: 525-530; 2003.
Naranjo-Gomez M., et al. J. Transl. Med. 9: 89-102; 2011.
Nashold F.E., et al. J. Neuroimmunol. 263: 64-74; 2013.
Nie S., et al. Internatl. J. Nanomed. 6: 151-166; 2011.
Nilsson B., et al.Blood 92: 1661-1667; 1998.
Nishimura K., et al. Ann. Intern. Med.146: 797-808; 2007.
Oda N., et al. Am. J. Respir. Crit. Care Med. 171 : 12-18 ; 2005.
Okamura T.;et al. Proc. Natl. Acad. Sci. USA106: 13974-13979; 2009.
Ota K., et al. J. Immunol. Res., Article ID 602846 ; 2014.
Pai S.S., et al. Am. Assoc. Pharmac. Sci. J. 11: 88-98; 2009.
Papenfuss T.L., et al. J. Immunol. 186: 3346-3355; 2011.
Peng Q., et al. J. Immunol. 176: 3330-3341; 2006.
PengK.-T., et al., Biomaterials 31: 5227-5236; 2010.
Petersen B.C., Lukacz, N.W. Future Med. Chem. 4: 833-836; 2012.
Pfrengle F., et al. J. Immunol. 191 : 1724-1731 ; 2013.
Pichler J., et al. Pediatr. Res. 52: 12-18; 2002.
Plenge R.M., et al. New Engl. J.Med.357: 1199-209; 2007.
Plum L.A., et al. Proc. Natl. Acad. Sci. USA 1001: 6900-6904; 2004.
Plum L.A., DeLuca, H.F., Nat. Rev. Drug Discov. 9: 941-955; 2010.
Polosa, R., Casale, T. Drug Discovery Today 17: 591-599; 2012.
Popov I., et al. Arthritis Res. Ther. 8: R141(doi:10.1186/ ar2031); 2006.
Porsbjerk C., et al. J. Asthma 46: 606-612; 2009.
Pot C.,et al. J. Immunol.183:797-801; 2009.
Pratt J.R., et al.Nat. Med. 8: 582-587 ; 2002.
Proctor L.M., et al.Brit. J. Pharmacol. 142: 756-764; 2004.
Putnam A.L., et al. Diabetes 58: 652-662; 2009.
Qiao M., et al. Int. J. Pharm. 294: 103-112; 2005.
Qu H., et al. Mol. Immunol. 47: 185-195; 2009.
Qureshi F., et al. J. Pediatr. 139 : 20-26 ; 2001.
Raedler H., et al. Am. J. Transplant. 9: 1784-1795; 2009.
Raich-Regué D., et al. Eur. J. Immunol. 42:771-782; 2012.
Rank M.A., et al. J Allergy Clin Immunol. 123:1047-54; 2009.
Ravichandran K.S., Immunity 35 : 445-455 ; 2011.
Raz I, et al. Lancet 358: 1749-1753; 2001.
Reichel H., et al.Proc. Natl. Acad. Sci. USA 84: 3385-3389; 1987.
Reichrath J., Holick M.F. In: Holick M.F. (editor). Vitamin D. Physiology, Molecular Biology and Clinical Applications. Vol. 2. New Jersey: Humana Press Totowa; pp. 1043-1060; 2010.
Rhen T., Cidlowski J.A. N. Engl. J. Med. 353 : 1711-1723 ; 2005.
Ricklin D., Lambris J.D., Adv. Exp. Med. Biol. 632: 273-292; 2008.
Ricklin D., et al. Nat. Immunol. 11: 785-797; 2010.
Robinson D.S. J. Allergy Clin. Immunol. 126: 1081-1091; 2010.
Roep B. O.,et al.Sci. Transl. Med.5: 191ra82; 2013.
Rouser G., et al. Lipids 5: 494-496; 1970.
Ruel-Gariepy, E., Leroux, J-C., Eur. J. Pharmaceutics Biopharmaceutics 58: 409-426; 2004.
Sabatos-Peyton C. A., et al.Curr.Opin. Immunol.22: 609-615;2010
Sahu A., et al. J. Immunol. 157: 884-891; 1996.
Sandstrom P.A., et al.J. Leukocyte Biol. 55: 221-226; 1994.
Sayegh M.H., et al. J. Exp. Med. 181:1869-1874; 1995.
Scalapino K.J., et al. J. Immunol. 177: 1451-1459; 2006.
Schatz D.A., Bingley PJ. J. Ped. Endocrinol. Metabol. 14 (Suppl. 1): 619-622; 2001.
Schloot NC, et al. Diabetes Metab. Res. Rev. 23: 276-285; 2007.
Schmidt S., et al. J. Biomed. Mater. Res. A66: 491-499; 2003.
Schmitz J., et al. Immunity 23:479-90; 2005.
Schnatbaum K., et al.Bioorg. Med. Chem. Lett. 16: 5088-5092; 2006.
Schweingruber N., et al. J. Immunol. 187 : 4310-4318 ; 2011.
Scott D.L., et al. Lancet376: 1094-108; 2010.
Shah SC, et al. N. Engl. J. Med., 320: 550-554; 1989.
Sharp A., et al. J. Neuroinflamm. 5 : 33 ; 2008.
Shephard R.M., DeLuca H.F. Arch. Biochem. Biophys. 202: 43-53; 1980.
Shigdar S., et al. Sensors 13: 13624-13637; 2013.
Silman A.J., et al. Brit. J. Rheumatol.32: 903-907; 1993.
Singh D.K., Verma R. Iranian J. Pharmacol. Ther. 7: 61-65; 2008.
Singh, S., et al.Int. J. Pharmaceutics 341: 68-77; 2007.
Sinha,V.R., et al. Int. J. Pharm. 278: 1-23; 2004.
Skyler J.S., et al. Diabetes Care, 28: 1068-1076; 2005.
Smithgall M.D., et al. Int Immunol. 20:1019-30; 2008.
Snir O., et al. Arthritis Rheum63:2873-2883; 2011.
Soetikno V., et al. Nutr. Metabol. 8:35; 2011.
Sokolove J., et al. PLoS One 2012, 7:e35296.
Soulika A.M., et al. Clin. Immunol. 96: 212-221; 2000.
Staal F.J., et al.Proc. Natl. Acad. Sci. USA 87: 9943-9947; 1990.
Steinbrink K., et al. Blood 99: 2468-2476; 2002.
Stoop J.N., et al. Arthritis Rheum. 62: 3656-3665; 2010.
Strainic M.G., et al.Immunity 28: 425-435; 2008.
Strainic M.G., et al. Nat. Immunol.14: 162-171; 2013.
Sugiyama D., et al. Ann. Rheum. Dis.69: 70-81; 2010.
Sumichika H., et al. J. Biol. Chem. 277: 49403-49407; 2002.
Sun Y.P., et al. J. Biol. Chem. 282: 9323-9334; 2007.
Taher Y.A., et al. J. Immunol. 180: 5211-5221; 2008.
Tamachi T., et al. J. Allergy Clin. Immunol. 118: 606-614; 2006.
Tan L.J., et al. J. Immunol. 147 : 1797-1802 ; 1991.
Tang C., et al. Immunology. 135: 112-124; 2012.
Tang J., et al. J. Immunol. 182: 4624-4632; 2009.
Tang Q., et al.J. Exp. Med.199, 1455-1465; 2004.
Tang W., et al. Int. Arch. Allergy Immunol. 63: 5-10; 2014.
Thakker P., et al. J. Immunol. 178: 2589-2598; 2007.
The EURODIAB Substudy 2 Study Group.Diabetologia42:51-54; 1999.
Thiel K.W., Giangrande P.H. Oligonucleotides 19: 209-222; 2009.
Thomas R., et al. Arthritis Rheum. 63: 2430; 2011.
Thomas R. Arthritis Res. Therapy 15: 204-214; 2013.
Thommesen L., Laegreid, A., J. Biochem. Mol. Biol. 38: 281-289; 2005.
Thompson J.A., et al. Curr. Diab. Rep. 12: 623-632; 2012.
Tilstra J.S., et al. Sci. Rep.4: 3631.DOI:10.1038/ srep03631; 2013
Ting E., et al.Br. J. Pharmacol. 153: 1043-1053; 2008.
Tjernberg J., et al. Transplantation 85: 1193-1199; 2008.
Tony H.P., et al.Eur. J. Biochem. 225: 659-665; 1994.
Torchilin V.P., Nature Rev. 4 : 145-160 ; 2005.
Toscano M.G., et al. Mol. Ther. 18: 1035-1045; 2010.
Tremezaygues L., Reichrath J. Dermatoendocrinol. 3: 180-186; 2011.
Tsark E.C., et al. J Immunol169:6625-6633; 2002.
Tsvetkova-Vicheva V., et al.Israel Med. Assoc. J. 16: 358-362; 2014.
Urry Z., et al.J. Clin. Invest. 119: 387-398; 2009.
Uyttenhove C., Van Snick J. Eur. J. Immunol.36: 2868-2874; 2006.
van Brussel I., et al. Autoimmun. Rev. 13: 138-150; 2014.
vander Aar A.M.G., et al. J. Allergy Clin. Immunol. 127: 1532-1540; 2011.
van der Helm-van Mil A.H.M., et al. Arthritis Rheum 56:425-432; 2007.
van de Stadt L.A., et al. Arthritis Rheum. 63: 3226-3233; 2011.
van Duivenvoorde L.M., et al. J. Immuol. 179: 1506-1515; 2007.
Velten F.W., et al. Eur. J. Immunol. 34: 2800-2811; 2004.
Verhoef C.M., et al. Ann. Rheum. Dis. 58 : 49-54 ; 1999.
Verschoor A., et al. J. Immunol. 171: 5363-5371; 2003.
Villadangos J.A., Schnorrer P. Nat. Rev. Immunol. 7 : 543-555 ; 2007.
Voigtlander C., et al. J. Immunother. 29: 407-415; 2006.
Volchenkov R., et al. Arthr. Res. Ther. 15: R114; 2013.
von Delwig A., et al. Arthritis Rheum62:143-149; 2010.
Von Haehling S., et al. Basic Res. Cardiol. 99: 18-28; 2004.
Vossenaar E.R., Van Venrooij W.J. Arthritis Res Ther 2004, 6:107-111; 2004.
Vukman K.V., et al. J. Immunol. 190: 2873-2879; 2013.
Wagner E., Frank M.M., Nature Rev. 9: 43-56; 2010.
Walczak A., et al. J. Am. Med. Assoc. Neurol.70: 1105-1109; 2013.
Waters S.M., et al. J. Biol. Chem. 280: 40617-40623; 2005.
Wen H., Baker J.F. J.Clin. Rheumatol.: Pract. Rep. Rheumatic & Musculoskeletal Dis.17: 102-7; 2011.
Wenzel S., et al. Lancet 370: 1422-1431; 2007.
Wenzel S.E. Nat. Med. 18: 716-725; 2012.
Wherrett D.K., et al. Lancet 378: 319-327; 2011.
Wherry E.J.Nat. Immunol.12: 492-499; 2011.
White R.R., et al. J. Clin. Invest. 106: 929-934; 2000.
Wingerchuk D.M., et al. J. Neurol. Neurosurg. Psychiatry 76: 1294-1296; 2005.
Woodruff T.M., et al. J. Pharmacol. Exp. Ther. 314: 811-817; 2005.
Woodruff T.M., et al., FASEB J. 20: 1407-1417; 2006.
Woodruff P.G., et al. Am. J. Respir. Crit. Care Med. 180: 388-395; 2009.
Woodward J.E., et al. Transplantation 66:14-20; 1998.
Wu Y., et al. Biomaterials 33: 2351-3260; 2012.
Wüst S., et al. J. Immunol. 180 : 8434-8443 ; 2008.
Xiao B.G., et al. Int. Immunol. 16: 13-22; 2004.
Xing Y., et al. J. Liposome Res. 24 : 10-16 ; 2014.
Xu W., et al. PLOS ONE 7 (7): e40314, doi:10.1371/ journal.pone.00401314; 2012.
Yadav M., et al. Frontiers Immunol. 4: 232; 2013.
Yamazaki S., et al.J. Exp. Med. 198: 235-247; 2003.
Yao X.J., et al. Clin.Exp. Allergy 44: 765-777; 2014.
Yiamouyiannis C.A., et al. Am. J. Pathol.154: 1911-1921; 1999.
Yoo S.-A. et al. J Exp Med209:871-886; 2012.
Zaharoff D.A., et al. Vaccine 25: 2085-2094; 2007.
Zella J.B., et al. Arch. Biochem. Biophys. 417: 77-80; 2003.
Zhang H., et al. J. Translat. Med. 12: 125; 2014.
Zhang, J., et al. Biomacromolecules 7: 2492-2500; 2006.
Zheng X., et al. J. Immunol. 184: 6457-6464; 2010.
Zhou W., Immunobiology 217: 225-234; 2012.
Zhou J., Frontiers Genetics 3 : Article 234 ; 2012b.
Zhu S., Qian Y. Clin. Sci. (Lond) 122: 487-511; 2012.

## Claims

1. Pharmaceutical composition made of at least one preparation, wherein the preparation comprises:
tolerogenic liposomes tailored for effective phagocytosis and loaded with at least one maturation inhibitor of dendritic cells (DCs) selected from a) calcipotriol, b) glucocorticoids, and c) antisense oligonucleotides capable of gene silencing of different pro-inflammatory molecules including CD40, CD80, and CD86 and
at least one antigen or allergen or peptide derived thereof selected from ovalbumin (OVA), methylated BSA (mBSA), or the myelin oligodendrocyte glycoprotein (MOG)-derived peptide 35-55 (MOG(35-55),
at least one immune modulator of phagocytosis selected from the nucleotides ATP and UTP,
wherein at least said liposomes are embedded in a matrix suitable for locally restricted sustained release of therapeutically effective doses of said liposomes selected from PLGA-PEG-PLGA triblock copolymers.

2. Pharmaceutical composition of claim 1, wherein said liposomes tailored for effective phagocytosis include liposomes exposing on their surface phosphatidylserine (PS), mannose or oligomannose, or combinations thereof, wherein surface-exposed PS is preferred.

3. Pharmaceutical composition of any of the claims 1 to 2, wherein said liposomes contain at least one encapsulated antigen or allergen, or at least one encapsulated peptide derived thereof, and at least one encapsulated or lipid bilayer-incorporated DC maturation inhibitor.

4. Pharmaceutical composition according to any of the claims 1 to 3, wherein said tolerogenic liposomes tailored for effective phagocytosis and loaded with at least one maturation inhibitor of dendritic cells (DCs) and at least one antigen or allergen or peptide derived thereof, said at least one immune modulator of phagocytosis, and, optionally, said at least one immune modulator suitable for enhancing the suppressive function of regulatory T cells and/or inhibiting the production of pro-inflammatory cytokines, and/or inhibiting the biological activity of secreted pro-inflammatory cytokines at the site of antigen or allergen presentation and being the same as the immune modulator of phagocytosis or different therefrom, are comprised in a single preparation and embedded in one said matrix and/or wherein the composition is galenically prepared for administration by injection or by implantation, intradermally, subcutaneously, nasally, transbucally, transmucosally, sublingually, intraocularly, intramuscularly, or topically.

5. Pharmaceutical composition according to any of the claims 1 to 4 for use in modulation of antigen-presenting cell, T cell and B cell responses by antigen- or allergen-specific immunotherapy in combination with tolerizing liposomes tailored for effective phagocytosis in an organism, preferably a human, in need thereof, in particular for the treatment of T cell-mediated diseases, preferably selected from the group consisting of allergy, allergic asthma, type 1 diabetes, rheumatoid arthritis, and multiple sclerosis, wherein the pharmaceutical composition is administered to the organism in need thereof in a therapeutically effective dose.

6. Method for manufacturing a pharmaceutical composition according to any of the claims 1 to 4, wherein the said components are mixed with each other in a therapeutically effective quantity and embedded into said matrix, and wherein, optionally, galenic compounds are additionally admixed to one or all of the preparations.

## Patentansprüche

1. Pharmazeutische Zusammensetzung, hergestellt aus mindestens einer Präparation, wobei die Präparation umfasst:
tolerogene Liposome, die für eine wirksame Phagozytose abgestimmt sind und mit mindestens einem Maturations-Inhibitor von dendritischen Zellen (DCs) beladen sind, ausgewählt aus a) Calcipotriol, b) Glukokortikoiden und c) Antisense-Oligonukleotiden, die zur Gen-Stilllegung von verschiedenen proinflammatorischen Molekülen einschließlich CD40, CD80 und CD86 in der Lage sind, und
mindestens ein Antigen oder Allergen oder ein davon abgeleitetes Peptid, ausgewählt aus Ovalbumin (OVA), methyliertem BSA (mBSA) oder dem von Myelin-Oligodendrozyten-Glycoprotein (MOG) abgeleiteten Peptid 35-55 (MOG(35-55)),
mindestens einen Immunmodulator von Phagozytose ausgewählt aus den Nukleotiden ATP und UTP,
wobei mindestens die Liposome in einer Matrix eingebettet sind, die für eine lokal eingeschränkte verzögerte Freisetzung von therapeutisch wirksamen Dosen der Liposome ausgewählt aus PLGA-PEG-PLGA-Triblockcopolymeren geeignet ist.

2. Pharmazeutische Zusammensetzung nach Anspruch 1, wobei die für eine wirksame Phagozytose abgestimmten Liposome Liposome umfassen, die auf ihrer Oberfläche Phosphatidylserin (PS), Mannose oder Oligomannose oder Kombinationen davon exponieren, wobei oberflächenexponiertes PS bevorzugt ist.

3. Pharmazeutische Zusammensetzung nach einem der Ansprüche 1 bis 2, wobei die Liposome mindestens ein eingekapseltes Antigen oder Allergen oder mindestens ein davon abgeleitetes eingekapseltes Peptid und mindestens einen eingekapselten oder in einer Lipiddoppelschicht aufgenommenen DC-Maturations-Inhibitor enthalten.

4. Pharmazeutische Zusammensetzung nach einem der Ansprüche 1 bis 3, wobei die für eine wirksame Phagozytose abgestimmten und mit mindestens einem Maturations-Inhibitor von dendritischen Zellen (DCs) und mindestens einem Antigen oder Allergen oder einem davon abgeleiteten Peptid beladenen Liposome, der mindestens eine Immunmodulator von Phagozytose und wahlweise der mindestens eine Immunmodulator zum Verstärken der suppressiven Funktion von regulatorischen T-Zellen und/oder zum Hemmen der Produktion von proinflammatorischen Zytokinen und/oder zum Hemmen der biologischen Aktivität von sekretierten proinflammatorischen Zytokinen am Ort der Antigen- oder Allergenpräsentation, und der gleich dem Immunmodulator von Phagozytose oder verschieden davon ist, in einer einzigen Präparation enthalten und in einer Matrix eingebettet sind, und/oder wobei die Zusammensetzung galenisch zur Verabreichung durch eine Injektion oder Implantation, intradermal, subkutan, nasal, transbukkal, transmukosal, sublingual, intraokulär, intramuskulär oder topisch angefertigt ist.

5. Pharmazeutische Zusammensetzung nach einem der Ansprüche 1 bis 4 zur Verwendung bei der Modulation von antigenpräsentierenden Zell-, T-Zell- und B-Zell-Reaktionen durch eine antigen- oder allergenspezifische Immuntherapie in Kombination mit für eine wirksame Phagozytose abgestimmten toleranzinduzierenden Liposomen in einem Organismus, bevorzugt einem Menschen, der dieses benötigt, insbesondere für die Behandlung von T-Zell-vermittelten Krankheiten, bevorzugt ausgewählt aus der Gruppe bestehend aus Allergie, allergischem Asthma, Diabetes Typ 1, rheumatoider Arthritis und multipler Sklerose, wobei die pharmazeutische Zusammensetzung dem dies benötigenden Organismus in einer therapeutisch wirksamen Dosis verabreicht wird.

6. Verfahren zum Herstellen einer pharmazeutischen Zusammensetzung nach einem der Ansprüche 1 bis 4, wobei die Komponenten miteinander in einer therapeutisch wirksamen Menge gemischt werden und in die Matrix eingebettet werden, und wobei wahlweise einer oder allen der Präparationen galenische Verbindungen zusätzlich beigemischt werden.

## Revendications

1. Composition pharmaceutique réalisée en au moins une préparation, dans laquelle la préparation comprend:
des liposomes tolérogènes adaptés pour la phagocytose efficace et chargés en au moins un inhibiteur de maturation de cellules dendritiques (CDs) choisies à partir a) du calcipotriol, b) des glucocorticoïdes, et c) des oligonucléotides antisens capables d'extinction de gènes de molécules pro-inflammatoires différentes inclusivement CD40, CD80 et CD86 et
au moins un antigène ou allergène ou un peptide dérivé de ceux-ci choisi à partir de l'ovalbumine (OVA), du BSA méthylé (mBSA), ou le peptide 35-55 (MOG(35-55)) dérivé de glycoprotéine myéline oligodendrocyte (MOG),
au moins un modulateur immunitaire de la phagocytose choisi à partir des nucléotides ATP et UTP,
dans laquelle au moins ces liposomes sont noyés dans une matrice utile pour la libération prolongée localement limitée de doses thérapeutiquement efficaces de ces liposomes choisis à partir de copolymères à trois blocs PLGA-PEG-PLGA.

2. Composition pharmaceutique selon la revendication 1, dans laquelle ces liposomes adaptés pour la phagocytose efficace comportent des liposomes exposant sur leur surface de la phosphatidylsérine (PS), du mannose ou de l'oligomannose, ou des combinaisons de ceux-ci, dans laquelle la PS exposée sur la surface est préférée.

3. Composition pharmaceutique selon une des revendications 1 à 2, dans laquelle ces liposomes comportent au moins un antigène ou allergène encapsulé, ou au moins un peptide encapsulé dérivé de ceux-ci, et au moins un inhibiteur de maturation CD encapsulé ou incorporé dans un lipide de type bicouche.

4. Composition pharmaceutique selon une des revendications 1 à 3, dans laquelle ces liposomes tolérogènes adaptés pour la phagocytose efficace et chargés en au moins un inhibiteur de maturation de cellules dendritiques (CDs) et au moins un antigène ou allergène ou un peptide dérivé de ceux-ci, cet au moins un modulateur immunitaire de la phagocytose, et, optionnellement, cet au moins un modulateur immunitaire utile pour améliorer la fonction suppressive de cellules T régulatrices et/ou inhiber la production de cytokines pro-inflammatoires, et/ou inhiber l'activité biologique de cytokines pro-inflammatoires sécrétées à l'endroit de la présentation d'antigène ou d'allergène et étant le même que le modulateur immunitaire de la phagocytose ou différent de celui-ci, sont compris dans une seule préparation et noyés dans une telle matrice et/ou dans laquelle la composition est galéniquement préparée pour l'administration par injection ou par implantation, intradermale, sous-cutanée, nasale, transbuccale, transmucosale, sublinguale, intraoculaire, intramusculaire, ou topique.

5. Composition pharmaceutique selon une des revendications 1 à 4 pour l'utilisation dans la modulation de réactions de cellules présentatrices d'antigène, cellules T et cellules B par une immunothérapie spécifique aux antigènes ou allergènes en combinaison avec des liposomes d'induction de tolérance adaptés pour la phagocytose efficace dans un organisme, de préférence un humain, en ayant besoin, en particulier pour le traitement de maladies contrôlées par des cellules T, de préférence choisies à partir du groupe consistant en de l'allergie, de l'asthme allergique, du diabète de type 1, de l'arthrite rhumatoïde, et de la sclérose en plaques, dans laquelle la composition pharmaceutique est administrée à l'organisme en ayant besoin à une dose thérapeutiquement efficace.

6. Procédé de fabrication d'une composition pharmaceutique selon une des revendications 1 à 4, dans lequel ces composants sont en mélange les uns avec les autres à une quantité thérapeutiquement efficace et noyés dans cette matrice, et dans lequel, optionnellement, on mélange additionnellement des composés galéniques à une ou toutes les préparations.
